# EUROPEAN PATENT APPLICATION

(11) **EP 4 353 266 A1**
(43) Date of publication of application: **17.04.2024**
(21) Application number: 22816021.4
(22) Date of filing: 27.05.2022
(51) Int. Cl.: A61K 47/54, A61K 31/7088, A61K 31/7105, A61K 31/713, A61P 43/00, C12N 15/113, C12N 15/115

(54) **LIGAND-BOUND NUCLEIC ACID COMPLEX**

(30) Priority: 31.05.2021 JP 2021091589
(71) Applicant: Rena Therapeutics Inc., Tokyo 100-0004 (JP)
(72) Inventor: TAKAGI Koh, Tokyo 100-0004 (JP); KIZAWA Hideki, Tokyo 100-0004 (JP); OKAMOTO Hiroshi, Tokyo 100-0004 (JP); KANEKO Keiko, Tokyo 100-0004 (JP); HUSE Nobuyuki, Tokyo 100-0004 (JP); OCHI Takashi, Tokyo 100-0004 (JP)
(74) Representative: J A Kemp LLP
(86) International application number: PCT/JP2022/021836
(87) International publication number: WO 2022/255273

(57) **Abstract**

This invention provides an S1P ligand-binding nucleic acid complex that is delivered to a target organ to regulate expression or editing of a target gene or a transcription or translation product thereof. The S1P ligand-binding nucleic acid complex in which a nucleic acid comprising an antisense strand comprising an oligonucleotide having a nucleic acid sequence complementary to a target transcription product and consisting of 12 to 30 nucleotides is bound to a S1P ligand is delivered to an organ to regulate expression or editing of a target gene or a transcription or translation product thereof. Thus, such nucleic acid complex is useful for treatment of a disease caused by such gene. In addition, the ligand-binding nucleic acid complex can be delivered to an organ, tissue, or cell in which the S1P receptor is expressed, such as the skeletal muscle, cardiac muscle, liver, kidney, lung, mammary gland, fat, podocyte, lymphocyte, or vascular endothelial cell. Thus, such nucleic acid complex is useful for treatment of diseases in such organs.

## Description

### Technical Field

The present invention relates to a nucleic acid complex that regulates expression or editing of a target gene or a transcription or translation product thereof. More particularly, the present invention relates to a ligand-binding nucleic acid complex in which a ligand that can be delivered to a target organ, cell, or the like binds to a nucleic acid complex.

### Background Art

Unlike conventional low molecular medicine, nucleic acid medicine acts on the sequence of a transcription product of a disease-causing gene to regulate expression of the transcription product or a protein. Accordingly, development thereof as next-generation medicine has been in progress.

Examples of known nucleic acids that have heretofore been used for nucleic acid medicine include short-interfering nucleic acid (siNA), short-interfering RNA (siRNA), double-stranded RNA (dsRNA), micro-RNA (miRNA), a short-stranded hairpin RNA (shRNA) molecule that can mediate RNA interference (RNAi) to a target nucleic acid sequence, and chemically modified low molecular weight nucleic acids using the antisense technique, such as the single-stranded antisense oligonucleotide (ASO), the antisense double-stranded DNA oligonucleotide (ADO), the hetero-duplex oligonucleotide (HDO) consisting of an antisense strand of DNA and a complementary strand of RNA, and the single-stranded hetero-duplex oligonucleotide (ss-HDO).

HDO is composed of an antisense strand comprising a nucleotide sequence capable of hybridizing to a target gene or target transcription product and a complementary strand, which is a nucleic acid strand complementary to the antisense strand (also referred to as a "sense strand"). A construct comprising an antisense strand annealed to a complementary strand is referred to as a "hetero-duplex oligonucleotide (HDO" (Patent Literature 1).

An ss-HDO construct is a single-stranded oligonucleotide when it is prepared, and such construct comprises an antisense strand consisting of a DNA nucleotide or DNA nucleotide analog, a linker sequence consisting of 3 to 10 nucleotides, and a sense strand consisting of an RNA nucleotide or RNA nucleotide analog complementary to the antisense strand. The single-stranded oligonucleotide consists of a X-L-Y structure (Patent Literature 2). The X-L-Y structure comprises a nucleotide X serving as an antisense strand, a nucleotide Y serving as a strand complementary to the antisense strand, and a nucleotide L serving as a linker. When such single-stranded oligonucleotide is used as a pharmaceutical composition, single-molecule annealing takes place between an antisense strand and a strand complementary to the antisense strand via a linker in physiological saline, a solvent used for an aqueous injection preparation, a non-aqueous injection preparation, a suspended injection preparation, or a solid injection preparation, the blood, or the plasma to form a double-stranded structure. When such nucleic acid complex acts as a pharmaceutical composition, it undergoes single-molecule annealing to form a double-stranded structure. Thus, this single-stranded oligonucleotide is a type of HDO.

As a technique of delivering a nucleic acid complex to a target organ, a technique of adding a ligand or the like that can be delivered to a target organ to the nucleic acid complex is known. Examples of a ligand or the like include molecules selected from among a lipid, a peptide, and a protein. A lipid can be selected from among cholesterol, fatty acid, fat-soluble vitamin, a glycolipid, and a glyceride. As a ligand or the like, it is also possible to select a ligand reacting with a receptor exposed on the target cell surface (Patent Literature 3).

When cholesterol is used as a ligand or the like, for example, a nucleic acid complex is integrated into a cell via an LDL receptor on a cell surface. Accordingly, a ligand or the like can deliver a nucleic acid complex to, in particular, the liver, which is the organ comprising cells with LDL receptors. However, many cells with LDL receptors are present in organs other than the liver. When an organ other than the liver is targeted, accordingly, a nucleic acid complex is delivered to other organs, disadvantageously. This necessitates examination of safety and side effects in other organs.

The ligand-binding nucleic acid complex used as nucleic acid medicine is required to deliver a nucleic acid complex of interest to an organ. In addition, the ligand-binding nucleic acid complex is required to be integrated into the organ tissue or a cell in the organ to exert antisense effects. When a nucleic acid molecular with a molecular weight larger than that of a ligand is bound, it is difficult to predict as to whether or not the ligand-binding nucleic acid complex would be integrated into a cell. It is accordingly difficult to attain the object with such technique. At present, ligand-binding nucleic acid complexes that can be delivered to various organs are not yet been sufficiently provided. At present, research and development of ligands that can be specifically delivered to target organs have been continuously performed by bioventure and pharmaceutical companies.

The S1P receptor is a G-protein-coupled receptor (GPCR) encoded by the endothelial cell differentiation gene (EDG) (Non-Patent Literature 1). GPCR was initially referred to as an endothelial cell differentiation gene (EDG) receptor, and it was then referred to as a sphingosine-1-phosphate (S1P) receptor. To date, 5 subtypes thereof from S1P1 (sphingosine-1-phosphate receptor 1) to S1P5 (sphingosine-1-phosphate receptor 5) have been found (Non-Patent Literature 2).

Sphingosine-1-phosphate (S1P) existing in a body as a type of lysophospholipid is generated from sphingosine by the action of sphingosine kinase (SphK), a phosphatase, when bound to the S1P receptor (also referred to as the "EDG receptor"). S1P plays a key role as a lipid mediator in a body.

As endogenous ligands reacting with the S1P receptors, sphingosine analogs (S1P analogs) are known. Examples include myriocin, sphingosine, sphingosine-1-phosphate, fingolimod (FTY720), and the (S)-enantiomer of FTY720 phosphate ((S)-FTY720-P) (Non-Patent Literature 3). Examples of known sphingosine analogs include VPC22041, VPC22051, VPC22053, VPC22061, VPC22157, VPC22173, VPC22199, VPC22211, VPC22179, VPC22181, VPC23031, VPC23019, VPC23065, VPC23075, VPC23069, and VPC23079 (Non-Patent Literature 4 and Non-Patent Literature 5).

When a sphingosine analog binds to the S1P receptor, conformational changes are induced in the G protein-coupled receptor (GPCR), guanosine diphosphate (GDP) is substituted with guanosine triphosphate (GTP) of the α subunit of a related G protein, and the G protein is then released into the cytoplasm. Thereafter, the α subunit is dissociated from the βγ subunit, and each subunit can then bind to an effector protein. An effector protein activates a secondary messenger that causes a cellular response. In the end, GTP of the G protein is hydrolyzed to GDP, G protein subunits rebind to each other, and the resultant then rebinds to the receptor. Amplification plays a key role in a general GPCR pathway. When a ligand binds to a receptor, many G proteins are activated, and such G proteins can each bind to any of many effector proteins that cause amplified cellular responses. Since each receptor has both tissue specificity and response specificity, the S1P receptor may be able to serve as a good drug target. These sphingosine analogs are known to have immunosuppressive activity, and research and development thereof as immunosuppressive agents or therapeutic agents for multiple sclerosis have been performed. In particular, a low molecular weight compound, fingolimod, is an S1P receptor agonist commercialized as a therapeutic agent for multiple sclerosis in September 2010 in U.S.A. and in November 2011 in Japan (Patent Literature 5).

However, no attempt had been made to regulate expression or editing of a target gene or a transcription or translation product thereof with the aid of a nucleic acid molecule by binding a ligand reacting with the S1P receptor (hereafter referred to as the "S1P ligand") to a nucleic acid molecule that regulates expression or editing of a target gene or a transcription or translation product thereof, and integrating a nucleic acid molecule comprising an antisense strand to the target gene or a transcription product thereof into tissue or a cell using the S1P ligand so as to deliver the sphingosine analogs to the organ, tissue, or cells.

### Citation List

### Patent Literature

Patent Literature 1: International Publication No. WO 2013/089283
Patent Literature 2: International Publication No. WO 2017/131124
Patent Literature 3: International Publication No. WO 2013/089283
Patent Literature 4: US Patent No. 7,064,217
Patent Literature 5: US Patent No. 7,638,637

### Non Patent Literature

Non Patent Literature 1: M. J. Lee et al., Science, 1998, Vol. 279, pp. 1552-1555
Non Patent Literature 2: (a) Kon, J.; Sato, K.; Watanabe, T.; Tomura, H.; Kuwabara, A.; Kimura, T.; Tamama, K.; Ishizuka, T.; Murata, N.; Kanda, T.; Kobayashi, I.; Ohta, H.; Ui, M.; Okajima, F. J. Biol. Chem., 1999, 274, 23940-23947
Non Patent Literature 3: Yamanashi Medical Journal, 30 (1), 1-6, 2015
Non Patent Literature 4: Mol. Ther., Nucleic Acids, 2013, Jan 22 (1): e66.doi:10.1038/mtna.2012.58.
Non Patent Literature 5: Jpn. J. Clin. Immunol., 32 (2), 92 to 101, 2009

### Summary of Invention

### Technical Problem

The S1P receptor is a membrane protein encoded by the S1P gene, which is the endothelial cell differentiation gene (EDG), it is a G-protein-coupled receptor (GPCR), and it has 7 transmembrane helices. The S1P receptor is likely to be expressed at particularly high levels in the podocyte, the lung, the lymphocyte, the kidney, and the vascular endothelial cell. The S1P receptor is also expressed in other organs, such as the skeletal muscle, the cardiac muscle, the liver, the fat, and the mammary gland.

The present inventors discovered that a nucleic acid complex comprising, bound thereto, a ligand reacting with the S1P receptor expressed in the human or mouse kidney, podocyte, lung, lymphocyte, skeletal muscle, cardiac muscle, or liver tissue (hereafter, referred to as the "S1P ligand") would enable delivery of the nucleic acid complex comprising the S1P ligand bound thereto to an organ expressing the S1P receptor and inhibit expression of the target gene. This has led to the completion of the present invention.

### Solution to Problem

The present inventors had conducted studies on a ligand reacting with the S1P receptor. As a result, they discovered that the ligand-binding nucleic acid complex of the present invention would be delivered to a target organ and expression or editing of a target gene or a transcription or translation product thereof would be regulated. The ligand-binding nucleic acid complex of the present invention can be used for treatment of a disease in an organ comprising a cell expressing the S1P receptor.

Specifically, the present invention is as described below.

[1] A ligand-binding nucleic acid complex in which a nucleic acid molecule that regulates expression or editing of a target gene or a transcription or translation product thereof binds to a compound represented by a formula below or a pharmaceutically acceptable salt with or without a linker: 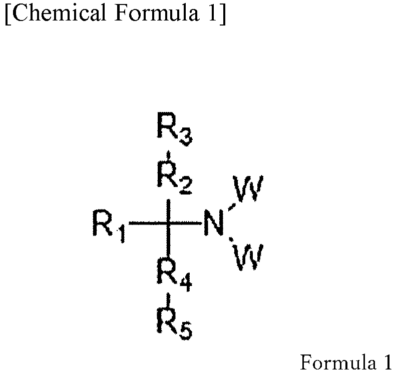 wherein
R₁ represents a C₆-C₅₀ linear or branched carbon chain, and the carbon chain may comprise at least one partial structure, bond, or hetero atom selected from among a double bond, a triple bond, carbocyclic Z, O, S, and N, and/or carbonyl and at least one Y as a substituent;
R₂ and R₄ each represent (CH₂)ₘ;
m is an integer of 0 to 4;
R₃ and R₅ are each selected from the group consisting of -H, -X-, -XW, -P(=X)(-XW)₂, and -XP(=X) (-XW)₂;
X is selected from the group consisting of O, S, and NH;
Y is selected from the group consisting of alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, alkyl halide, hydroxy, carboxy, sulfonic acid, sulfonic acid derivative, azide, and thiol;
Z represents a C₁-C₃₀ linear or branched carbon chain, the carbon chain is selected from the group consisting of cycloalkylalkyl, arylalkyl, heterocycloalkylalkyl, and heteroarylalkyl, and the carbon chain may comprise a bond or hetero atom selected from among a double bond, a triple bond, O, S, N, sulfinyl, sulfonyl, and/or carbonyl and at least one Y as a substituent; and
W independently represents H or a C₁-C₆₀ linear or branched carbon chain, and the carbon chain may comprise at least one partial structure, bond, or hetero atom selected from among a double bond, a triple bond, carbocyclic Z, O, S, and N, and/or carbonyl and at least one Y as a substituent.

[2] The compound and the pharmaceutically acceptable salt according to [1], wherein, in Formula 1,
R₁ represents a C₆-C₅₀ linear or branched carbon chain, and the carbon chain may comprise at least one partial structure, bond, or hetero atom selected from among a double bond, a triple bond, carbocyclic Z, O, and N, and/or carbonyl and at least one Y as a substituent;
R₂ and R₄ each represent (CH₂)ₘ;
m is an integer of 0 to 4;
R₃ and R₅ are each selected from the group consisting of -H, -X-, -XW, and -XP(=X) (-XW)₂;
X is selected from the group consisting of O and S;
Y is selected from the group consisting of alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, alkyl halide, hydroxy, carboxy, sulfonic acid, azide, and thiol;
Z represents a C₁-C₃₀ linear or branched carbon chain, the carbon chain is selected from the group consisting of arylalkyl and heteroarylalkyl, and the carbon chain may comprise a bond or hetero atom selected from among a double bond, a triple bond, O, S, N, sulfinyl, sulfonyl, and/or carbonyl and at least one Y as a substituent; and
W independently represents H or a C₁-C₆₀ linear or branched carbon chain, and the carbon chain may comprise at least one partial structure, bond, or hetero atom selected from among a double bond, a triple bond, carbocyclic Z, O, S, and N, and/or carbonyl and at least one Y as a substituent.

[3] The compound and the pharmaceutically acceptable salt according to [1], wherein, in Formula 1,
R₁ represents a C₆-C₅₀ linear or branched carbon chain, and the carbon chain may comprise at least one partial structure, bond, or hetero atom selected from among a double bond, a triple bond, carbocyclic Z, O, and S, and/or carbonyl and at least one Y as a substituent;
R₂ and R₄ each represent (CH₂)ₘ;
m is an integer of 0 to 4;
R₃ and Rs are each selected from the group consisting of -H, -X-, -XW, and -XP(=X) (-XW)₂;
X is selected from the group consisting of O and S;
Y is selected from the group consisting of alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, alkyl halide, hydroxy, carboxy, and azide;
Z represents a C₁-C₃₀ arylalkyl, and the arylalkyl chain may comprise a bond or hetero atom selected from among a double bond, a triple bond, O, S, N, sulfinyl, and carbonyl and at least one Y as a substituent; and
W independently represents H or a C₁-C₅ linear or branched group selected from the group consisting of alkyl, acyl, and alkoxy.

[4] The compound and the pharmaceutically acceptable salt according to [1], which are (R)-1 -azide-17-((4-decylphenyl)carbamoyl)-15 -oxo-3,6,9,12-tetraoxa-16-azaoctadeca-18-nyl phosphate or (R)-2-amino-3-((4-decylphenyl)amino)-3-oxopropyl(14-azide-3,6,9,12-tetraoxatetradecyl)phosphate.
[5] The compound and the pharmaceutically acceptable salt according to [1], which are 1-azide-17-(hydroxymethyl)-17-(4-octylphenethyl)-15-oxo-3,6,9,12-tetraoxa-16-azaoctadeca-18-nyl phosphate or 2-amino-2-(hydroxymethyl)-4-(4-octylphenyl)butyl(14-azide-3,6,9,12-tetraoxatetradecyl)phosphate.
[6] The compound and the pharmaceutically acceptable salt according to [1], wherein the compound represented by Formula 1 or a conjugate of the compound represented by Formula 1 and a linker is selected from among compounds having structures shown below.

| Compound No. | Structural formula |
|---|---|
| RN01-N-PEG4-azide | 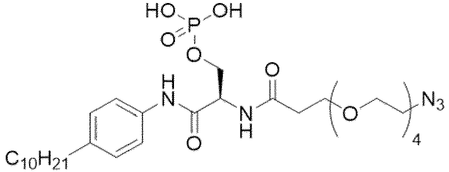 |
| RN01-P-PEG4-azide | 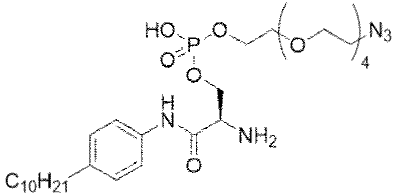 |
| RN02-N-PEG4-azide | 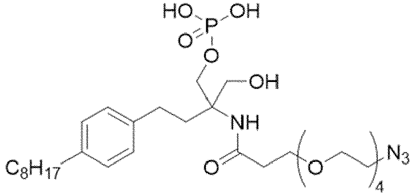 |
| RN02-P-PEG4-azide | 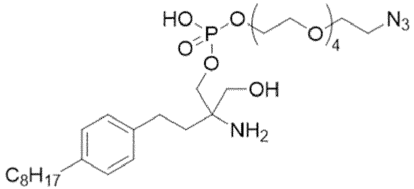 |
| RN01-N-PEG1-azide | 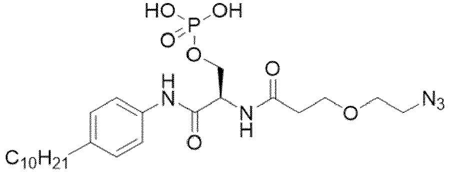 |
| RN01-N-PEG9-azide | 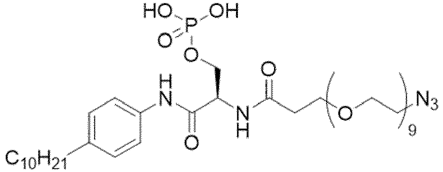 |
| RN01-N-C10-azide | 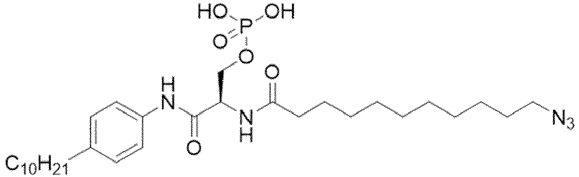 |
| RN01-N-PEG4-maleimide | 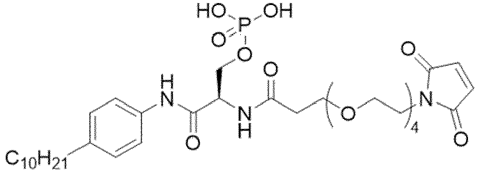 |
| RN01-P-amidite | 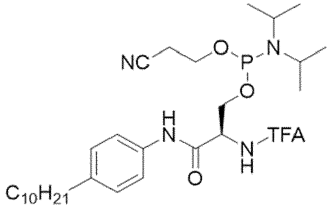 |
| RN01-N-PEG4SPDP | 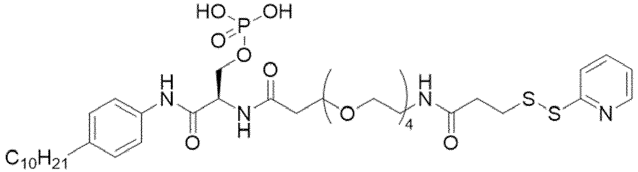 |

[7] The ligand-binding nucleic acid complex according to any of [1] to [6], wherein the nucleic acid binds to the compound represented by Formula 1 or a pharmaceutically acceptable salt via a linker.
[8] The ligand-binding nucleic acid complex according to [7], wherein the linker is a cleavable linker having a cleavable structure.
[9] The ligand-binding nucleic acid complex according to [8], wherein the linker has the structure shown below.

| Linker type | Structural formula |
|---|---|
| Having a disulfide bond | 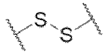 |
| Having an amide bond (n=0-9) | 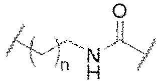 |
| Having a polyethylene glycol group (n=1-30) | 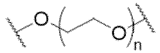 |
| Having a phosphate group | 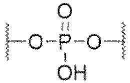 |
| Having a pyrrolidinyl group | 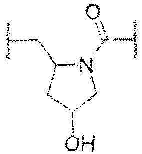 |
| Having a BCN group | 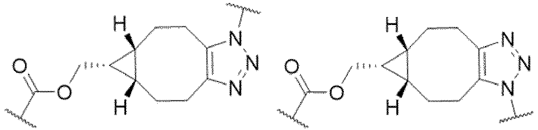 |
| Having a DBCO group | 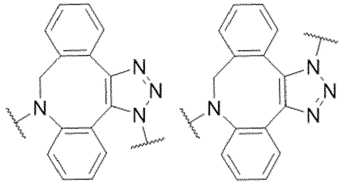 |
| Having a maleimide thioether group | 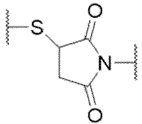 |

[10] The ligand-binding nucleic acid complex according to [1], wherein the nucleic acid molecule is selected from among a nucleic acid molecule comprising an antisense strand consisting of an oligonucleotide having a nucleic acid sequence complementary to a target gene or a transcription product thereof, an aptamer comprising a nucleic acid sequence binding specifically to a target protein, and a decoy consisting of an oligonucleotide having a nucleic acid sequence complementary to a target transcription factor.
[11] The ligand-binding nucleic acid complex according to [1], wherein the nucleic acid molecule is selected from among ADO, ASO, HDO, and RNAi.
[12] The ligand-binding nucleic acid complex according to [11], wherein the antisense strand of the nucleic acid molecule consists of 12 to 30 continuous nucleotides.
[13] The ligand-binding nucleic acid complex according to [9], wherein the nucleic acid molecule is a decoy comprising a nucleic acid sequence complementary to a target transcription factor and consisting of 8 to 30 nucleotides.
[14] The ligand-binding nucleic acid complex according to [10], wherein the oligonucleotide is siRNA consisting of an antisense strand consisting of RNA and a nucleic acid strand complementary to the antisense strand.
[15] A ligand-binding nucleic acid complex, wherein a nucleic acid molecule that regulates expression or editing of a target gene or a transcription or translation product thereof binds to a ligand reacting with an S1P receptor.
[16] The ligand-binding nucleic acid complex according to [15], wherein the nucleic acid molecule that regulates expression or editing of a target gene or a transcription or translation product thereof binds to the ligand reacting with an S1P receptor via a linker.
[17] The ligand-binding nucleic acid complex according to [16], wherein the linker is a cleavable linker having a cleavable structure.
[18] The ligand-binding nucleic acid complex according to [15], wherein the nucleic acid molecule is selected from among a nucleic acid molecule comprising an antisense strand consisting of an oligonucleotide having a nucleic acid sequence complementary to a target gene or a transcription product thereof, an aptamer comprising a nucleic acid sequence binding specifically to a target protein, and a decoy consisting of an oligonucleotide having a nucleic acid sequence complementary to a target transcription factor.
[19] The ligand-binding nucleic acid complex according to [18], wherein the nucleic acid molecule is selected from among ASO, HDO, and RNAi.
[20] The ligand-binding nucleic acid complex according to [15], wherein the nucleic acid strand of the nucleic acid molecule comprises nucleotides, modified nucleotides, and/or nucleotide analogs.
[21] The ligand-binding nucleic acid complex according to [20], wherein the total number of nucleotides, modified nucleotides, and nucleotide analogs in the antisense strand of the nucleic acid molecule is 12 to 30.
[22] The ligand-binding nucleic acid complex according to [20], wherein the nucleic acid molecule is HDO and the total number of nucleotides, modified nucleotides, and nucleotide analogs in the antisense strand and that in the complementary strand of HDO are each 12 to 30.
[23] The ligand-binding nucleic acid complex according to [15] or [16], wherein the nucleic acid molecule is HDO comprising an antisense strand consisting of an oligonucleotide having a nucleic acid sequence complementary to a target gene or transcription product thereof and a complementary strand having a nucleic acid sequence complementary to the antisense strand, and the ligand reacting with the S1P receptor binds to the complementary strand of HDO.
[24] The ligand-binding nucleic acid complex according to [15], wherein the nucleic acid molecule is a decoy having a nucleic acid sequence complementary to a target transcription factor and consisting of 8 to 30 nucleotides.
[25] The ligand-binding nucleic acid complex according to [20], wherein the antisense strand is a gapmer and consists of a gap region comprising nucleotides and/or modified nucleotides and a wing region (or wing regions) comprising one or a plurality of nucleotide analogs and/or modified nucleotides provided on the 5' terminal side and/or 3' terminal side thereof.
[26] The ligand-binding nucleic acid complex according to [20], wherein the antisense strand is a non-gapmer and comprises nucleotides, modified nucleotides, and/or nucleotide analogs.
[27] The ligand-binding nucleic acid complex according to [22], wherein the complementary strand comprises nucleotides, modified nucleotides, and/or nucleotide analogs.
[28] The ligand-binding nucleic acid complex according to [27], wherein the complementary strand consists of a center region comprising nucleotides and/or modified nucleotides and a wing region (or wing regions) comprising one or a plurality of nucleotide analogs and/or modified nucleotides provided on the 5' terminal side and/or 3' terminal side thereof.
[29] The ligand-binding nucleic acid complex according to [20], wherein the modified nucleotides are nucleotides comprising a 2'-O-CH₃ group or a 2'-O-CH₂CH₂OCH₃ (MOE) group.
[30] The ligand-binding nucleic acid complex according to [20], wherein the nucleotide analogs include bridged nucleotides selected independently from among LNA, cEt-BNA, amide BNA (AmNA), and cMOE-BNA.
[31] The ligand-binding nucleic acid complex according to [20], wherein the nucleotide analogs include bridged nucleotides selected independently from among LNA, cEt-BNA, amide BNA (AmNA), and cMOE-BNA.
[32] The ligand-binding nucleic acid complex according to [20], wherein the nucleotide analogs are selected independently from among PNA, GNA, TNA, tcDNA, morpholino nucleic acid, and BNA.
[33] The ligand-binding nucleic acid complex according to [20], wherein at least one nucleotide or modified nucleotide in the nucleic acid molecule is phosphorothioated or boranophosphated.

The description incorporates the contents disclosed by JP Patent Application No. 2021-091589, based on which the priority of the present application claims.

### Advantageous Effects of Invention

The ligand-binding nucleic acid complex of the present invention can be used for treatment of diseases of organs comprising S1P receptor-expressing cells.

The ligand-binding nucleic acid complex of the present invention enables organ- or cell-specific delivery with the use of an S1P ligand, and a nucleic acid molecule enables regulation of expression or editing of a target gene or a transcription or translation product thereof. The nucleic acid complex targeting a disease-causing gene or a transcription or translation product thereof can be used as a therapeutic agent for a disease of a particular organ. Brief Description of Drawings

[Figure 1] Figure 1A shows expression of the target transcription product, Malat-1, inhibited by the S1P ligand-binding HDO (RN01-N-SP01-HDO06) and Figure 1B shows expression of the target transcription product, Malat-1, inhibited by the S1P ligand-binding HDO (RN01-P-SP01-HDO06).
[Figure 2] Figure 2 shows changes in animal body weight between before and after administration of the S1P ligand-binding HDO.
[Figure 3] Figure 3A and Figure 3B each show changes in ALT and AST activity in blood 3 days after administration of the S1P ligand-binding HDO to mice.
[Figure 4] Figure 4 shows changes in Malat1 ncRNA expression levels in the mouse liver after administration of the S1P ligand-binding HDO.
[Figure 5] Figure 5 shows changes in Malat1 ncRNA expression levels in the mouse lung after administration of the S1P ligand-binding HDO.
[Figure 6] Figure 6 shows changes in Malat1 ncRNA expression levels in the mouse kidney after administration of the S1P ligand-binding HDO.
[Figure 7] Figure 7 shows changes in animal body weight between before and after administration of the S1P ligand-binding HDO.
[Figure 8A] Figure 8A shows changes in ALT in blood 3 days after administration of the S1P ligand-binding HDO to mice.
[Figure 8B] Figure 8B shows changes in AST activity in blood 3 days after administration of the S1P ligand-binding HDO to mice.
[Figure 9] Figure 9 shows changes in Malat1 ncRNA expression levels in the mouse liver after administration of the S1P ligand-binding HDO.
[Figure 10] Figure 10 shows changes in Malat1 ncRNA expression levels in the mouse kidney after administration of the S1P ligand-binding HDO.
[Figure 11] Figure 11 shows changes in Malat1 ncRNA expression levels in the mouse lung after administration of the S1P ligand-binding HDO.
[Figure 12A] Figure 12 A shows changes in Malat1 ncRNA expression levels in the mouse skeletal muscle after administration of the S1P ligand-binding HDO.
[Figure 12B] Figure 12 B shows changes in Malat1 ncRNA expression levels in the mouse liver after administration of the S1P ligand-binding HDO.
[Figure 12C] Figure 12 C shows changes in Malat1 ncRNA expression levels in the mouse lung after administration of the S1P ligand-binding HDO.
[Figure 12D] Figure 12D shows changes in Malat1 ncRNA expression levels in the mouse kidney after administration of the S1P ligand-binding HDO.
[Figure 13] Figure 13 shows changes in Malat1 ncRNA expression levels in the mouse skeletal muscle after administration of the S1P ligand-binding HDO.
[Figure 14] Figure 14 shows changes in Malat1 ncRNA expression levels in the mouse skeletal muscle after administration of the S1P ligand-binding HDO.
[Figure 15] Figure 15 shows changes in Malat1 ncRNA expression levels in the mouse skeletal muscle after administration of the S1P ligand-binding HDO.
[Figure 16] Figure 16 shows changes in Malat1 ncRNA expression levels in the mouse skeletal muscle after administration of the S1P ligand-binding HDO.
[Figure 17] Figure 17 shows changes in Malat1 ncRNA expression levels in the mouse skeletal muscle after administration of the S1P ligand-binding HDO.
[Figure 18] Figure 18 shows changes in Malat1 ncRNA expression levels in the mouse skeletal muscle after administration of the S1P ligand-binding HDO.
[Figure 19] Figure 19 shows changes in Malat1 ncRNA expression levels in the mouse skeletal muscle after administration of the S1P ligand-binding HDO.

### Description of Embodiments

The present invention relates to a ligand-binding nucleic acid complex in which a ligand binds to a nucleic acid complex. The ligand binds to the nucleic acid complex indirectly via a linker or directly without a linker.

### 1. Nucleic acid complex

In the present invention, a "nucleic acid complex" is a nucleic acid molecule that regulates expression or editing of a target gene or a transcription or translation product thereof. An example of the nucleic acid molecule is a nucleic acid molecule comprising a nucleic acid sequence complementary to that of a target gene or a transcription product thereof and having antisense activity. Specific examples of the nucleic acid molecules include a single-stranded antisense strand (single-stranded antisense oligonucleotide, ASO), miRNA, anti-miR, RNA interference (RNAi), short interference RNA (siRNA), short hairpin RNA (shRNA), antisense double-stranded nucleic acid (antisense double-stranded DNA oligonucleotide, ADO), and a hetero-duplex nucleic acid (hetero-duplex oligonucleotide, HDO).

Another example of the nucleic acid molecule is an aptamer having high specificity and high binding affinity to a target molecule, such as a protein. Another example of the nucleic acid molecule is a decoy. A decoy acts as follows. When a transcription factor binds to a binding site of a particular transcription regulator, such as a promoter of a gene, the gene is activated and gene functions are turned on or off by the transcription regulator under ordinary circumstances. However, a decoy hybridizes to the transcription factor and inhibits inherent functions of the transcription factor. A further example of the nucleic acid molecule is a nucleic acid molecule that binds specifically to a particular target molecule in a cell, which is a bait that modifies functions of the target molecule.

A "target gene" is a gene to which the antisense strand of the nucleic acid complex of the present invention can bind.

A target gene is not particularly limited, provided that it is expressed *in vivo.* An example of a target gene is an organism-derived gene into which the nucleic acid complex of the present invention is to be introduced, such as a gene, the expression level of which is elevated in the case of various diseases. Examples thereof include the Indian Hedgehog gene, the interferon gene, the apolipoprotein B gene, the Huntington's gene, the dystrophin gene, the dystrophia myotonica-protein kinase (DMPK) gene, and the metastasis-associated lung adenocarcinoma transcript product 1 (Malat1) gene.

The Indian Hedgehog (IHH) is a secretory protein of the hedgehog family. It is known to be located downstream of the transcription factor TAZ and worsen NASH fibrosis.

The cytokine interleukin 1 (IL-1) gene is known to cause diseases of chronic inflammation. A treatment strategy in which skipping of exon 9 encoding the transmembrane domain of IL-1RAcP, which is pre-mRNA, aimed at substantial inhibition of IL-1 signal transmission has been reported (Mol. Ther. Nucleic Acids, 2013, Jan, 22 (1): e66.doi:10.1038/mtna.2012.58.).

The apolipoprotein B gene, ApoB-100, is known to cause an inherited metabolic disorder; i.e., familial hypercholesterolemia, and nucleic acid medicine (mipomersen) was commercialized in 2013 in U.S.A.

Huntington's disease is inherited in an autosomal dominant manner, and it is a chronic progressive neurodegenerative disease with presenting symptoms characterized by involuntary choreic movement, mental symptoms, and dementia. The short arm of chromosome 4 (4p16.3) of the Huntington gene is known to be a causal gene of Huntington's disease.

Duchenne muscular dystrophy is muscular dystrophy caused by mutations in the dystrophin gene, and it is known to be inherited in an X-linked recessive manner and predominantly found in male children. Exon skipping comprising skipping over exons with abnormalities in the dystrophin gene is effective for treatment of Duchenne muscular dystrophy.

The DMPK gene encodes myotonin protein kinase, and it is known as a causal gene of myotonic dystrophy that is developed most frequently in adults among various types of muscular dystrophies.

Malat1 is a long-chain non-coding RNA (lncRNA) expressed at high levels in the case of malignant tumors including lung cancer, and it is known to remain in the nuclei of muscle cells.

The "target transcription product" is any RNA that serves as a direct target of the nucleic acid complex of the present invention and is synthesized by RNA polymerase. The "transcription product of the target gene" is equivalent to the "target transcription product." Specific examples include mRNA transcribed from the target gene (including mature mRNA, an mRNA precursor, and RNA with unmodified bases), non-coding RNA (ncRNA) such as miRNA, long non-coding RNA (lncRNA), and natural antisense RNA. Examples of target transcription products include pre-mRNA, which is a transcription product of the IL-1RAcP gene, mRNA, which is a transcription product of the ApoB-100 gene, mRNA, which is a transcription product of the IHH gene, pre-mRNA, which is a transcription product of the dystrophin gene, DMPK mRNA, which is a transcription product of the DMPK gene, and Malat1 non-coding RNA (ncRNA), which is a transcription product of the Malat1 gene.

The "target translation product" is a protein that is synthesized by catalyzing a reaction in which, among mRNAs as transcription products, mRNA other than non-coding RNA is used as a template, and ribosomes ligate amino acids delivered by transfer RNA in accordance with codons to form a peptide chain. The "translation product of the target gene" and the "translation product of the target transcription product" are equivalent to the "target translation product."

The "aptamer" is a nucleic acid molecule that binds specifically to a target translation product, such as a particular target molecule in a cell, on a cell membrane, or outside a cell, such as on a cell membrane or outside a cell. The aptamer is either the DNA or RNA aptamer, and it can be prepared by a method known in the art, such as *in vitro* selection performed with the use of the systematic evolution of ligands by exponential enrichment (SELEX) procedure. The nucleic acid base length of an aptamer is not particularly limited, and it is in the range of 10 to 70 bases and preferably in the range of 20 to 50 bases.

The "decoy" is a nucleic acid having the sequence of the binding site of a transcription factor (e.g., NF-kB) or a sequence similar thereto. When a "decoy" is introduced into a cell, it inhibits the action of the transcription factor (e.g., a decoy inhibits transcription when a transcription factor is a transcription activator and it promotes transcription when a transcription factor is a transcription inhibitor). A decoy nucleic acid can be easily designed based on information on a binding sequence of a target transcription factor. The base length of the decoy nucleic acid is not particularly limited, and it is in the range of 8 to 30 bases and preferably in the range of 10 to 25 bases.

The "nucleic acid" or "nucleic acid molecule" is a nucleoside or nucleotide in the case of a monomer, an oligonucleotide in the case of an oligomer, and a polynucleotide in the case of a polymer. The term "nucleic acid strand" is used to indicate an "oligonucleotide" herein. A nucleic acid strand may be prepared entirely or partially by a method of chemical synthesis performed with the use of an auto synthesizer, or it may be prepared by enzymatic treatment with the use of a polymerase, a ligase, or a restriction enzyme, although a method of preparation is not limited thereto.

The "nucleoside" is a molecule composed of bases and sugars, in general. A sugar portion of a nucleoside is not limited. In general, it is composed of pentofuranosyl sugars. Specific examples thereof include ribose and deoxyribose. In general, a base portion (nucleic acid bases) of a nucleoside is a heterocyclic base portion. Examples thereof include, but are not limited to, adenine, cytosine, guanine, thymine, uracil, and other modified nucleic acid bases (modified bases).

The "nucleotide" is a molecule composed of a sugar portion of the nucleoside comprising a phosphoric acid group covalently bound thereto. In the case of a nucleotide comprising pentofuranosyl sugar, in general, a phosphoric acid group is ligated to a hydroxyl group at position 2', 3', or 5' of the sugar.

The "oligonucleotide" is a linear oligomer formed by covalent binding of several to dozens of hydroxyl groups and phosphoric acid groups in sugar portions between adjacent nucleotides. The "polynucleotide" is a polymer comprising the number of nucleotides that is larger than the number of nucleotides constituting an oligonucleotide, and it is formed by covalent binding of dozens or more, and preferably hundreds or more nucleotides. In general, phosphoric acid groups seem to form the internucleoside bond in the oligonucleotide or polynucleotide structure.

The "antisense technique" that utilizes an antisense strand is characterized in that a nucleic acid molecule having an antisense strand hybridizes to a target nucleic acid and regulates the amount, activity, and/or functions of the target nucleic acid. In some embodiments, for example, a nucleic acid molecule having an antisense strand changes transcription or translation of the target. Such expression can be regulated by, for example, target mRNA degradation or occupancy-based inhibition. An example of regulation of RNA target functions by degradation is degradation of target RNA by RNase H at the time of hybridization to a nucleic acid molecule having a DNA-like antisense strand. An antisense strand (ASO) consisting of DNA constituted to be hybridize to target RNA hybridizes to target RNA to form a double strand, and it is then degraded by RNase H. By repeating such procedure, target RNA is decreased in a cell, target RNA expression is inhibited, and target RNA activity is inhibited. Antisense effects thus attained are referred to as "RNase H-dependent antisense effects." In some embodiments, a nucleic acid molecule having an antisense strand exerts, as RNase H-independent antisense effects, effects of converting splicing functions, such as inhibition of transcription or translation or exon skipping of target RNA. In such a case, ASO hybridizes to target RNA to inhibit or promote expression of the target gene. Another example of RNase H-independent antisense effects is RNA interference (RNAi). RNAi is antisense-mediated gene silencing that uses an RNA-induced silencing complex (RISC). Examples of RNAi include siRNA, shRNA, and miRNA. Such antisense effects are referred to as "RNAi-dependent antisense effects."

The "antisense effects" are effects of regulating expression or editing of a target gene or a transcription product thereof attained when an antisense strand of a nucleic acid molecule hybridizes to the target gene or a transcription product thereof (an RNA sense strand).

A nucleic acid molecule "that regulates expression or editing of a target gene or a transcription or translation product thereof" inhibits, lowers, or enhances expression of the target gene or the expression level of the target transcription product ("the expression level of the target transcription product" is often referred to as "the target transcription product level" herein), inhibits translation, inhibits function of the translation product, controls RNA splicing (e.g., splicing switch, exon inclusion, and exon skipping), or inhibits degradation of the transcription product or binding of the target gene to a protein.

In some embodiments, the term "antisense effects" refers to effects of converting splicing functions, such as inhibition of translation or exon skipping that can occur by covering the transcription product by hybridization, and/or the inhibition that can occur upon degradation of the transcription product when the hybridized region is recognized. Upon exon inclusion into a target gene or a transcription product, some exons are excluded from mRNA due to gene abnormalities, unlike the case of exon skipping, such exons are included into mRNA by the antisense activity, such inclusion leads to enhanced expression of normal mRNA, and such enhanced expression is referred to as "antisense effects" in other embodiments.

In the case of post-transcriptional inhibition of the target gene, for example, an RNA oligonucleotide is introduced into a cell as ASO, and ASO then forms a partial double strand by annealing to mRNA, which is transcription product of a target gene. This partial double strand serves as a cover to block translation by a ribosome, and expression of the target protein encoded by the target gene is inhibited at the translation level (steric blocking). When an oligonucleotide comprising DNA is introduced into a cell as ASO, a partial DNA-RNA hetero-duplex is formed. When the hetero-duplex structure is recognized by RNase H, mRNA of the target gene is degraded, and expression of the protein encoded by the target gene is inhibited at the expression level. The antisense effects can also be attained by targeting the intron in the mRNA precursor. The antisense effects can also be attained by targeting miRNA. In such a case, inhibition of miRNA functions would lead to enhanced expression of a gene that is generally regulated to express by the miRNA. In an embodiment, regulation of expression of the target transcription product may be reduction in the amount of the target transcription product.

When ADO is used as a nucleic acid molecule having an antisense strand, for example, a DNA double strand is cleaved by DNA nuclease (DNase) in a cell, the DNA antisense strand hybridizes to target RNA to form a double strand, and the target RNA is then degraded by RNase H. By repeating such procedure, expression of the target RNA is inhibited, and action of the target RNA is inhibited. In another embodiment, a DNA double strand of ADO is cleaved by DNA nuclease (DNase), the DNA antisense strand hybridizes to target RNA, and expression of the target gene is inhibited or enhanced by regulation of RNA splicing, such as inhibition of transcription or translation or exon skipping of the target RNA.

When HDO is used as a nucleic acid molecule having an antisense strand, for example, a complementary strand consisting of RNA of HDO is cleaved by RNase H in a cell, the DNA antisense strand hybridizes to target RNA to form a double strand, and target RNA is then degraded by RNase H. By repeating such procedure, expression of the target RNA is inhibited, and action of the target RNA is inhibited. Alternatively, an RNA complementary strand of HDO is cleaved by RNase H in a cell, the DNA antisense strand hybridizes to target RNA, and expression of the target gene is inhibited or enhanced by regulation of RNA splicing, such as inhibition of transcription or translation or exon skipping of the target RNA.

When RNA interference (RNAi) is used as a nucleic acid molecule having an antisense strand, for example, RNAi is antisense-mediated gene silencing based on the mechanism involving the use of an RNA-induced silencing complex (RISC). Examples of RNAi include siRNA and shRNA. In another embodiment, functions of target RNA are regulated by the occupancy-based mechanism, such as a mechanism that is naturally used by microRNA. microRNA is small non-coding RNA that regulates expression of RNA that encodes a protein. When a nucleic acid molecule having an antisense strand binds to microRNA, binding of the microRNA to the messenger RNA target is inhibited, and functions of microRNA are thus interfered. A microRNA mimic can enhance inherent microRNA functions. A nucleic acid molecule having a particular antisense strand changes pre-mRNA splicing. Regardless of a particular mechanism, sequence specificity enables use of a nucleic acid molecule having an antisense strand as a tool for target examination and gene functionalization and as a therapeutic agent that selectively regulates expression of a gene related to a cause of the disease.

The length of an antisense strand is not particularly limited, and an antisense strand comprises at least 8 bases, such as 8 to 40, preferably 12 o 30, and more preferably 12 to 25 or 13 to 20 bases. In some embodiments, a chain length is selected in accordance with other factors, such as the level of antisense effects of the nucleic acid strand on the target, the cost, and the synthetic yield, in general. In the case of a double-stranded nucleic acid, a chain length may be selected to adjust Tm of the double strand preferably to 50°C or higher, and more preferably to 60°C or higher.

In the case of a double-stranded nucleic acid, the length of a complementary strand may be the same as that of the antisense strand. In such a case, the complementary strand comprises at least 8 bases, such as 8 to 40, preferably 12 o 30, and more preferably 12 to 25 or 13 to 20 bases. The complementary strand may be longer or shorter than the antisense nucleic acid strand by several to dozen nucleotides.

The term "complimentary" used herein refers to a correlation such that a so-called Watson-click base pairing (natural base pairing) or non-Watson-click base pairing (Hoogsteen base pairing or the like) can be formed via hydrogen bond. When a sufficient number of nucleic acid bases in an antisense strand can form hydrogen bonds with corresponding nucleic acid bases in the target nucleic acid or target transcription product, desirable effects are achieved because the antisense strand is complementary to the target nucleic acid or target transcription product. Nucleic acid bases that are noncomplementary between the antisense strand and the target nucleic acid or target transcription product are acceptable, provided that the antisense strand can specifically hybridize to the target nucleic acid. In addition, an antisense strand can hybridize to one or more segments of the target nucleic acid or target transcription product. Thus, intervening or adjacent segments are not involved in hybridization events (e.g., a loop, mismatch, or hairpin structure). The antisense strand is complementary to the sequence of the target nucleic acid or target transcription product. At a sufficient extent of complementarity, an antisense strand can bind to the target nucleic acid or target transcription product. For example, an extent of complementarity may be 80% or higher, 90% or higher, 95% or higher, 96% or higher, 97% or higher, 98% or higher, or 99% or higher. An extent of complementarity may be 100%. There may be approximately 0 to 4 mismatches.

In a particular embodiment, "the nucleic acid complex" of the present invention may be a single-stranded oligonucleotide when it is prepared, and the nucleic acid complex may comprise an antisense strand consisting of a DNA nucleotide or DNA nucleotide analog, a linker sequence consisting of 3 to 10 nucleotides, and a sense strand consisting of an RNA nucleotide or RNA nucleotide analog complementary to the antisense strand. The nucleic acid complex as described above is referred to as a single-stranded hetero-duplex oligonucleotide (ss-HDO), which is an oligonucleotide consisting of a X-L-Y structure (Patent Literature 4). The X-L-Y structure comprises a nucleotide X serving as an antisense strand, a nucleotide Y serving as a strand complementary to the antisense strand, and a nucleotide L serving as a linker. When such single-stranded oligonucleotide is used as a pharmaceutical composition, single-molecule annealing takes place between an antisense strand and a strand complementary to the antisense strand via a linker in physiological saline, a solvent used for an aqueous injection preparation, a non-aqueous injection preparation, a suspended injection preparation, or a solid injection preparation, the blood, or the plasma to form a double-stranded structure. When such nucleic acid complex acts as a pharmaceutical composition, it undergoes single-molecule annealing to form a double-stranded structure. Thus, it is a duplex oligonucleotide complex.

In several embodiments described above, preferable representative examples of single-stranded ASOs and duplex nucleic acid complexes were described. It should be noted that single-stranded ASOs and duplex nucleic acid complexes in several embodiments are not limited to the representative examples above.

In an embodiment, an antisense strand comprises nucleotides, modified nucleotides, and/or nucleotide analogs. The antisense strand may comprise DNA nucleotides and RNA nucleotides, and the nucleic acid strand may further optionally comprise modified nucleotides and nucleotide analogs.

In an embodiment, a complementary strand comprises nucleotides, modified nucleotides, and/or nucleotide analogs.

This indicates that the complementary strand may comprise DNA nucleotides and RNA nucleotides, and the nucleic acid strand may further optionally comprise modified nucleotides and nucleotide analogs.

In an embodiment, a complementary strand comprises a center region comprising nucleotides and/or modified nucleotides and a wing region (or wing regions) comprising one or a plurality of nucleotide analogs and/or modified nucleotides provided on the 5' terminal side and/or 3' terminal side thereof.

The term "DNA nucleotide" used herein refers to a DNA nucleotide that exists in nature or a DNA nucleotide with a modified base, sugar, or phosphate binding subunit.

In the same manner, the term "RNA nucleotide" used herein refers to an RNA nucleotide that exists in nature or an RNA nucleotide with a modified base, sugar, or phosphate binding subunit.

In a "modified nucleotide," a substituent is added to a base, sugar, or phosphate binding subunit of the nucleotide, or one substitution is implemented in a subunit, and the entire subunit is not substituted with different chemical groups. From the viewpoint of high resistance to a DNA-degrading enzyme or the like, DNAs in the entire region comprising nucleotides or a part thereof may be modified nucleotides. Examples of modification include: 5-methylation, 5-fluorination, 5-bromation, 5-iodization, and N4-methylation of cytosine; 5-demethylation, 5-fluorination, 5-bromation, and 5-iodization of thymidine; N6-methylation and 8-bromation of adenine; N2-methylation and 8-bromation of guanine; phosphorothioation, boranophosphatation, methylphosphonation, methylthiophosphonation, chiral-methylphosphonation, phosphorodithioation, phosphoroamidation, 2'-O-methylation, 2'-methoxyethylation (MOE), 2'-aminopropylation (AP), and 2'-fluorination. From the viewpoint of excellent dynamics, phosphorothioation is preferable. Such modification may be provided in combinations of two or more on the same DNA. As described below, an RNA nucleotide may be modified to exert similar effects.

In some embodiments, the number or position of modified nucleotide(s) may affect the antisense effects exerted by the duplex oligonucleotide according to the present invention. While such embodiments vary depending on target gene sequences or other factors, a person skilled in the art may be able to determine the number or position of modified nucleotide(s) with reference to the literatures concerning the antisense method described below. When the assayed antisense effects of the modified duplex nucleic acid complex are not significantly lowered compared with those of the duplex nucleic acid complex before modification (e.g., the assayed value for the modified duplex nucleic acid complex is 30% or more of the assayed value for the duplex nucleic acid complex before modification), such modification can be evaluated effective. The antisense effects can be adequately assayed in the manner described below. For example, a test nucleic acid compound is introduced into a cell, and the target gene expression level (e.g., the mRNA level, the cDNA level, or the protein level) in the cell that is suppressed by the antisense effects exerted by the test nucleic acid compound is then assayed via a conventional technique as described in the examples below, such as Northern blotting, quantitative PCR, or Western blotting.

The "nucleotide analog" is a nucleotide that does not exist in nature. In a base, sugar, or phosphate binding subunit of a nucleotide, two or more substituents are added, two or more substituents in the subunit are substituted, or the entire subunit is substituted with different chemical groups. An example of an analog involving substitution of two or more substituents is a bridged nucleic acid. A bridged nucleic acid is a nucleotide analog comprising a crosslinking unit added thereto on the basis of substitution at 2 sites in a sugar ring. A typical example is a nucleotide analog in which carbon at position 2' is bound to carbon at position 4'. In an embodiment, the first nucleic acid strand further comprises a nucleotide analog to enhance the affinity to a partial sequence of a target gene transcription product and/or resistance to a nucleic acid-degrading enzyme. A "nucleotide analog" may be any nucleotide, provided that its affinity to a partial sequence of a target gene transcription product and/or resistance to a nucleic acid-degrading enzyme are enhanced via modification (e.g., crosslinking or substitution). Examples thereof that are preferably used in the antisense method are disclosed in JP H10-304889 A, WO 2005/021570, JP H10-195098A, JP 2002-521310 A, WO 2007/143315, WO 2008/043753, WO 2008/029619, and WO 2008/049085 (hereafter, such literatures are also referred to as "the literatures concerning the antisense method"). Specific examples include the nucleic acids disclosed in the literatures mentioned above: hexitol nucleic acid (HNA), cyclohexene nucleic acid (CeNA), peptide nucleic acid (PNA), glycol nucleic acid (GNA), threose nucleic acid (TNA), morpholinonucleic acid, tricyclo-DNA (tcDNA), 2'-O-methylated nucleic acid (2'-OMe), 2'-MOE (2'-O-methoxyethylated) nucleic acid, 2'-AP (2'-O-aminopropylated) nucleic acid, 2'-fluorinated nucleic acid, 2'F-arabinonucleic acid (2'-F-ANA), and BNA (bridged nucleic acid).

In an embodiment, BNA may be a ribonucleotide or deoxyribonucleotide in which carbon at position 2' is bridged with carbon at position 4' with the aid of two or more atoms. Examples of bridged nucleic acids are known in the art. An example of a BNA subgroup is BNA in which carbon at position 2' is bridged with carbon at position 4' with the aid of 4'-(CH₂)ₚ-O-2', 4'-(CH₂)ₚ-S-2', 4'-(CH₂)ₚ-OCO-2', and 4'-(CH₂)ₙ-N(R₃)-O-(CH₂)ₘ2' (wherein p, m, and n are each an integer of 1 to 4, 0 to 2, and 1 to 3; and R₃ represents a hydrogen atom, an alkyl group, an alkenyl group, a cycloalkyl group, an aryl group, an aralkyl group, an acyl group, a sulfonyl group, and a unit substituent (e.g., a fluorescence or chemiluminescence label molecule, a functional group having nucleic acid cleavage activity, or intracellular or nuclear transfer signal peptide)). In an embodiment, BNA comprises a substituent of carbon at position 3' (OR₂) and a substituent of carbon at position 5' (OR₁), wherein OR₁ and OR₂ are typically hydrogen atoms, OR₁ and OR₂ may be the same with or different from each other, and OR₁ and OR₂ may be each a hydroxyl protective group in nucleic acid synthesis, an alkyl group, an alkenyl group, a cycloalkyl group, an aryl group, an aralkyl group, an acyl group, a sulfonyl group, a silyl group, a phosphoric acid group, a phosphoric acid group protected with a protective group of nucleic acid synthesis, or -P(R₄)R₅ (wherein R₄ and R₅ may be the same with or different from each other and R₄ and R₅ each represent a hydroxyl group, a hydroxyl group protected with a protective group of nucleic acid synthesis, a mercapto group, a mercapto group protected with a protective group of nucleic acid synthesis, an amino group, an alkoxy group having 1 to 5 carbon atoms, an alkylthio group having 1 to 5 carbon atoms, a cyanoalkoxy group having 1 to 6 carbon atoms, or an amino group substituted with an alkyl group having 1 to 5 carbon atoms). Examples of BNAs include α-L-methyleneoxy(4'-CH₂-O-2')BNA or β-D-methyleneoxy(4'-CH₂-O-2')BNA or β-D-methyleneoxy(4'-CH₂-O-2')BNA also referred to as LNA^{™} (a locked nucleic acid, 2',4'-BNA), ethyleneoxy(4'-CH₂)₂-O-2')BNA, β-D-thio(4'-CH₂-S-2')BNA, and aminoxy(4'-CH₂-O-N(R₃)-2')BNA also referred to as ENA, oxyamino(4'-CH₂-N(R₃)-O-2')BNA, 2',4'-BNA^{COC}, 3' amino-2',4'-BNA, and 5'-methyl BNA also referred to as 2',4'-BNA^{NC}, (4'-CH(CH₃)-O-2')BNA also referred to as cEt-BNA, (4'-CH(CH₂OCH₃)-O-2')BNA also referred to as cMOE-BNA, amide BNA(4'-C(O)-N(R)-2')BNA (R = H, Me) also referred to as AmNA, and other BNAs known in the art.

A modified nucleic acid according to an embodiment may be modified at its base site. Examples of modification at its base site include: 5-methylation, 5-fluorination, 5-bromation, 5-iodization, and N4-methylation of cytosine; 5-demethylation, 5-fluorination, 5-bromation, and 5-iodization of thymidine; N6-methylation and 8-bromation of adenine; and N2-methylation and 8-bromation of guanine. A modified nucleic acid according to another embodiment may be modified at its phosphodiester binding site. Examples of modification at the phosphodiester binding site include phosphorothioation, boranophosphatation, methylphosphonation, methylthiophosphonation, chiral-methylphosphonation, phosphorodithioation, and phosphoroamidation. From the viewpoint of excellent dynamics, phosphorothioation is adopted. Such modification at a base site or that at a phosphodiester binding site may be provided in combinations of two or more on the same nucleic acid.

In general, modified nucleotides and nucleotide analogs are not limited to those exemplified herein. Many modified nucleotides and nucleotide analogs are known in the art. For example, the description of US Patent No. 8299039 of Tachas et al., in particular, the description in the sections 17 to 22, can be adopted as the embodiments of the present application.

A person skilled in the art can adequately select nucleotide analogs from among such modified nucleic acids in view of antisense effects, affinity to a partial sequence of the target gene transcription product, resistance to a nucleic acid degrading enzyme, and other conditions as nucleic acids constituting the nucleic acid complex. In an embodiment, a nucleotide analog is LNA.

In an embodiment, an antisense strand is composed of a region comprising a plurality of DNA nucleotides (hereafter, may be referred to as a "DNA gap region") and a wing region (or wing regions) comprising one or a plurality of nucleotide analogs provided on the 5' terminal side and/or 3' terminal side thereof. This antisense strand is also referred to as "Gapmer." The Gapmer comprises at least 8 bases, such as 8 to 40 bases, preferably 12 to 30 bases, and more preferably 12 to 25 bases or 13 to 20 bases.

In an embodiment, a plurality of DNA nucleotides may be modified nucleotides.

In another embodiment, a plurality of DNA nucleotides may be nucleotide analogs.

A region comprising a nucleotide analog provided at the 5' terminus of the DNA gap region (hereafter, it may be referred to as a "5' wing region") and a region comprising a nucleotide analog provided at the 3' terminus of the DNA gap region (hereafter, it may be referred to as a "3' wing region") are independently of each other, it would be sufficient if such region comprises at least one nucleotide analog mentioned in the literature concerning the antisense method, and such region may further comprise a naturally-occurring nucleic acid (DNA or RNA) or modified nucleotide other than the nucleotide analog. The 5' wing region and the 3' wing region each generally comprise 1 to 10 bases, 1 to 7 bases, or 2 to 5 bases.

In an embodiment, an antisense strand is not a Gapmer, but it is composed of a plurality of DNA nucleotides, modified nucleotides, or nucleotide analogs or two or more thereof in combination. This antisense strand is also referred to as "Non-Gapmer." The Non-Gapmer comprises at least 8 bases, such as 8 to 40 bases, preferably 12 to 30 bases, and more preferably 12 to 25 bases or 13 to 20 bases. In an embodiment, a complementary strand of a duplex nucleic acid complex may be a Gapmer composed of a region comprising a plurality of DNA nucleotides or RNA nucleotides and a wing region (or wing regions) comprising one or a plurality of modified nucleotides and/or nucleotide analogs provided on the 5' terminal side and/or 3' terminal side thereof. The 5' or 3' wing region composed of modified nucleotides and/or nucleotide analogs can more strongly bind to an antisense strand.

In an embodiment, a complementary strand of a duplex nucleic acid complex may comprise a plurality of DNA nucleotides and/or modified nucleotides or nucleotide analogs, or it may comprise RNA nucleotides and/or modified nucleotides or nucleotide analogs. Further, a complementary strand of a duplex nucleic acid complex may comprise a center region comprising nucleotides and/or modified nucleotides and a wing region (or wing regions) comprising one or a plurality of nucleotide analogs and/or modified nucleotides provided on the 5' terminal side and/or 3' terminal side thereof.

### 2. Ligand

A "ligand" is a substance that forms a complex with a biomolecule to serve biological purpose. In an embodiment, a ligand has functions of delivery to a target. An example of a preferable ligand is a lipid since it can efficiently deliver a given nucleic acid complex to the liver or other organ with high specificity. Examples of lipids include lipids, such as cholesterol and fatty acid (e.g., vitamin E (tocopherols and tocotrienols), vitamin A, and vitamin D), fat-soluble vitamin such as vitamin K (e.g., acylcarnitine), an intermediate metabolite such as acyl-CoA, a glycolipid, a glyceride, and a derivative of any thereof. In an embodiment, use of cholesterol and vitamin E (tocopherols and tocotrienols) is particularly preferable because of higher safety. Another example of a preferable ligand is a sugar (e.g., glucose or sucrose) since it can efficiently deliver nucleic acid molecules to the brain with high specificity. A ligand binds to various proteins on the cell surfaces of organs to efficiently deliver a nucleic acid complex to the organ with high specificity. In this respect, an example of a preferable ligand is a peptide or protein of a receptor ligand, an antibody, and/or a fragment thereof.

An "S1P ligand" is a ligand that can bind to an S1P receptor.

In an embodiment, the S1P ligand is a compound represented by Formula 1 below.

A compound represented by Formula 1 or a pharmaceutically acceptable salt: 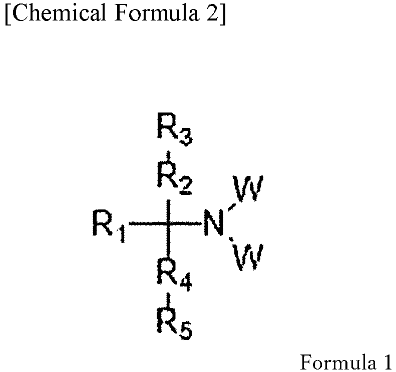 wherein
R₁ represents a C₆-C₅₀ linear or branched carbon chain, and the carbon chain may comprise at least one partial structure, bond, or hetero atom selected from among a double bond, a triple bond, carbocyclic Z, O, S, and N, and/or carbonyl and at least one Y as a substituent;
R₂ and R₄ each represent (CH₂)ₘ;
m is an integer of 0 to 4;
R₃ and R₅ are each selected from the group consisting of -H, -X-, -XW, -P(=X)(-XW)₂, and -XP(=X) (-XW)₂;
X is selected from the group consisting of O, S, and NH;
Y is selected from the group consisting of alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, alkyl halide, hydroxy, carboxy, sulfonic acid, sulfonic acid derivative, azide, and thiol;
Z represents a C₁-C₃₀ linear or branched carbon chain, the carbon chain is selected from the group consisting of cycloalkylalkyl, arylalkyl, heterocycloalkylalkyl, and heteroarylalkyl, and the carbon chain may comprise a bond or hetero atom selected from among a double bond, a triple bond, O, S, N, sulfinyl, sulfonyl, and/or carbonyl and at least one Y as a substituent; and
W independently represents H or a C₁-C₆₀ linear or branched carbon chain, the carbon chain may comprise at least one partial structure, bond, or hetero atom selected from among a double bond, a triple bond, carbocyclic Z, O, S, and N, and/or carbonyl and at least one Y as a substituent, and two substituents represented by W bound to N may be the same with or different from each other.

In some embodiments, the S1P ligand of the present invention is preferably a compound represented by Formula 1 or a pharmaceutically acceptable salt.

In Formula 1,
R₁ represents a C₆-C₅₀ linear or branched carbon chain, and the carbon chain may comprise at least one partial structure, bond, or hetero atom selected from among a double bond, a triple bond, carbocyclic Z, O, and N, and/or carbonyl and at least one Y as a substituent;
R₂ and R₄ each represent (CH₂)ₘ;
m is an integer of 0 to 4;
R₃ and R₅ are each selected from the group consisting of -H, -X-, -XW, and -XP(=X) (-XW)₂;
X is selected from the group consisting of O and S;
Y is selected from the group consisting of alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, alkyl halide, hydroxy, carboxy, sulfonic acid, azide, and thiol;
Z represents a C₁-C₃₀ linear or branched carbon chain, the carbon chain is selected from the group consisting of arylalkyl and heteroarylalkyl, and the carbon chain may comprise a bond or hetero atom selected from among a double bond, a triple bond, O, S, N, sulfinyl, sulfonyl, and/or carbonyl and at least one Y as a substituent; and
W independently represents H or a C₁-C₆₀ linear or branched carbon chain, the carbon chain may comprise at least one partial structure, bond, or hetero atom selected from among a double bond, a triple bond, carbocyclic Z, O, S, and N, and/or carbonyl and at least one Y as a substituent, and two substituents represented by W bound to N may be the same with or different from each other.

In some embodiments, the S1P ligand of the present invention is more preferably a compound represented by Formula 1 or a pharmaceutically acceptable salt.

In Formula 1,
R₁ represents a C₆-C₅₀ linear or branched carbon chain, and the carbon chain may comprise at least one partial structure, bond, or hetero atom selected from among a double bond, a triple bond, carbocyclic Z, O, and N, and/or carbonyl and at least one Y as a substituent;
R₂ and R₄ each represent (CH₂)ₘ;
m is an integer of 0 to 4;
R₃ and R₅ are each selected from the group consisting of -H, -X-, -XW, and -XP(=X) (-XW)₂;
X is selected from the group consisting of O and S;
Y is selected from the group consisting of alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, alkyl halide, hydroxy, carboxy, and azide;
Z represents a C₁-C₃₀ arylalkyl, and the arylalkyl chain may comprise a bond or hetero atom selected from among a double bond, a triple bond, O, S, N, sulfinyl, and carbonyl and at least one Y as a substituent; and
W independently represents H or is selected from the group consisting of a C₁-C₅ linear or branched alkyl, acyl, and alkoxy, and two substituents represented by W bound to N may be the same with or different from each other.

In some embodiments, the S1P ligand of the present invention is a compound represented by Formula 1, wherein R₁ is selected from among compounds shown in Table 1.

**[Table 1]**

| Substituent | Structure | Structural formula | Name of structural formula |
|---|---|---|---|
| R₁ | C₆₋₅₀ linear carbon chain | 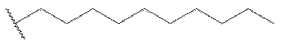 | Decyl group |
| | C₆₋₅₀ branched carbon chain | 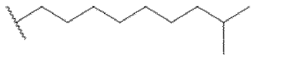 | Methylnonyl group |
| | C₆₋₅₀ carbon chain having a double bond | 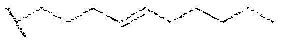 | (E)-Decenyl group |
| | C₆₋₅₀ carbon chain having a triple bond | 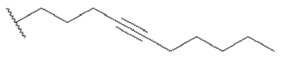 | Decinyl group |
| | C₆₋₅₀ carbon chain containing O | 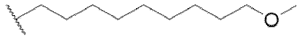 | Methoxynonyl group |
| | C₆₋₅₀ carbon chain containing S | 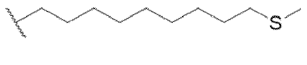 | Methyl(nonyl)sulfanyl group, including sulfinyl and sulfonyl |
| | C₆₋₅₀ carbon chain containing N | 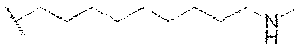 | Methylnonylamino group |
| | C₆₋₅₀ carbon chain containing carbonyl | 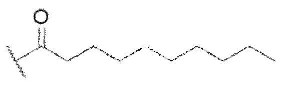 | Decylnonyl group |

In some embodiments, the S1P ligand of the present invention is a compound represented by Formula 1, wherein R₂ and R₄ are each selected from among compounds shown in Table 2.

**[Table 2]**

| Substituent | Structure | Structural formula | Name of structural formula |
|---|---|---|---|
| R₂ | (CH₂)m | 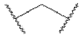 | Methylene group |
| R₄ | m: integer of 0-4 | | |

In some embodiments, the S1P ligand of the present invention is a compound represented by Formula 1, wherein R₃ and R₅ are each selected from among compounds shown in Table 3.

**[Table 3]**

| Substituent | Structure | Structural formula | Name of structural formula |
|---|---|---|---|
| R₃ | -H | | -H |
| R₅ | | | |
| | -XW | 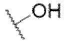 | Hydroxy group |
| | -X⁻ | 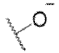 | |
| | -P(=X)(-XW)₂ | 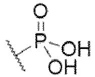 | Phosphonate group |
| | -XP(=X) (-XW)₂ | 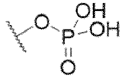 | Phosphate group |

In some embodiments, the S1P ligand of the present invention is a compound represented by Formula 1, wherein Y is selected from among compounds shown in Table 4.

**[Table 4]**

| Substituent | Structure | Structural formula | Name of structural formula |
|---|---|---|---|
| Y | Alkoxy | 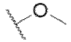 | Methoxy group |
| | Alkenyloxy | 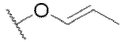 | Propionyloxy group |
| | Alkynyloxy | 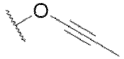 | Propionyloxy group |
| | Aralkyloxy | 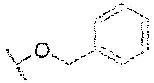 | Benzyloxy group |
| | Acyl | 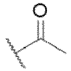 | Acetyl group |
| | Alkylamino | 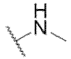 | Methylamino group |
| | Alkylthio | 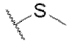 | Methylsulfanyl group, including sulfinyl and sulfonyl |
| | Acylamino | 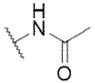 | Acetylamide group |
| | Alkoxycarbonyl | 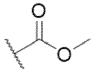 | Methoxycarbonyl group |
| | Alkoxycarbonylamino | 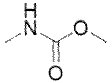 | Methoxycarbamate group |
| | Acyloxy | 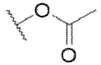 | Acetic ester |
| | Alkylcarbamoyl | 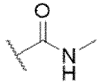 | Methylamide |
| | Nitro | 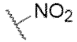 | Nitro group |
| | Halogen | 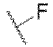 | Fluoro group |
| | Alkyl halide | 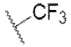 | Trifluoromethyl group |
| | Hydroxy | 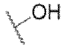 | Hydroxy group |
| | Carboxy | 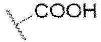 | Carboxylic acid |
| | Sulfonic acid | 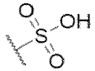 | Sulfonic acid |
| | Sulfonic acid derivative | 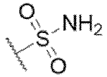 | Sulfonic acid amide |
| | Azide | 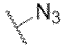 | Azide |
| | Thiol | 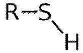 | Thiol |

In some embodiments, the S1P ligand of the present invention is a compound represented by Formula 1, wherein Z is selected from among compounds shown in Table 5.

**[Table 5]**

| Substituent | Brief description | Structure example | Name of structure example |
|---|---|---|---|
| Z | C₁₋₂₀ cycloalkylalkyl | 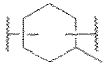 | Methylcyclohexane |
| | C₁₋₂₀ arylalkyl | 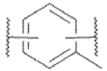 | Toluene |
| | C₁₋₂₀ cycloalkylalkyl | 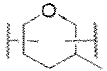 | Methylpyran |
| | C₁₋₂₀ heteroarylalkyl | 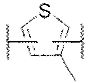 | Methylthiofen |
| | C₁₋₂₀ arylalkyl containing Y | 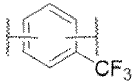 | Y=F |

In some embodiments, the S1P ligand of the present invention is a compound represented by Formula 1, wherein W is selected from among compounds shown in Table 6.

**[Table 6]**

| Substituent | Structure | Structural formula | Name of structural formula |
|---|---|---|---|
| W | C₁₋₆₀ linear carbon chain | 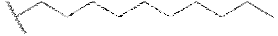 | Decyl group |
| | C₁₋₆₀ branched carbon chain | 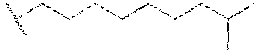 | Methylnonyl group |
| | C₁₋₆₀ carbon chain containing double bond | 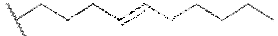 | (E)-Decenyl group |
| | C₁₋₆₀ carbon chain containing triple bond | 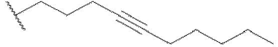 | Decinyl group |
| | C₁₋₆₀ carbon chain containing O | 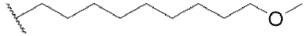 | Methoxynonyl group |
| | C₁₋₆₀ carbon chain containing S | 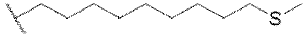 | Methyl(nonyl)sulfanyl group, including sulfinyl and sulfonyl |
| | C₁₋₆₀ carbon chain containing N | 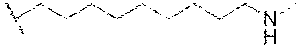 | Methylnonylamino group |
| | C₁₋₆₀ carbon chain containing carbonyl | 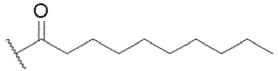 | Decylnonyl group |
| | -H | 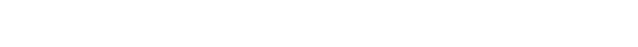 | |
| | C₁₋₅ linear alkyl group | 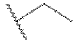 | Ethyl group |
| | C₁₋₅ branched alkyl group | 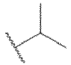 | Isopropyl group |
| | C₁₋₅ acylalkyl group | 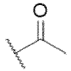 | Acetyl group |
| | C₁₋₅ alkoxyalkyl group | 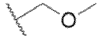 | Methoxymethyl group |

In the nucleic acid complex of the present invention, a ligand binds to an antisense strand of a nucleic acid molecule. In the case of a double-stranded nucleic acid molecule, a ligand binds to an antisense strand and/or complementary strand of a nucleic acid molecule. In an embodiment in which a nucleic acid molecule has HDO, a ligand binds to a complementary strand of a nucleic acid molecule. In such a case, HDO is introduced into a target tissue or cell, a complementary strand is cleaved by an enzyme such as ribonuclease (mainly RNase H), and the ligand is liberated from the antisense strand together with the cleaved complementary strand. Thus, the antisense strand becomes capable of binding to a target transcription factor in a target tissue or cell without being influenced by the ligand. In some embodiment, a ligand binds to the 3' or 5' terminus of the nucleic acid strand. In some embodiment, a ligand binds to a site other than the terminus of the nucleic acid strand. A method of binding a ligand to position 2 of a pentose of a nucleotide is known. For example, such method can be performed in accordance with the method disclosed in WO 2018/003739.

Examples of salts of the compounds include sodium salt, potassium salt, calcium salt, hydrochloric acid salt, sulfuric acid salt, nitric acid salt, acetic acid salt, methanesulfonic acid salt, toluenesulfonic acid salt, citric acid salt, fumaric acid salt, maleic acid salt, and hydrobromic acid salt.

### 3. Binding of ligand to nucleic acid molecule

A ligand can bind to a nucleic acid by, for example, binding a bindable group of a ligand to a bindable group of a nucleic acid molecule covalently or non-covalently by means of hydrogen binding, electrostatic interaction, or hydrophobic interaction. Examples of bindable groups of a ligand include, but are not limited to, amino, hydroxy, carboxylic acid, thiol, disulfide, and azide groups. Examples of bindable groups of a nucleic acid molecule include carbon at position 2 and a hydroxy group at position 3 or 5 of a nucleoside sugar, a phosphoric acid group at position 5 of a nucleotide, and a base portion of a nucleoside. Another example of a bindable group in a nucleic acid molecule is a phosphoric acid group in an oligonucleotide.

### 4. Linker

In some embodiments, a ligand binds to a nucleic acid molecule via a linker. A linker may be referred to as a "spacer." When a nucleic acid molecule is HDO, a ligand may bind to an antisense strand and/or complementary strand of HDO via a linker. A cleavable or uncleavable linker can be used.

A "cleavable linker" is a linking group that is cleaved in, for example, a cell or animal body (e.g., human body) under physiological conditions. In a particular embodiment, a cleavable linker is selectively cleaved by an endogenous enzyme, such as a nuclease. Examples of cleavable linkers include an amide bond, an ester bond, one or both ester bonds of phosphodiester bonds, an ester phosphate bond, a carbamate bond, a disulfide bond, and a natural DNA linker. An "uncleavable linker" is a linker that is not cleaved in, for example, a cell or animal body (e.g., human body) under physiological conditions. Examples of uncleavable linkers include, but are not limited to, phosphorothioate bond, a modified or unmodified deoxyribonucleoside linked via a phosphorothioate bond, and a linker consisting of a modified or unmodified ribonucleoside. When a linker is a nucleic acid or an oligonucleotide, such as DNA, a chain length is not particularly limited, and, in general, a linker may comprise 2 to 20, and preferably 3 to 10 bases.

In some embodiments, a linker used in the present invention may be a chain structure, such as a hydrocarbyl chain, or an oligomer, which is a repeating unit such as an ethylene glycol, nucleoside, or amino acid unit.

In some embodiments, a linker comprises at least one group selected from among alkyl, amino, oxo, amide, disulfide, polyethylene glycol, ether, thioether, and hydroxylamino groups.

In some embodiments, a linker comprises a group selected from among alkyl, amino, oxo, amide, and ether groups.

In some embodiments, a linker comprises a group selected from among alkyl and amide groups.

In some embodiments, a linker comprises a group selected from among alkyl and ether groups.

In some embodiments, a linker comprises at least one phosphorus portion.

In some embodiments, a linker comprises at least one phosphate group.

In some embodiments, a linker comprises at least one neutral linking group.

In some embodiments, a length of a linker is 1 to 1000 Å. A length of a particular linker is 3 to 500 Å. A length of a linker is preferably 10 to 200 Å. (The length was estimated based on the crystalline structure comprising the S1P1 receptor bound to W146 (PDB3V2Y; Science 2012, 335, 851-855).)

In some embodiments, a bond between a linker and an oligonucleotide can be a bifunctional bond. In general, a bifunctional bond is formed of at least 2 functional groups. A functional group of a linker binding to a particular site of an oligonucleotide is selected, and another functional group of a linker binding to a ligand portion is selected. Examples of functional groups used for a bifunctional bond of a linker include, but are not limited to, an electrophile to react with a nucleophilic group and a nucleophilic agent to react with an electrophilic group. In a particular embodiment, a bifunctional bond can be formed with the use of one or more groups selected from among amino, hydroxyl, carboxylic acid, thiol, alkyl, alkenyl, and alkynyl groups.

Examples of linkers include, but are not limited to, 6-aminohexanoic acid (AHA or AHEM), (2,5-dioxypyrrolidin-1-yl)4-(2-azatricyclo[10.4.0.04,9]hexadeca-1(16),4,6,8,12,14-hexe-10-n-2-yl-4-oxobutanoate (DBCO-NHS), 3-mercaptopropionic acid, and succinimidyl 4-(N-meleimidemethyl)cyclohexane-1-carboxylate. Examples of other linkers include, but are not limited to, substituted or unsubstituted C¹ to C¹⁰ alkyl, substituted or unsubstituted C² to C¹⁰ alkenyl, and substituted or unsubstituted C² to C¹⁰ alkynyl. Examples of preferable substituents include, but are not limited to, hydroxyl, amino, alkoxy, carboxy, benzyl, phenyl, nitro, thiol, thioalkoxy, halogen, alkyl, aryl, alkenyl, and alkynyl.

In a particular embodiment, a linker can be selected from among compounds having the structures shown below and derivatives thereof.

**[Table 7]**

| Linker type | Structural formula |
|---|---|
| Having a disulfide bond | 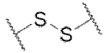 |
| Having an amide bond (n=0-9) | 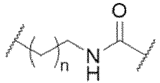 |
| Having a polyethylene group (n=1-30) | 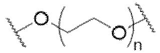 |
| Having a phosphoric acid group | 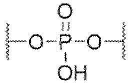 |
| Having a pyrrolidinyl group | 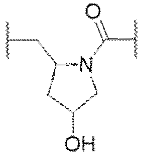 |
| Having a BCN group | 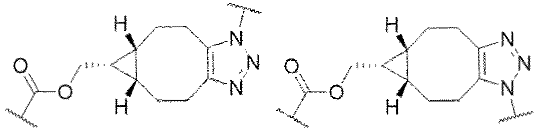 |
| Having a DBCO group | 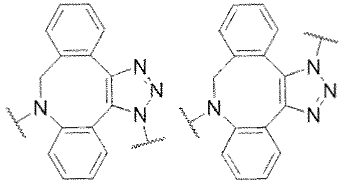 |
| Having a maleimide thioether group | 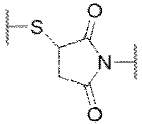 |

In a particular embodiment, a linker may be composed of 2 to 20 linker-nucleoside bonds. In a particular embodiment, a linker-nucleoside bond is composed of continuous modified nucleosides. In a particular embodiment, such linker-nucleoside bond may comprise a modified sugar portion. In a particular embodiment, a linker-nucleoside bond is not modified. In a particular embodiment, a linker-nucleoside bond comprises a protected heterocyclic base selected from among purine, substituted purine, pyrimidine, and substituted pyrimidine, according to need.

In a particular embodiment, a linker can be selected from among compounds having the structures shown below and derivatives thereof.

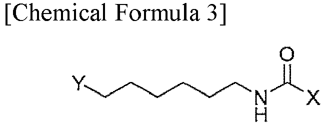

In the formula, X represents a ligand-binding site, and Y represents a nucleic acid molecule-binding site.

In a particular embodiment, a compound having active ester represented by Chemical Formula 4: 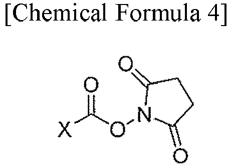 (wherein X represents a ligand-binding site) may be allowed to react with an oligonucleotide having terminal amine comprising a compound represented by Chemical Formula 5: 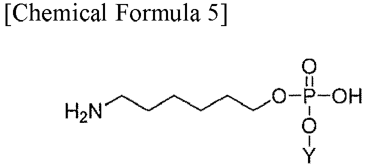 (wherein Y represents a nucleic acid molecule-binding site) to obtain a linker represented by Chemical Formula 6 below: 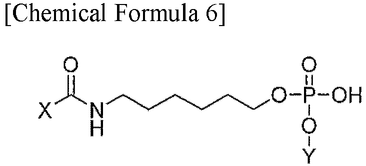 (wherein X represents a ligand-binding site, Y represents a nucleic acid molecule-binding site, and a nucleic acid molecule is selected from among a natural nucleotide, a modified nucleotide, or a nucleotide analogs or an oligonucleotide comprising the same).

In a particular embodiment, a linker comprising phosphate/triethylene glycol can be used.

In a particular embodiment, a linker represented by Chemical Formula 7 can be used. 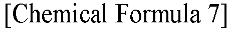 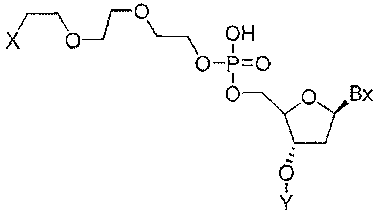 In the formula, X represents a ligand-binding site, Y represents a nucleic acid molecule-binding site, and Bx represents a modified or unmodified nucleic acid base.

In a particular embodiment, a compound having amidite represented by Chemical Formula 7 above and a compound represented by Chemical Formula 8: 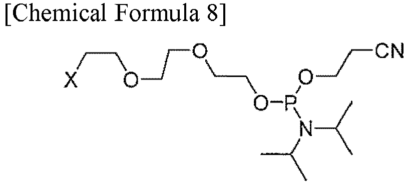 (wherein X represents a ligand) are allowed to react with an oligonucleotide site Y on a solid-phase support and cleaved from the solid-phase support to obtain a linker represented by Chemical Formula 9: 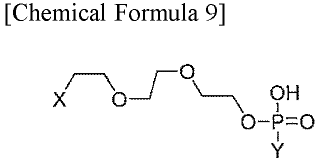 (wherein Y represents a nucleic acid molecule).

In a particular embodiment, a linker has a structure represented by Chemical Formula 10 below: 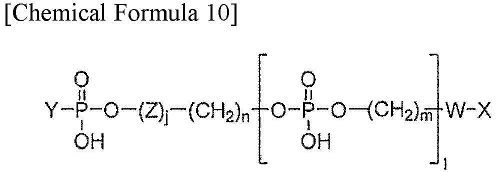 (wherein X represents a ligand-binding site; Y represents a nucleic acid molecule-binding site; W represents a phosphodiester or amino group; Z represents a pyrrolidinyl group represented by Chemical Formula 11 below; j is 0 or 1; n is about 1 to about 10; m is about 1 to about 10; l is 0 or 1 to 4; and, when X represents an amino group, l is 1).

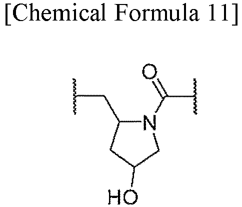

In a particular embodiment, a linker is prepared by click chemistry. Linkers that are preferably used in several embodiments can be prepared by click chemistry described in "Click Chemistry for Biotechnology and Materials Science," Ed. Joerg Laham, Wiley, 2009 (which is incorporated herein by reference in their entirety).

In a particular embodiment, a compound represented by Chemical Formula 12: 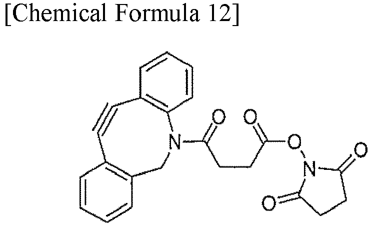 and a compound represented by Chemical Formula 13: 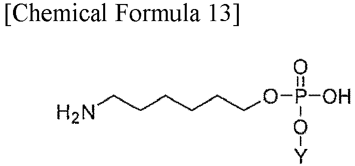 (wherein Y represents a nucleic acid molecule-binding site) were allowed to react with an oligonucleotide having terminal amine to obtain a compound represented by Chemical Formula 14.

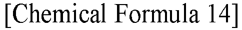

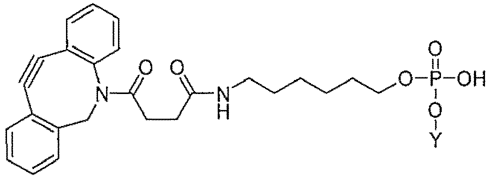

The resulting compound represented by Chemical Formula 14 was allowed to react with a ligand having azide to obtain a linker represented by Chemical Formula 15: 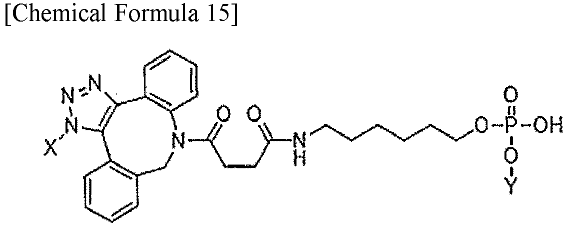 (wherein X represents a ligand-binding site, and Y represents a nucleic acid molecule-binding site).

In a particular embodiment, a maleimide linker can be used. The maleimide linker has a structure represented by Chemical Formula 16.

The linker comprises: 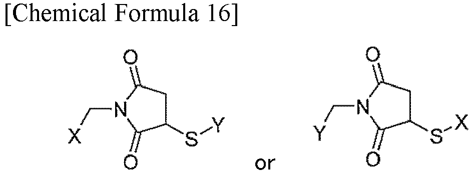 wherein X represents a ligand-binding site, and Y represents a nucleic acid molecule-binding site.

In a particular embodiment, a compound represented by Chemical Formula 17: 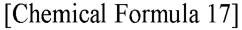 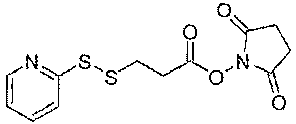 and a compound represented by Chemical Formula 18: 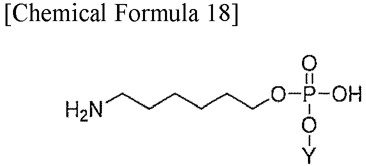 (wherein Y represents a nucleic acid molecule-binding site) were allowed to react with an oligonucleotide having terminal amine to obtain a compound represented by Chemical Formula 19.

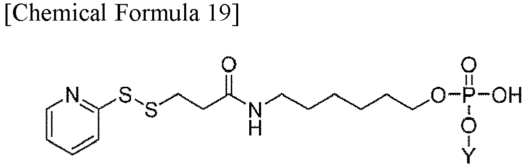

The compound represented by Chemical Formula 19 was allowed to react with a ligand conjugate portion having maleimide to obtain a linker represented by Chemical Formula 20: 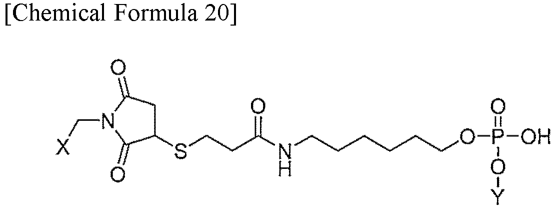 (wherein X represents a ligand-binding site, and Y represents a nucleic acid molecule-binding site).

In a particular embodiment, a compound represented by Chemical Formula 21: 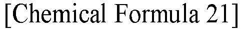 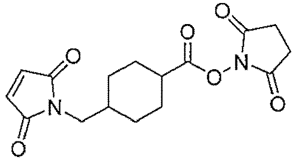 and a compound represented by Chemical Formula 22: 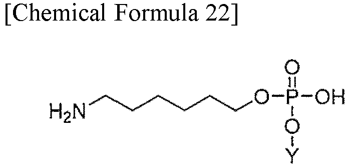 (wherein Y represents a nucleic acid molecule-binding site) were allowed to react with an oligonucleotide having terminal amine to obtain a compound represented by Chemical Formula 23.

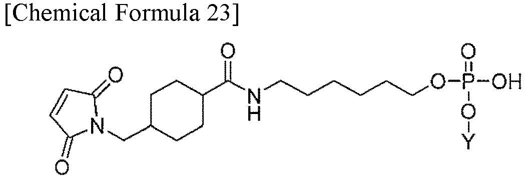

The compound represented by Chemical Formula 23 was allowed to react with a ligand conjugate portion having thiol to obtain a linker represented by Chemical Formula 24: 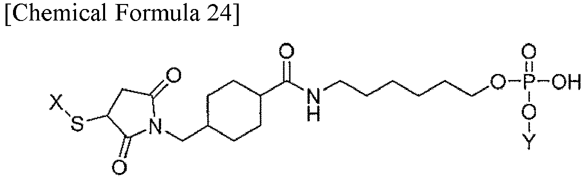 (wherein X represents a ligand-binding site, and Y represents a nucleic acid molecule-binding site).

### Specific examples of disulfide linkers and attachment method

In a particular embodiment, a linker comprising a disulfide bond can be used. In a particular embodiment, a linker comprises activated disulfide that forms a disulfide bond with a ligand-binding portion.

In a particular embodiment, a compound represented by Chemical Formula 25: 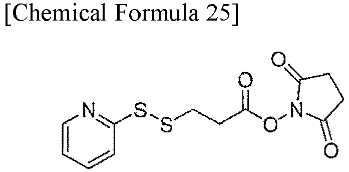 and a compound represented by Chemical Formula 26: 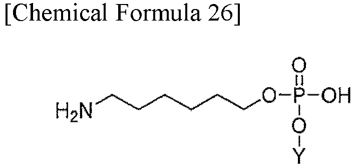 (wherein Y represents a nucleic acid molecule binding site) were allowed to react with an oligonucleotide having terminal amine to obtain a compound represented by Chemical Formula 27.

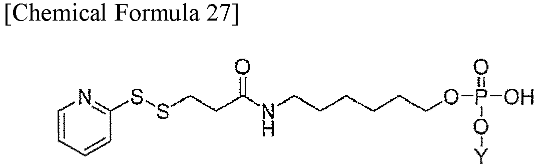

The disulfide bond thereof was cleaved to obtain a compound represented by Chemical Formula 28.

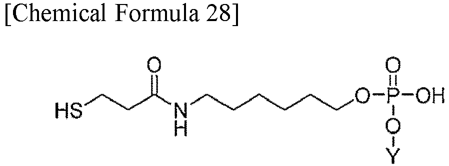

The compound represented by Chemical Formula 28 was allowed to react with a ligand conjugate portion having thiol to obtain a linker represented by Chemical Formula 29: 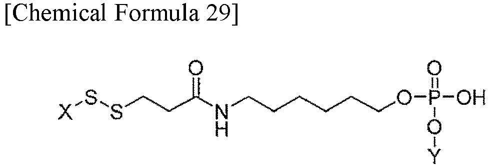 (wherein X represents a ligand-binding site, and Y represents a nucleic acid molecule-binding site).

In a particular embodiment, a linker chemically binds to the S1P ligand. The S1P ligand binds to a functional group via a linker so as to be attached to an oligonucleotide.

In a particular embodiment, an amino group (but is not limited thereto) in the ligand-binding portion (X-NH₂; in which X represents a ligand conjugate portion excluding the amino group) was allowed to react with a compound represented by Chemical Formula 30: 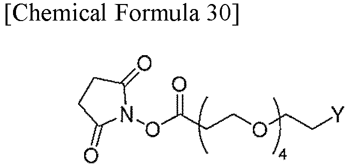 (wherein Y represents a functional group to be directly or indirectly attached to an oligo, such as azide or maleimide) to obtain a linker comprising a linker introduced into the ligand-binding portion represented by Chemical Formula 31: 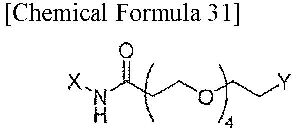 (wherein X represents a ligand-binding site, and Y represents a nucleic acid molecule-binding site).

In a particular embodiment, a hydroxy group (but is not limited thereto) in the ligand-binding site (PX-OH; in which PX represents a precursor of the ligand conjugate portion excluding the hydroxy group) was allowed to react with a compound represented by Chemical Formula 32: 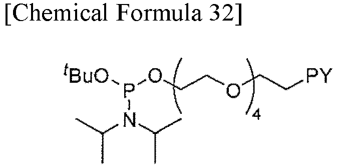 (wherein PY represents a precursor of a functional group to be directly or indirectly attached to an oligonucleotide, such as tosyl or trifluoromethanesulfonyl) to obtain a compound represented by Chemical Formula 33: 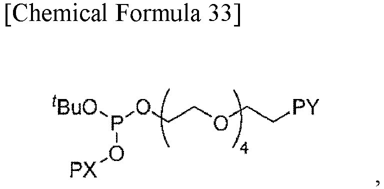 and a linker represented by Chemical Formula 34 was obtained as a final product: 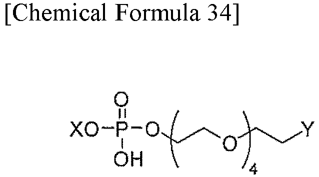 (wherein X represents a ligand-binding site excluding a hydroxyl group, which is a functional group to be directly or indirectly attached to an oligo, such as azide or maleimide).

### 5. Pharmaceutical composition

Diseases targeted by the ligand-binding nucleic acid complex of the present invention may be any diseases, provided that such diseases are associated with the target organs and tissue and cells in the target organs. Examples of such diseases include, but are not limited to, diabetes, metabolic syndrome, cardiac disease, cardiac myopathy, muscular dystrophy, myotonic dystrophy, Becker muscular dystrophy, congenital muscular dystrophy, Duchenne muscular dystrophy, distal muscular dystrophy, Emery-Dreifuss muscular dystrophy, facioscapulohumeral muscular dystrophy, limb girdle muscular dystrophy, oculopharyngeal muscular dystrophy, chronic kidney disease (CKD), renal fibrosis, diabetic nephropathy, chronic glomerulonephritis, IgA nephropathy, lupus nephritis, primary glomerular disease, chronic obstructive pulmonary disease (COPD), lung emphysema, interstitial pneumonia, lung fibrosis, heart disease, muscular disease, liver disease, acute liver diseases such as acute viral hepatitis and drug-induced hepatopathy, and chronic liver diseases, such as chronic hepatitis B, chronic hepatitis C, cirrhosis, liver cancer, alcoholic liver injury, primary biliary cirrhosis, autoimmune hepatitis, and nonalcoholic steatohepatitis (NASH).

A composition comprising the ligand-binding nucleic acid complex according to any of some embodiments can be prepared in the form of a pharmaceutical product in accordance with a conventional pharmaceutical method. Such composition can be used in the form of, for example, a capsule, a tablet, a pill, a liquid, a powder, granules, fine grains, a film-coated agent, a pellette, a troche, a sublingual formulation, a masticatory formulation, a buccal tablet, a paste, a syrup, a suspension, an elixir, an emulsion, an endermic liniment, an ointment, a plaster, a poultices, a transdermal formulation, a lotion, an inhalant, an aerosol, an injection preparation, or a suppository for enteral (oral) or nonenteral administration.

When manufacturing a pharmaceutical preparation, such composition can be used in adequate combination with a carrier that is pharmacologically acceptable or acceptable for a food or beverage product. Specific examples include sterilized water, physiological saline, vegetable oil, a solvent, a base, an emulsifier, a suspending agent, a surfactant, a pH modifier, a stabilizer, a flavoring agent, an aromatic agent, an excipient, a vehicle, a preservative, a binder, a diluent, an isotonizing agent, a soothing agent, a filler, a disintegrator, a buffer, a coating agent, a lubricant, a coloring agent, a sweetening agent, a thickener, a corrigent, a solubilizer, and other additives.

When manufacturing a pharmaceutical preparation, the ligand-binding nucleic acid complex according to any of the embodiments concerning enteral administration may form a composite with a substance having activity of increasing permeability of the large intestinal epithelium (e.g., medium chain fatty acid, long chain unsaturated fatty acid, or a derivative thereof (salt, ester, or ether) and a surfactant (a nonionic surfactant or anionic surfactant) (i.e., mixed micelle or emulsion)) in order to enhance efficiency for enteral administration.

Preferable administration routes of the composition comprising the ligand-binding nucleic acid complex according to some embodiments are not particularly limited, and enteral (oral) or nonenteral routes may be adopted. Specific examples include intravenous administration, intraarterial administration, intraperitoneal administration, subcutaneous administration, intracutaneous administration, intraspinal administration, tracheobronchial administration, rectal administration, intramuscular administration, and transfusion.

The composition comprising the ligand-binding nucleic acid complex according to some embodiments can be used for animal targets including humans. Animals other than humans are not particularly limited, and targets can be various livestock animals, fowls, pet animals, and experimental animals.

When the compositions comprising the ligand-binding nucleic acid complex according to some embodiments are administered or ingested, the amount of administration or ingestion may be adequately determined in accordance with, for example, age, body weight, symptoms, and health conditions of a target, and a composition type (e.g., a pharmaceutical product or food or beverage product). According to an embodiment, an effective amount of the composition to be ingested is preferably 0.001 mg/kg/day to 50 mg/kg/day in terms of nucleotides.

### [Examples]

### [Example 1]

As the S1P ligands, RN01-N-PEG4-azide, RN01-N-PEG1-azide, RN01-N-PEG9-azide, RN01-N-C10-azide, RN01-N-PEG4-maleimide, and RN01-N-PEG4-SPDP were synthesized.

### Process of synthesis 1

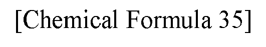

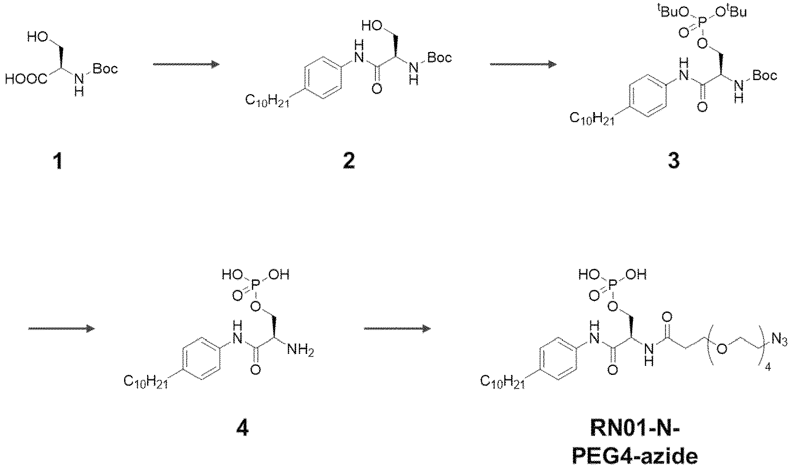

### <Synthesis of t-butyl(R)-(1-((4-decylphenyl)amino)-3-hydroxy-1-oxopropan-2-yl)carbamate (Compound 2)>

4-Decylaniline (1.16 g) was dissolved in 25 ml of dry dichloromethane, (t-butoxycarbonyl)-D-serine (Compound 1, 1.02 g), 750 µl of triethylamine and 2.14 g of 1-[bis(dimethylamino)methylene] -1H-1,2,3 -triazolo [4,5 -b]pyridinium 3-oxide hexafluorophosphate were successively added thereto, the mixture was agitated under nitrogen atmosphere at room temperature for 2 hours, 0.2 ml of water was added to the reaction solution, and the resultant was then agitated for 1 hour. After the solvent was removed from the reaction solution by distillation, 50 ml of ethyl acetate was added, and the resultant was washed with 50 ml of an aqueous solution of saturated sodium bicarbonate and 50 ml of saturated saline. The organic phase was dried over sodium sulfate, the solvent was removed by distillation, and the residue was obtained as a white solid (2.84 g). The residue was dissolved in chloroform, purified by silica gel column chromatography (Yamazen HI-FLASH PREMIUM silica; ϕ46 × 130 mm; methanol/chloroform (methanol 0% -> 10%)), and Compound 2 was obtained as a white solid (1.84 g).
MS (ESI) m/z: 419.2 [M-H]

¹H-NMR (CDCl₃) δ (ppm): 8.68 (br, 1 H), 7.38 (d, J = 8.4 Hz,2H),7.12 (d, J = 8.7 Hz, 2 H), 5.68 (br, 1 H), 4.29-4.21 (m, 2 H), 3.75-3.68 (m, 1 H), 3.09 (br, 1 H), 2.56 (t, J = 7.7 Hz, 2 H), 1.57 (t, J = 7.4 Hz, 2 H), 1.47 (s, 9 H), 1.27 (m, 14 H), 0.88 (t, J = 6.8 Hz, 3 H)

### <Synthesis of t-butyl(R)-(1-(4-decylphenyl)amino)-3-((di-t-butoxyphosphoryl)oxy)-1-oxopropan-2-yl)carbamate (Compound 3)>

Compound 2 (210 mg) was dissolved in 5 ml of dry dichloromethane, 73 mg of tetrazole and 310 µl of di-t-butyl-N,N-diisopropylphosphoroamidite were successively added thereto, and the mixture was agitated under nitrogen atmosphere at room temperature for 18 hours. Under ice cooling, 5 ml of tetrahydrofuran and 0.3 ml of a solution of 35% hydrogen peroxide were added to the reaction solution, and the resultant was then agitated at room temperature for 6 hours. Thereafter, 1 ml of an aqueous solution of 15% sodium thiosulfate was added, and the resultant was then agitated at room temperature for 17 hours. Ethyl acetate (30 ml) was added to the reaction solution, and the organic phase was washed with an aqueous solution of saturated sodium bicarbonate (40 ml × 2) and saturated saline (40 ml). The organic phase was dried over sodium sulfate, the solvent was removed by distillation, and a roughly purified product was obtained as colorless transparent oil (326 mg). The resulting oil was purified by silica gel column chromatography (Yamazen universal column silica; ϕ30 × 165 mm; methanol/chloroform (methanol 0%→3%)), and Compound 3 was obtained as colorless transparent oil (244 mg).
MS (ESI) m/z: 611.2 [M-H]

### <Synthesis of (R)-2-amino-3-((4-decylphenyl)amino)-3-oxopropylphorphoric acid (Compound 4)>

Compound 3 (244 mg) was dissolved in 5 ml of dichloromethane, 5 ml of trifluoroacetic acid was added thereto, and the mixture was agitated at room temperature for 3 hours. Toluene (5 ml) was added to the reaction solution to remove the solvent by distillation. The residue was washed 5 times with 3 ml of diethyl ether, and Compound 4 was obtained as a white solid (111 mg).
MS (ESI) m/z: 399.1 [M-H]

### <Synthesis of (R)-1-azide-17-((4-decylphenyl)carbamoyl)-15-oxo-3,6,9,12-tetraoxa-16-azaoctadeca-18-nyl phosphate (RN01-N-PEG4-azide)>

2,5-Dioxypyrrolidin-1-yl 1-azide-3,6,9,12-tetraoxapentadeca-15-noate (PEG4-azide-NHS ester, 24 mg) was dissolved in 1 ml of dichloromethane, 24 mg of Compound 4 and 24 µl of triethylamine were added thereto, and the resultant was agitated at room temperature for 2 hours. After the solvent was removed by distillation under reduced pressure, the resulting residue was dissolved in an aqueous solution of methanol/triethyl ammonium bicarbonate (0.1 M) (1:1), purified by reverse phase column chromatography (Yamazen universal column ODS; ϕ23 × 123 mm; an aqueous solution of methanol/triethyl ammonium bicarbonate (0.1 M) (methanol 44%-7100%)), and RN01-N-PEG4-azide·triethylammonium salt was obtained as colorless transparent oil (28 mg).
MS (ESI) m/z: 672.1 [M-H]
¹H-NMR (CD₃OD) δ (ppm): 7.47 (d, 8.3 Hz, 2 H), 7.12 (d, J = 8.3 Hz, 2 H), 4.67 (t, J = 5.1 Hz, 1 H), 4.23-4.15 (m, 2 H), 3.84-3.75 (m, 2 H), 3.68-3.59 (m, 14 H), 3.35 (t, J = 4.9 Hz, 2 H), 3.14 (q, J = 7.3 Hz, 6 H),2.67-2.51 (m, 4 H),1.59 (t, J = 7.0 Hz, 2 H), 1.31-1.25 (m, 23 H),0.90 (t, J = 6.6 Hz, 3 H)
³¹P-NMR (CD₃OD) δ (ppm): 0.79

In the same manner as in the process of synthesis 1, PEG1-azide-NHS ester, PEG9-azide-NHS ester, C10-azide-NHS ester, PEG4-maleimide-NHS ester, and PEG4-SPDP-NHS ester were used instead of PEG4-azide-NHS ester to obtain RN01-N-PEG1-azide, RN01-N-PEG9-azide, RN01-N-C10-azide, RN01-N-PEG4-maleimide, and RN01-N-PEG4-SPDP.
(R)-2-(3-(2-azidoethoxy)propanamide)-3-((4-decylphenyl)amino)-3-oxoppropylphosphoric acid (RN01-N-PEG1-azide)
MS (ESI) m/z: 540.1 [M-H]
   ¹H-NMR (CD₃OD) δ (ppm): 7.47-7.44 (m, 2 H), 7.11 (d, J = 8.3 Hz, 2 H), 4.62 (t, J = 5.1 Hz, 1 H), 4.22-4.12 (m, 2 H), 3.81 (t, J = 6.4 Hz, 2 H), 3.67-3.63 (m, 2 H), 3.35 (t, J = 5.1 Hz, 2 H), 3.15 (q, J = 7.3 Hz, 6 H), 2.67-2.54 (m, 4 H), 1.59 (t, J = 7.0 Hz, 2 H), 1.31-1.26 (m, 23 H), 0.90 (t, J = 6.8 Hz, 3 H)
   ³¹P-NMR (CD₃OD) δ (ppm): 1.23
   (R)-1-Azide-32-(((4-decylphenyl)carbamoyl)-30-oxo-3,6,9,12,15,18,21,24,27-nonaoxa-31-azatritriacontan-33-yl phosphoric acid (RN01-N-PEG9-azide)
MS (ESI) m/z: 894.4 [M + H]
   ¹H-NMR (CD₃OD) δ (ppm): 7.47 (d, J = 8.7 Hz, 2 H), 7.12 (d, J = 8.3 Hz, 2 H), 4.62 (t, J = 5.1 Hz, 1 H), 4.21-4.14 (m, 2 H), 3.83-3.52 (m, 36 H), 3.37 (t, J = 5.3 Hz, 2 H), 3.09 (q, J = 7.3 Hz, 6 H), 2.65-2.52 (m, 4 H), 1.59 (t, J = 7.0 Hz, 2 H), 1.31-1.23 (m, 23 H), 0.90 (t, J = 6.8 Hz, 3 H)
   ³¹P-NMR (CD₃OD) δ (ppm): 1.43
   (R)-2-(11-Undecanamide)-3-((4-decylphenyl)amino)-3-oxoppropylphosphoric acid (RN01-N-C10-azide)
   MS (ESI) m/z: 610.3 [M + H]
   ¹H-NMR (CD₃OD) δ (ppm): 7.37 (d, J = 8.3 Hz, 2 H), 7.02 (d, J = 8.3 Hz, 2 H), 4.54 (t, J = 5.3 Hz, 1 H), 4.10-4.05 (m, 2 H), 3.17 (t, J = 6.8 Hz, 2 H), 2.48 (t, J = 7.7 Hz, 2 H), 2.23 (t, J = 7.2 Hz, 2 H), 1.58-1.46 (m, 6 H), 1.24-1.19 (m, 26 H), 0.81 (t, J = 6.8 Hz, 3 H)
   ³¹P-NMR (CD₃OD) δ (ppm): 1.43
   (R)-17-((4-Decylphenyl)carbamoyl)-1-(2,5-dioxo-2,5-dehydro-1H-pyrrol-1-yl)-15-oxo-3,6,9,12-tetraoxa-16-azaoctadecan-18-yl phosphoric acid (RN01-N-PEG4-maleimide) MS (ESI) m/z: 845.3 [M + H]
   ¹H-NMR (CD₃OD) δ (ppm): 7.46 (d, J= 8.3 Hz, 2H), 7.11 (d, J= 8.3 Hz, 2 H), 6.81(s,2 H), 4.63 (t, J = 5.1 Hz, 1 H), 4.21-4.14 (m, 2 H), 3.83-3.52 (m, 18 H),3.18 (q, J = 7.3 Hz, 6 H), 2.67-2.52 (m, 4 H), 1.59 (t, J = 7.0 Hz, 2 H), 1.32-1.27 (m, 23 H), 0.90 (t, J = 6.8 Hz, 3 H)
   ³¹P-NMR (CD₃OD) δ (ppm): 1.11
   (R)-2-((4-Decylphenyl)carbamoyl)-4,20-dioxa-22-(pyridin-2-yl disulfenyl)-7,10,13,16-tetraoxa-3,19-diazadocosyl phosphoric acid (RN01-N-PEG4SPDP)
MS (ESI) m/z: 845.3 [M + H]
   ¹H-NMR (CD₃OD) δ (ppm): 8.41-8.39 (m, 1 H), 7.85-7.80 (m, 2 H), 7.47 (dd, J = 7.8, 6.1 Hz, 2 H), 7.24-7.20 (m, 1 H), 7.11 (t, J = 8.3 Hz, 2 H), 4.63 (t, J = 5.3 Hz, 1 H), 4.21-4.10 (m, 2 H), 3.79 (m, 2 H), 3.66-3.58 (m, 12 H), 3.53 (t, J = 5.5 Hz, 2 H), 3.35 (t, J = 5.3 Hz, 2 H), 3.06 (t, J = 7.2 Hz, 2 H), 2.65-2.54 (m, 6 H), 1.61-1.58 (m, 2 H), 1.31-1.26 (m, 23 H), 0.89 (t, J = 6.6 Hz, 3 H)
   ³¹P-NMR (CD₃OD) δ (ppm): 1.11

Table 8 shows structural formulae of RN01-N-PEG4-azide, RN01-N-PEG1-azide, RN01-N-PEG9-azide, RN01-N-C10-azide, RN01-N-PEG4-maleimide, and RN01-N-PEG4-SPDP synthesized in Example 1.

**[Table 8]**

| Compound No. | Structural formula |
|---|---|
| RN01-N-PEG4-azide | 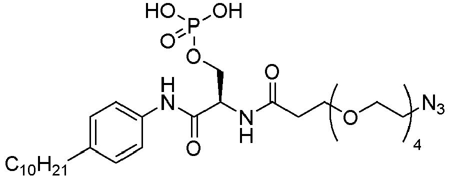 |
| RN01-N-PEG1-azide | 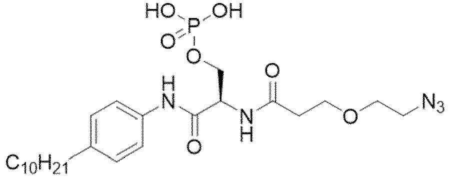 |
| RN01-N-PEG9-azide | 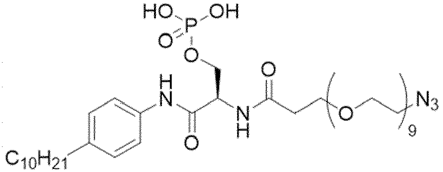 |
| RN01-N-C10-azide | 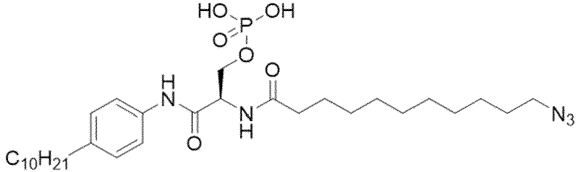 |
| RN01-N-PEG4-Maleimide | 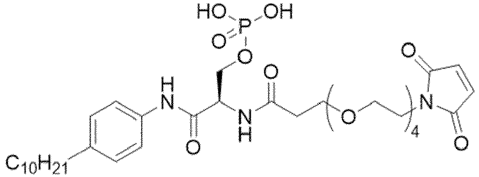 |
| RN01-N-PEG4SPDP | 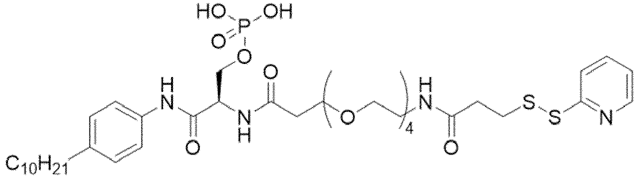 |

### Scheme of synthesis 2

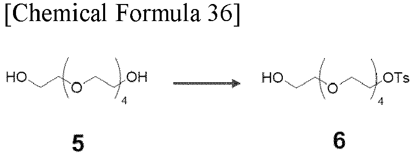

### <Synthesis of 14-hydroxy-3,6,9,12-tetraoxatetradecyl-4-methylbenzenesulfonate (Compound 6)>

Compound 5 (1.22 g) was dissolved in 5 ml of dry dichloromethane, and 700 µl of triethylamine was added. After 959 mg of tosyl chloride was dissolved in 5 ml of dry dichloromethane, the solution was added dropwise to a solution containing Compound 5 under ice cooling. The resultant was agitated under nitrogen atmosphere in an ice bath for 15 minutes and then at room temperature for 40 minutes. Methanol (0.1 ml) was added to the reaction solution, the resultant was agitated at room temperature for 1 hour, the solvent was removed by distillation, and the residue was obtained as white gel (2.90 g). The residue was dissolved in chloroform and purified by silica gel column chromatography (Yamazen universal column silica; ϕ30 × 165 mm; methanol/chloroform (methanol 1%→8%)). Thus, Compound 6 was obtained as colorless transparent oil (994 mg).
MS (ESI) m/z: 393.0 [M + H]
¹H-NMR (CDCl₃) δ (ppm): 7.80 (d, J = 8.3 Hz, 2 H), 7.34 (d, J = 7.9 Hz, 2 H), 4.16 (t, J = 4.9 Hz, 2 H), 3.71-3.59 (m, 18 H),2.57 (br, 1 H), 2.45 (s, 3 H)

### Scheme of synthesis 3

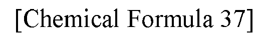

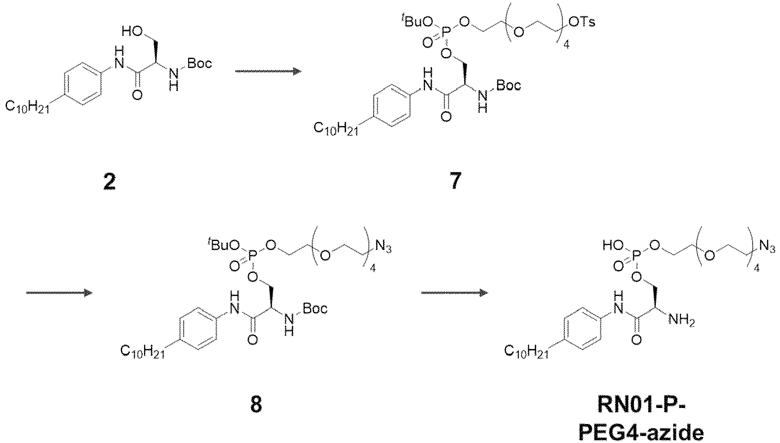

### <Synthesis of 14-((t-butoxy((R)-3-((4-decylphenyl)amino-3-oxo-2-pivalamidepropyloxy)phosphoryl)oxy)3,6,9,12-tetraoxatetradecyl-4-methylbenzenesulfonate (Compound 7)>

Compound 2 (420 mg) and 605 mg of t-butoxy-N,N,N,N-tetra-isopropylphosphinediamine were dissolved in 10 ml of dry dichloromethane, 346 mg of diisopropylammonium tetrazolide was added thereto, and the reaction solution was agitated under nitrogen atmosphere at room temperature for 1 hour. An aqueous solution of saturated sodium bicarbonate (30 ml) was added to the reaction solution, followed by extraction with dichloromethane (20 ml × 3). The organic phase was dried over sodium sulfate, the solvent was removed by distillation, and the residue was obtained as colorless transparent oil (695 mg). The residue and 476 mg of Compound 6 were dissolved in 5 ml of dry tetrahydrofuran, 5 ml of a solution of tetrazole (344 mg) in dry tetrahydrofuran was added dropwise thereto, and the resultant was agitated under nitrogen atmosphere for a day. A solution (0.5 ml) of 35% hydrogen peroxide was added to the reaction solution, and the resultant was agitated at room temperature for 1.5 hours. The reaction solution was dissolved in 50 ml of ethyl acetate and then washed with 50 ml of an aqueous solution of 10% sodium thiosulfate. The organic phase was dried over sodium sulfate, the solvent was removed by distillation, and the residue was obtained as colorless transparent oil (880 mg). The residue was dissolved in chloroform and purified by silica gel column chromatography (Yamazen universal column silica; ϕ30 × 165 mm; ethyl acetate/hexane (ethyl acetate 80%→100%)). Thus, Compound 7 was obtained as colorless transparent oil (139 mg).
MS (ESI) m/z: 929.2 [M-H]
¹H-NMR (CDCl₃) δ (ppm): 8.56 (br, 1 H), 7.79 (d, J = 8.3 Hz, 2 H), 7.45 (d, J = 7.2 Hz, 2 H), 7.33 (d, J = 7.9 Hz, 2 H), 7.11 (d, J = 8.7 Hz, 2 H), 5.94 (m, 1 H), 4.49-4.44 (m, 2 H), 4.25-4.07 (m, 5H),3.69-3.50 (m, 16 H), 2.55 (t, J = 7.7 Hz, 2 H), 2.44 (s, 3 H), 1.55 (m, 2 H), 1.48 (s, 9 H), 1.46 (s, 9 H), 1.30-1.20 (m, 14 H), 0.88 (t, J = 6.6 Hz, 3 H)
³¹P-NMR (CDCl₃) δ (ppm): -5.44

### <Synthesis of 14-azide-3,6,9,12-tetraoxatetradecyl-t-butyl((R)-3-((4-decylphenyl)amino)-3-oxo-2-pivalamide propyl phosphate (Compound 8)>

Compound 7 (139 mg) was dissolved in 1.5 ml of dry N,N-dimethylformamide, 51 mg of sodium azide was added thereto, and the reaction solution was agitated under nitrogen atmosphere at 60°C for 2.5 hours. Ethyl acetate (20 ml) was added to the reaction solution, and the resultant was washed with 20 ml of an aqueous solution of saturated sodium bicarbonate. The organic phase was dried over sodium sulfate, the solvent was removed by distillation, and Compound 8 was obtained as colorless transparent oil (87 mg).
MS (ESI) m/z: 801.2 [M-H]
¹H-NMR (CDCl₃) δ (ppm): 8.63 (br, 1 H), 7.53 (dd, J = 8.3,1.1 Hz, 2 H), 7.11 (d, J = 8.3 Hz, 2 H), 6.03-5.90 (m, 1 H), 4,59-4.49 (m, 2 H), 4.27-4.09 (m, 3 H), 3.69-3.50 (m, 16 H), 3.35 (t, J = 4.9 Hz, 2 H), 2.55 (t, J = 7.7 Hz, 2 H), 1.57 (t, J = 7.4 Hz, 2 H), 1.49 (s, 9 H), 1.47 (s, 9 H), 1.30-1.20 (m, 14 H), 0.88 (t, J = 6.8 Hz, 3 H)
³¹P-NMR (CDCl₃) δ (ppm): -5.48

### <Synthesis of (R)-2-amino-3-((4-decylphenyl)amino)-3-oxopropyl(14-azide-3,6,9,12-tetraoxatetradecyl)phosphate (RN01-P-PEG4-azide)>

To a solution of 87 mg of Compound 8 in 2 ml of dichloromethane, 0.2 ml of trifluoroacetic acid was added, and the resultant was agitated at room temperature for 1 hour. In addition, 0.2 ml of trifluoro was added to the reaction solution, and the resultant was agitated at room temperature for 2 hours. Toluene (2 ml) was added to the reaction solution, the solvent was removed by distillation, and the residue was obtained as colorless transparent oil (79 mg). The residue was dissolved in methanol and purified by reversed-phase chromatography (Yamazen universal column ODS; cp 18 × 114 mm; methanol). Thus, RN01-P-PEG4-azide was obtained as colorless transparent oil (42 mg).
MS (ESI) m/z: 644.1 [M-H]
¹H-NMR (CD₃OD) δ (ppm): 7.49 (d, J = 8.7 Hz, 2 H), 7.15 (d, J = 8.7 Hz, 2 H), 4.36-4.21 (m, 3 H), 4.04-3.98 (m, 2 H), 3.69-3.61 (m, 16 H), 3.37 (t, J = 5.1 Hz, 2 H), 2.58 (t, J = 7.6 Hz, 2 H), 1.59 (t, J = 6.8 Hz, 2 H), 1.32-1.23 (m, 14 H), 0.89 (t, J = 6.8 Hz, 3 H)
³¹P-NMR (CD₃OD) δ (ppm): -0.35

### Scheme of synthesis 4

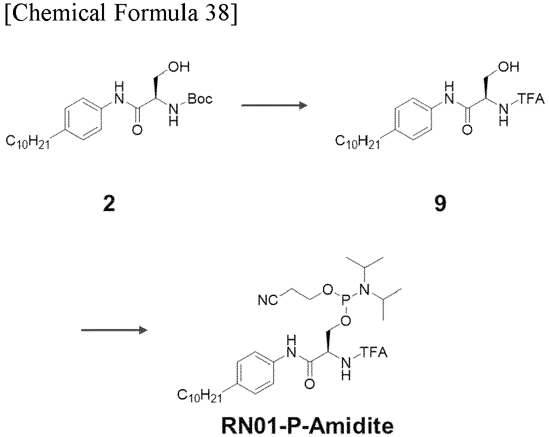

### <Synthesis of (R)-N-(4-decylphenyl)-3-hydroxy-2-(2,2,2-trifluoroacetamide)propanamide (Compound 9)>

Compound 2 (846 mg) was dissolved in 8 ml of dichloromethane, 2 ml of trifluoroacetic acid was added thereto, and the resultant was agitated at room temperature for 3.5 hours. Toluene (5 ml) was added to the reaction solution, and the solvent was removed by distillation. The residue was dissolved in 20 ml of methanol, 1.4 ml of triethylamine and 0.8 ml of trifluoroethyl acetate ester were added thereto, and the resultant was agitated at room temperature for 1.5 hours. After the solvent was removed by distillation, the residue was dissolved in 80 ml of dichloromethane, and the resultant was washed with an aqueous solution of saturated sodium bicarbonate (80 ml × 2) and saturated saline (80 ml). After the organic phase was dried over sodium sulfate, the solvent was removed by distillation, and Compound 9 was obtained as a white solid (771 mg).
MS (ESI) m/z: 415.1 [M-H]
¹H-NMR (CDCl₃) δ (ppm): 8.37 (br, 1 H), 7.60 (br, 1 H), 7.39 (d, J = 8.7 Hz, 2 H), 7.15 (d, J= 8.3 Hz, 2 H), 4.59(td, J= 6.4,3.4 Hz, 1 H), 4.26 (dd, J= 11.3,3.0 Hz, 1 H), 3.76 (dd, J = 11.3,6.0 Hz, 1 H), 3.08 (br, 1 H), 2.57 (t, J = 7.7 Hz, 2 H), 1.58 (t, J = 7.4 Hz, 2 H), 1.39-1.20 (m, 14 H), 0.88 (t, J = 6.6 Hz, 3 H)
¹⁹F-NMR (CDCl₃) δ (ppm): -75.55

### <Synthesis of 2-cyanoethyl (R)-3-((4-decylphenyl)amino-3-oxo-2-(2,2,2-trifluoroacetamide)propylphosphoroamidite (RN01-P-amidite)>

Compound 9 was dissolved in 10 ml of dry dichloromethane, 520 µl of N,N-diisopropylethylamine and 330 □l of 2-cyanoethyl-N,N-diisopropylchlorophosphoroamidite were added thereto, and the reaction solution was agitated under nitrogen atmosphere at room temperature for 1 hour. Dichloromethane (40 ml) was added to the reaction solution, and the resultant was washed with 50 ml of an aqueous solution of saturated sodium bicarbonate and 50 ml of saturated saline. The organic phase was dried over sodium sulfate, the solvent was removed by distillation, and the residue was obtained as yellow tar (666 mg). The residue was dissolved in hexane and ethyl acetate and purified by silica gel column chromatography (Yamazen universal column amino silica; ϕ23 × 123 mm; ethyl acetate/hexane (ethyl acetate 15%→50%, containing 1% triethylamine)). Thus, RN01-P-amidite was obtained as colorless transparent oil (329 mg).
MS (ESI) m/z: 615.2 [M-H]
¹H-NMR (CDCl₃) δ (ppm): 8.22 (d, J = 9.8 Hz, 1 H), 7.57-7.40 (m, 3 H), 7.14 (d, J = 8.3 Hz, 2 H), 4.73 (t, J = 6.4 Hz, 1 H), 4.21-3.55 (m, 6 H), 2.72-2.54 (m, 4 H), 1.58 (t, J = 7.0 Hz, 2 H), 1.29-1.10 (m, 26 H), 0.88 (t, J= 6.6 Hz, 3 H)
¹⁹F-NMR (CDCl₃) δ (ppm): -75.72
³¹PNMR (121 MHz, CDCl₃) δ 151.11,149.23 (for the diastereomer).

### Scheme of synthesis 5

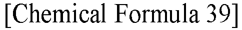

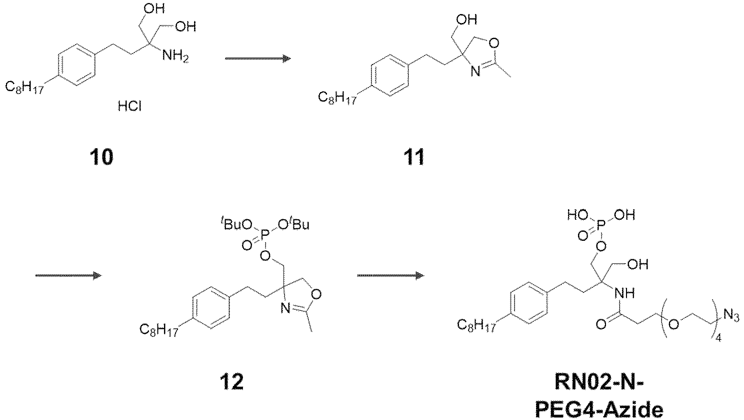

### <Synthesis of (2-methyl-4-(4-octylphenethyl)-4,5-dihydrooxazol-4-yl)methanol (Compound 11)>

2-Amino-2-(4-octylphenethyl)propane-1,3-diol hydrochloride (Compound 10,174 mg) was dissolved in 8 ml of dry N,N-dimethylformamide, 260 µl of N,N-diisopropylethylamine and 75 µl of trimethyl orthoacetate were added thereto, and the reaction solution was agitated under nitrogen atmosphere at 120°C for 3 hours. The reaction solution was cooled to room temperature, 40 ml of ethyl acetate was added thereto, and the resultant was washed with 40 ml of water and 40 ml of saturated saline. The organic phase was dried over sodium sulfate, the solvent was removed by distillation, and Compound 11 was obtained as brown oil (232 mg).
MS (ESI) m/z: 332.2 [M + H]

### <Synthesis of di-t-butyl(2-methyl-4-(4-octylphenethyl))-4,5-dihydrooxazol-4-yl)methyl)phosphate (Compound 12)>

To 232 mg of Compound 11, 8 ml of a solution of 344 mg of tetrazole in dry tetrahydrofuran and 310 µl of di-t-butyl-N,N-diisopropylphosphoroamidite (310µL) were successively added, and the mixture was agitated under nitrogen atmosphere at room temperature for 16 hours. A solution of 35% hydrogen peroxide (0.3 ml) was added to the reaction solution, and the resultant was agitated at room temperature for 3 days. Ethyl acetate (30 ml) was added to the reaction solution, and the organic phase was washed with 30 ml of an aqueous solution of 10% sodium thiosulfate and 30 ml of water. The organic phase was dried over sodium sulfate, the solvent was removed by distillation, and a roughly purified product was obtained as yellow oil (302 mg). The roughly purified product was purified by silica gel column chromatography (Yamazen universal column silica; ϕ30 × 165 mm × 2 columns; ethyl acetate/hexane (ethyl acetate 72%→ 100%)), and Compound 12 was obtained as yellow oil (71 mg).
MS (ESI) m/z: 524.2 [M + H]
¹H-NMR (CDCl₃) δ (ppm): 7.08 (s, 4 H), 4.33 (d, J = 9.1 Hz, 1 H), 3.99 (d, J = 8.7 Hz, 1 H), 3.96-3.85 (m, 2 H), 2.64-2.53 (m, 4 H), 2.00 (s, 3 H), 1.98-1.77 (m, 2 H), 1.57-1.53 (m, 2 H), 1.48 (s, 9 H), 1.47 (s, 9 H)1.27 (m, 10 H),0.87 (t, J= 6.8 Hz, 3 H)
³¹P-NMR (CDCl₃) δ (ppm): -9.77

### <Synthesis of 1-azide-17-(hydroxymethyl)-17-(4-octylphenethyl)-15-oxo-3,6,9,12-tetraoxa-16-azaoctadeca-18-nyl phosphate (RN02-N-PEG4-azide)>

Compound 12 (71 mg) was dissolved in 5 ml of ethanol, 1 ml of concentrated hydrochloric acid was added thereto, and the reaction solution was agitated at 50°C for 4 hours. Toluene (2 ml) was added to the reaction solution, the solvent was removed by distillation, and the residue was obtained as white foam. A dichloromethane solution (2 ml) containing 1 ml of dichloromethane, 400 µl of triethylamine, and 57 mg of 2,5-dioxypyrrolidi-1-nyl 1-azide-3,6,9,12-tetraoxapentadeca-15-noate was added to the residue, and the resultant was agitated at room temperature for 24 hours. The solvent was removed from the reaction solution by distillation, the resultant was dissolved in an aqueous solution of acetonitrile/triethyl ammonium bicarbonate (0.1 M) (1:1), purified by column chromatography (Luna Omega Polar C18; Φ21.2 × 250 mm; an aqueous solution of acetonitrile/triethyl ammonium bicarbonate (0.1M) (acetonitrile 50%)), and triethylamine salt of RN02-N-PEG4-azide was obtained as colorless transparent oil (17 mg).
MS (ESI) m/z: 661.1 [M + H]
¹H-NMR (CD₃OD) δ (ppm): 7.12-7.03 (m, 4 H), 4.14-3.98 (m, 2 H), 3.85-3.56 (m, 16 H), 3.39-3.33 (m, 2 H), 3.18 (q, J = 7.3 Hz, 4 H), 2.63-2.52 (m, 4 H), 2.48 (dd, J = 6.0 Hz, 2 H), 2.13-2.00 (m, 2 H), 1.58 (t, J = 6.4 Hz, 2 H), 1.37-1.18 (m, 17 H), 0.89 (t, J = 6.8 Hz, 3 H)
³¹P-NMR (CD₃OD) δ (ppm): 1.45

### Scheme of synthesis 6

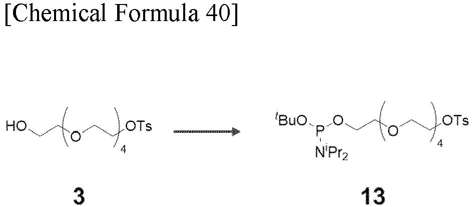

### <Synthesis of 14-azide-3,6,9,12-tetraoxatetradecyl-t-butyldiisopropylphosphoroamidite (Compound 13)>

Compound 3 (438 mg) was dissolved in 10 ml of dry dichloromethane, 259 mg of diisopropyl ammonium tetrazolide and 612 mg of t-butoxy-N,N,N,N-tetra-isopropylphosphinediamine were added thereto, and the reaction solution was agitated under nitrogen atmosphere at room temperature for 3 hours. Dichloromethane (40 ml) was added to the reaction solution, and the organic phase was washed with 50 ml of an aqueous solution of saturated sodium bicarbonate. The organic phase was dried over sodium sulfate, the solvent was removed by distillation, and a roughly purified product was obtained as colorless transparent oil (1.15 g). The roughly purified product was purified by silica gel column chromatography (Yamazen universal column amino; ϕ23 × 123 mm × 2 columns; ethyl acetate/1% triethylamine-containing hexane (ethyl acetate 4%→25%)), and Compound 13 was obtained as colorless transparent oil (438 mg).
MS (ESI) m/z: 540.2 [M-Bu+2H]
¹H-NMR (CDCl₃) δ (ppm): 7.82 (d, J= 8.3 Hz, 2 H), 7.36 (d, J = 7.9 Hz, 2 H), 4.18 (t, J = 4.9 Hz, 2 H), 3.78-3.55 (m, 20H),2.47 (s, 3 H), 1.36 (s, 9 H), 1.18 (t, J = 6.4 Hz, 12 H)
³¹P-NMR (CDCl₃) δ (ppm): 137.43

### Scheme of synthesis 7

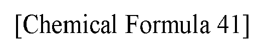

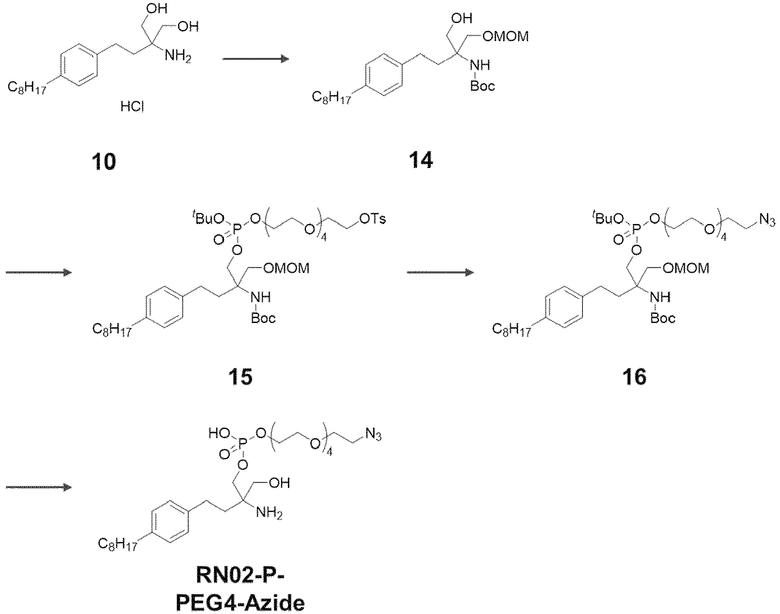

### <Synthesis of t-butyl(1-hydroxy-2-((methoxymethoxy)methyl)-4-(octylphenyl)butan-2-yl)carbamate (Compound 14)>

Compound 10 (349/ mg) was dissolved in 7 ml of dry methanol, 520 µl of N,N-diisopropylethylamine and 460 µl of di-t-butyl dicarbonate were added thereto, the mixture was agitated under nitrogen atmosphere at room temperature for 3 hours, 230 µl of di-t-butyl dicarbonate was added again, and the resultant was agitated under nitrogen atmosphere at room temperature for 19 hours. An aqueous solution of saturated sodium bicarbonate (30 ml) was added to the reaction solution, the resultant was extracted with 60 ml of ethyl acetate, and the organic phase was washed with saturated saline. The organic phase was dried over sodium sulfate, the solvent was removed by distillation, and colorless transparent oil was obtained. Dry dichloromethane (6 ml) and 250 µl of N,N-diisopropylethylamine were added to the colorless transparent oil, the resultant was cooled to 0°C, 90 µl of chloromethyl methyl ether was added, and the resultant was agitated under nitrogen atmosphere at room temperature for 3 hours. Methanol (1 ml) was added to the reaction solution, the mixture was agitated at room temperature for 14 hours, the reaction solution was dissolved in 30 ml of the dichloromethane, and the resultant was then washed with saturated saline. The organic phase was dried over sodium sulfate, the solvent was removed by distillation, and a roughly purified product was obtained as colorless transparent oil (505 mg). The roughly purified product was dissolved in 3 ml of a solution of ethyl acetate/hexane (1:1) and purified by silica gel column chromatography (Yamazen universal column silica; ϕ23 × 123 mm × 2 columns; ethyl acetate/hexane (ethyl acetate 15%→40%)). Thus, Compound 14 was obtained as colorless transparent oil (170 mg).
MS (ESI) m/z: 352.0[M-Boc+2H]
¹H-NMR (CDCl₃) δ (ppm): 7.08 (d, J = 9.4 Hz, 4 H), 5.14 (br, 1 H), 4.64 (s, 2 H), 4.05 (br, 1 H), 3.80-3.50 (m, 4 H), 3.39 (s, 3 H), 2.68-2.48 (m, 4 H), 2.11-1.86 (m, 2 H), 1.60-1.56 (m, 2 H), 1.45 (s, 9 H), 1.27 (m, 10 H),0.87 (t, J = 6.8 Hz, 3 H)

### <Synthesis of 14-((t-butoxy(2-((t-butoxycarbonyl)amino)-2-((methoxymethoxy)methyl)-4-(4-octylphenyl)butoxy)phosphoryl)-3,6,9,12-tetraoxatetradecyl-4-methylbenzenesulfonate (Compound 15)>

Compound 13 (438 mg) and Compound 14 (170 mg) were dissolved in 6 ml of dry dichloromethane, 2 ml of a solution of tetrazole (86 mg) in dry acetonitrile was added thereto, and the reaction solution was agitated under nitrogen atmosphere at room temperature for 22 hours. Thereafter, a solution of t-butylhydroperoxide/n-decane (about 5.5 M, 270 µl) was added, and the mixture was agitated under nitrogen atmosphere at room temperature for 26 hours. Ethyl acetate (50 ml) was added to the reaction solution, and the organic phase was washed with 50 ml of an aqueous solution of 10% sodium thiosulfate. The organic phase was dried over sodium sulfate, the solvent was removed by distillation, and a roughly purified product was obtained as pale yellow oil (572 mg). The roughly purified product was purified by silica gel column chromatography (Yamazen universal column silica; ϕ23 × 123 mm; methanol/ethyl acetate (methanol 0%→17%)), and Compound 15 (a diastereomeric mixture) was obtained as colorless transparent oil (269 mg).
MS (ESI) m/z: 979.3[M+NH₄]
¹H-NMR (CDCl₃) δ (ppm): 7.80 (d, J = 8.3 Hz, 2 H), 7.34 (d, J = 8.3 Hz, 2 H), 7.11-7.05 (m, 4 H), 5.00 (br, 1 H), 4.62 (s, 2 H), 4.18-4.10 (m, 6 H), 3.75-3.58 (m, 18 H), 3.36 (s, 3 H), 2.62-2.52 (m, 4 H), 2.45 (s, 3 H), 2.13-2.10 (m, 2 H), 1.60-1.53 (m, 2 H), 1.50 (s, 9 H), 1.44 (s, 9 H), 1.32-1.23 (m, 10 H),0.87 (t, J = 6.8 Hz, 3 H)
³¹P-NMR (CDCl₃) δ (ppm): -5.32,-5.43

### <Synthesis of t-butyl(1-((((14-azide-3,6,9,12-tetraoxatetradecyl)oxy)(hydroxy)phosphoryl)oxy)-2-((methoxymethoxy)methyl)-4-(4-octylphenyl)butan-2-yl)carbamate (Compound 16)>

Compound 15 (269 mg) was dissolved in 8 ml of dry N,N-dimethylformamide, 96 mg of sodium azide was added thereto, and the resultant was agitated under nitrogen atmosphere at 80°C for 1.5 hours. Ethyl acetate (50 ml) was added to the reaction solution, and the mixture was washed with 50 ml of an aqueous solution of saturated sodium bicarbonate. The organic phase was dried over sodium sulfate, the solvent was removed by distillation, and Compound 16 was obtained as pale yellow oil (228 mg).
MS (ESI) m/z: 850.3[M+NH₄]

### <Synthesis of 2-amino-2-(hydroxymethyl)-4-(4-octylphenyl)butyl(14-azide-3,6,9,12-tetraoxatetradecyl)phosphate (RN02-P-PEG4-azide)>

Compound 16 (97 mg) was dissolved in 4 ml of THF, 0.5 ml of water, and 0.5 ml of hydrochloric acid (6 M), and the reaction solution was agitated at 50°C for 2 days. Triethylamine (0.6 ml) was added to the reaction solution, the solvent was removed by distillation, and a roughly purified product was obtained as a white solid (464 mg). The roughly purified product was dissolved in an aqueous solution of acetonitrile/triethyl ammonium bicarbonate (0.1 M) (1:1), purified by column chromatography (Luna Omega Polar C18AXIA 5 mm; Φ21.2 × 250 mm; an aqueous solution of acetonitrile/triethyl ammonium bicarbonate (0.1 M) (acetonitrile 50%→90%)), and RN02-P-PEG4-azide was obtained as colorless transparent oil (25 mg).
MS (ESI) m/z: 631.2 [M-H]
¹H-NMR (CD₃OD) δ (ppm): 7.12 (dd, J = 18.3,8.1 Hz, 4 H), 4.10-3.99 (m, 4 H), 3.76-3.59 (m, 18 H),3.35 (t, J = 4.9 Hz, 2 H), 2.66(td, J = 8.7,6.4 Hz, 2 H), 2.56 (t, J = 7.7 Hz, 2 H), 2.01-1.95 (m, 2 H), 1.58 (t, J = 7.2 Hz, 2 H), 1.30 (d, J = 7.2 Hz, 10 H),0.89 (t, J = 6.6 Hz, 3 H) ³¹P-NMR (CD₃OD) δ (ppm): 0.04

### [Example 2]

The nucleic acid complex of the present invention is not particularly limited, provided that it has an antisense strand that can hybridize to a target gene or a target transcription product and has antisense effects. In the case of a duplex complex, a complementary strand that can hybridize to an antisense strand can further be used in combination. For example, a nucleic acid molecule can be selected from among ASO, HDO, and siRNA comprising the sequences shown below. When a different disease is targeted, the target transcription product is different. Accordingly, a nucleic acid molecule having an antisense strand comprising a relevant nucleic acid sequence in accordance with the target can be used.

In some embodiments, a nucleic acid complex comprising ASO having an antisense strand consisting of any of the following nucleic acid sequences and/or HDO having a strand complementary to the antisense strand to which any of the S1P ligands shown in Table 9 or an S1P ligand-linker conjugate has been bound can be used.

**[Table 9]**

| Compound No. | Structural formula |
|---|---|
| RN01-N-PEG4-azide | 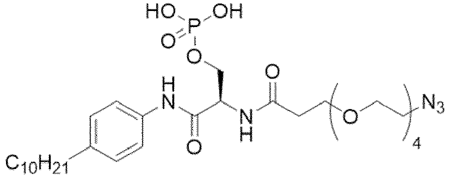 |
| RN01-P-PEG4-azide | 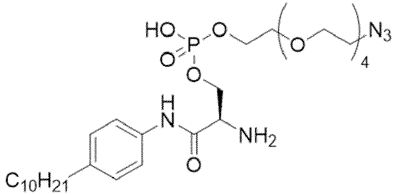 |
| RN02-N-PEG4-azide | 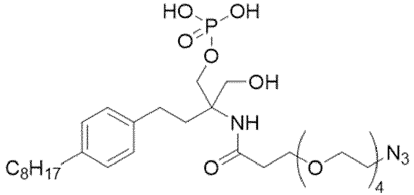 |
| RN02-P-PEG4-azide | 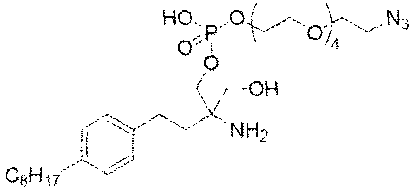 |
| RN01-N-PEG1 -azide | 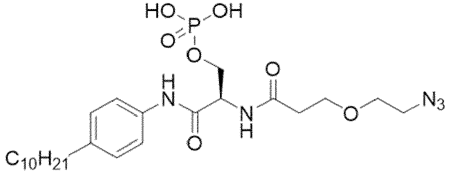 |
| RN01-N-PEG9-azide | 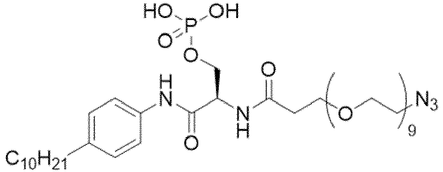 |
| RN01-N-C10-azide | 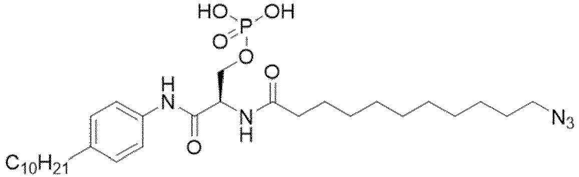 |
| RN01-N-PEG4-maleimide | 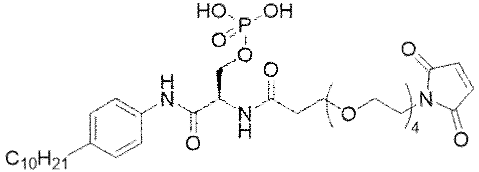 |
| RN01-P-Aaidite | 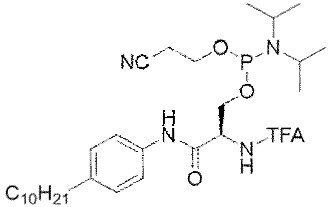 |
| RN01-N-PEG4SPDP | 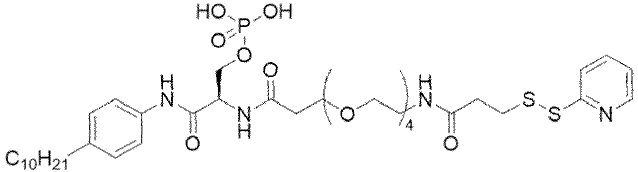 |

In some embodiments, a nucleic acid complex comprising the S1P ligand shown in Table 9 bound thereto can be used. Table 10 shows nucleic acid complexes each comprising the S1P ligand shown in Table 9 bound to ASO having an antisense strand consisting of any of the following nucleic acid sequences and/or HDO having a sequence complementary to the antisense strand. In each HDO shown in Table 9, the S1P ligand binds to a complementary strand of HDO.

**[Table 10]**

| Oligo | Type | Gene No. | Target gene | Ligand mother skeleton 1 | Ligand mother skeleton 2 | Site of binding | Spacer | Spacer No. | ASO | ASO sequence | SEQ ID NO: | SO | Sequence of complementary strand | SEQ ID NO: |
|---|---|---|---|---|---|---|---|---|---|---|---|---|---|---|
| RN01-N-SP01-ASO01 | ASO | 1 | IHH | VPC | RN01 | N | PEG4-DBCO-C6 | SP01 | ASO01 | A*A*T*g*c*a*c*g*t *g*g*G*C*C | 1 | | | |
| RN01-N-SP01-ASO03 | ASO | 3 | DMPK | VPC | RN01 | N | PEG4-DBCO-C6 | SP01 | ASO03 | A*C*A*a*t*a*a*a*t *a*c*c*g*A*G*G | 5 | | | |
| RN01-N-SP01-ASO05 | ASO | 5 | IL-1 | VPC | RN01 | N | PEG4-DBCO-C6 | SP01 | ASO05 | G*C*A*c*a*c*u*u* c*c*a*a*u*a*c*u*u* A*C*C | 9 | | | |
| RN01-N-SP01-HDO01 | HDO | 1 | IHH | VPC | RN01 | N | PEG4-DBCO-C6 | SP01 | ASO01 | A*A*T*g*c*a*c*g*t *g*g*G*C*C | 1 | SO01 | G*G*C*CCACG UGC*A*U*U | 2 |
| RN01-N-SP01-HDO03 | HDO | 3 | DMPK | VPC | RN01 | N | PEG4-DBCO-C6 | SP01 | ASO03 | A*C*A*a*t*a*a*a*t *a*c*c*g*A*G*G | 5 | SO03 | C*C*U*CGGUA UUUAU*U*G* U | 6 |
| RN01-N-SP01-HDO05 | HDO | 5 | IL-1 | VPC | RN01 | N | PEG4-DBCO-C6 | SP01 | ASO05 | G*C*A*c*a*c*u*u* c*c*a*a*u*a*c*u*u* A*C*C | 9 | SO05 | G*G*U*aaguau uggaagug*U*G* C | 10 |
| RN01-N-SP01-HDO06 | HDO | 6 | Malat-1 | VPC | RN01 | N | PEG4-DBCO-C6 | SP01 | ASO06 | C*T*A*g*t*t*c*a*c* t*g*a*a*T*G*C | 11 | SO06 | G*C*A*UUCAG UGAAC*U*A*G | 12 |
| RN01-N-SP02-HDO06 | HDO | 6 | Malat-1 | VPC | RN01 | N | PEG1-DBCO-C6 | SP02 | ASO06 | C*T*A*g*t*t*c*a*c* t*g*a*a*T*G*C | 11 | SO06 | G*C*A*UUCAG UGAAC*U*A*G | 12 |
| RN01-N-SP03-HDO06 | HDO | 6 | Malat-1 | VPC | RN01 | N | PEG9-DBCO-C6 | SP03 | ASO06 | C*T*A*g*t*t*c*a*c* t*g*a*a*T*G*C | 11 | SO06 | G*C*A*UUCAG UGAAC*U*A*G | 12 |
| RN01-N-SP04-HDO06 | HDO | 6 | Malat-1 | VPC | RN01 | N | C10-DBCO-C6 | SP04 | ASO06 | C*T*A*g*t*t*c*a*c* t*g*a*a*T*G*C | 11 | SO06 | G*C*A*UUCAG UGAAC*U*A*G | 12 |
| RN01-N-SP05-HDO06 | HDO | 6 | Malat-1 | VPC | RN01 | N | PEG4-BCN-C6 | SP05 | ASO06 | C*T*A*g*t*t*c*a*c* t*g*a*a*T*G*C | 11 | SO06 | G*C*A*UUCAG UGAAC*U*A*G | 12 |
| RN01-N-SP06-HDO06 | HDO | 6 | Malat-1 | VPC | RN01 | N | PEG4-SS-C6 | SP06 | ASO06 | C*T*A*g*t*t*c*a*c* t*g*a*a*T*G*C | 11 | SO06 | G*C*A*UUCAG UGAAC*U*A*G | 12 |
| RN01-N-SP07-HDO06 | HDO | 6 | Malat-1 | VPC | RN01 | N | PEG4-Mal-Thio-C6 | SP07 | ASO06 | C*T*A*g*t*t*c*a*c* t*g*a*a*T*G*C | 11 | SO06 | G*C*A*UUCAG UGAAC*U*A*G | 12 |
| RN01-P-SP01-ASO02 | ASO | 2 | APOB | VPC | RN01 | P | PEG4-DBCO-C6 | SP01 | ASO02 | G*C*a*t*t*g*g*t*a*t *T*C*A | 3 | | | |
| RN01-P-SP01-ASO04 | ASO | 4 | mDystrophin | VPC | RN01 | P | PEG4-DBCO-C6 | SP01 | ASO04 | ggccaaacctcggcttacct gaaat | 7 | | | |
| RN01-P-SP01-ASO04 | ASO | 4 | mDystrophin | VPC | RN01 | P | PEG4-DBCO-C6 | SP01 | ASO04 | ggccaaacctcggcttacct gaaat | 7 | | | |
| RN01-P-SP01-ASO06 | ASO | 6 | Malat-1 | VPC | RN01 | P | PEG4-DBCO-C6 | SP01 | ASO06 | C*T*A*g*t*t*c*a*c* t*g*a*a*T*G*C | 11 | | | |
| RN01-P-SP01-HDO02 | HDO | 2 | APOB | VPC | RN01 | P | PEG4-DBCO-C6 | SP01 | ASO02 | G*C*a*t*t*g*g*t*a*t *T*C*A | 3 | SO02 | U*G*A*A*UAC CAAU*G*C | 4 |
| RN01-P-SP01-HDO04 | HDO | 4 | mDystrophin | VPC | RN01 | P | PEG4-DBCO-C6 | SP01 | ASO04 | ggccaaacctcggcttacct gaaat | 7 | SO04 | A*U*U*UCAGG UAAGCCGAGG UUUAG*G*C* C | 8 |
| RN01-P-SP01-HDO06 | HDO | 6 | Malat-1 | VPC | RN01 | P | PEG4-DBCO-C6 | SP01 | ASO06 | C*T*A*g*t*t*c*a*c* t*g*a*a*T*G*C | 11 | SO06 | G*C*A*UUCAG UGAAC*U*A*G | 12 |
| RN01-P-SP01-SO06 | SO | 6 | Malat-1 | VPC | RN01 | P | PEG4-DBCO-C6 | SP01 | | | | SO06 | G*C*A*UUCAG UGAAC*U*A*G | 12 |
| RN01-P-SP08-HDO06 | HDO | 6 | Malat-1 | VPC | RN01 | P | Direct | SP08 | ASO06 | C*T*A*g*t*t*c*a*c* t*g*a*a*T*G*C | 11 | SO06 | G*C*A*UUCAG UGAAC*U*A*G | 12 |
| RN02-N-SP01-ASO01 | ASO | 1 | IHH | FTY-P | RN02 | N | PEG4-DBCO-C6 | SP01 | ASO01 | A*A*T*g*c*a*c*g*t *g*g*G*C*C | 1 | | | |
| RN02-N-SP01-ASO02 | ASO | 2 | APOB | FTY-P | RN02 | N | PEG4-DBCO-C6 | SP01 | ASO02 | G*C*a*t*t*g*g*t*a*t *T*C*A | 3 | | | |
| RN02-N-SP01-ASO03 | ASO | 3 | DMPK | FTY-P | RN02 | N | PEG4-DBCO-C6 | SP01 | ASO03 | A*C*A*a*t*a*a*a*t *a*c*c*g*A*G*G | 5 | | | |
| RN02-N-SP01-ASO04 | ASO | 4 | mDystrophin | FTY-P | RN02 | N | PEG4-DBCO-C6 | SP01 | ASO04 | ggccaaacctcggcttacct gaaat | 7 | | | |
| RN02-N-SP01-ASO05 | ASO | 5 | IL-1 | FTY-P | RN02 | N | PEG4-DBCO-C6 | SP01 | ASO05 | G*C*A*c*a*c*u*u* c*c*a*a*u*a*c*u*u* A*C*C | 9 | | | |
| RN02-N-SP01-ASO06 | ASO | 6 | Malat-1 | FTY-P | RN02 | N | PEG4-DBCO-C6 | SP01 | ASO06 | C*T*A*g*t*t*c*a*c* t*g*a*a*T*G*C | 11 | | | |
| RN02-N-SP01-HDO01 | HDO | 1 | IHH | FTY-P | RN02 | N | PEG4-DBCO-C6 | SP01 | ASO01 | A*A*T*g*c*a*c*g*t *g*g*G*C*C | 1 | SO01 | G*G*C*CCACG UGC*A*U*U | 2 |
| RN02-N-SP01-HDO02 | HDO | 2 | APOB | FTY-P | RN02 | N | PEG4-DBCO-C6 | SP01 | ASO02 | G*C*a*t*t*g*g*t*a*t *T*C*A | 3 | SO02 | U*G*A*A*UAC CAAU*G*C | 4 |
| RN02-N-SP01-HDO03 | HDO | 3 | DMPK | FTY-P | RN02 | N | PEG4-DBCO-C6 | SP01 | ASO03 | A*C*A*a*t*a*a*a*t *a*c*c*g*A*G*G | 5 | SO03 | C*C*U*CGGUA UUUAU*U*G* U | 6 |
| RN02-N-SP01-HDO04 | HDO | 4 | mDystrophin | FTY-P | RN02 | N | PEG4-DBCO-C6 | SP01 | ASO04 | ggccaaacctcggcttacct gaaat | 7 | SO04 | A*U*U*UCAGG UAAGCCGAGG UUUAG*G*C* C | 8 |
| RN02-N-SP01-HDO05 | HDO | 5 | IL-1 | FTY-P | RN02 | N | PEG4-DBCO-C6 | SP01 | ASO05 | G*C*A*c*a*c*u*u* c*c*a*a*u*a*c*u*u* A*C*C | 9 | SO05 | G*G*U*aaguau uggaagug*U*G* C | 10 |
| RN02-N-SP01-HDO06 | HDO | 6 | Malat-1 | FTY-P | RN02 | N | PEG4-DBCO-C6 | SP01 | ASO06 | C*T*A*g*t*t*c*a*c* t*g*a*a*T*G*C | 11 | SO06 | G*C*A*UUCAG UGAAC*U*A*G | 12 |
| RN02-P-SP01-ASO01 | ASO | 1 | IHH | FTY-P | RN02 | P | PEG4-DBCO-C6 | SP01 | ASO01 | A*A*T*g*c*a*c*g*t *g*g*G*C*C | 1 | | | |
| RN02-P-SP01-ASO02 | ASO | 2 | APOB | FTY-P | RN02 | P | PEG4-DBCO-C6 | SP01 | ASO02 | G*C*a*t*t*g*g*t*a*t *T*C*A | 3 | | | |
| RN02-P-SP01-ASO03 | ASO | 3 | DMPK | FTY-P | RN02 | P | PEG4-DBCO-C6 | SP01 | ASO03 | A*C*A*a*t*a*a*a*t *a*c*c*g*A*G*G | 5 | | | |
| RN02-P-SP01-ASO04 | ASO | 4 | mDystrophin | FTY-P | RN02 | P | PEG4-DBCO-C6 | SP01 | ASO04 | ggccaaacctcggcttacct gaaat | 7 | | | |
| RN02-P-SP01-ASO05 | ASO | 5 | IL-1 | FTY-P | RN02 | P | PEG4-DBCO-C6 | SP01 | ASO05 | G*C*A*c*a*c*u*u* c*c*a*a*u*a*c*u*u* A*C*C | 9 | | | |
| RN02-P-SP01-ASO06 | ASO | 6 | Malat-1 | FTY-P | RN02 | P | PEG4-DBCO-C6 | SP01 | ASO06 | C*T*A*g*t*t*c*a*c* t*g*a*a*T*G*C | 11 | | | |
| RN02-P-SP01-HDO01 | HDO | 1 | IHH | FTY-P | RN02 | P | PEG4-DBCO-C6 | SP01 | ASO01 | A*A*T*g*c*a*c*g*t *g*g*G*C*C | 1 | SO01 | G*G*C*CCACG UGC*A*U*U | 2 |
| RN02-P-SP01-HDO02 | HDO | 2 | APOB | FTY-P | RN02 | P | PEG4-DBCO-C6 | SP01 | ASO02 | G*C*a*t*t*g*g*t*a*t *T*C*A | 3 | SO02 | U*G*A*A*UAC CAAU*G*C | 4 |
| RN02-P-SP01-HDO03 | HDO | 3 | DMPK | FTY-P | RN02 | P | PEG4-DBCO-C6 | SP01 | ASO03 | A*C*A*a*t*a*a*a*t *a*c*c*g*A*G*G | 5 | SO03 | C*C*U*CGGUA UUUAU*U*G* U | 6 |
| RN02-P-SP01-HDO04 | HDO | 4 | mDystrophin | FTY-P | RN02 | P | PEG4-DBCO-C6 | SP01 | ASO04 | ggccaaacctcggcttacct gaaat | 7 | SO04 | A*U*U*UCAGG UAAGCCGAGG UUUAG*G*C* C | 8 |
| RN02-P-SP01-HDO05 | HDO | 5 | IL-1 | FTY-P | RN02 | P | PEG4-DBCO-C6 | SP01 | ASO05 | G*C*A*c*a*c*u*u* c*c*a*a*u*a*c*u*u* A*C*C | 9 | SO05 | G*G*U*aaguau uggaagug*U*G* C | 10 |
| RN02-P-SP01-HDO06 | HDO | 6 | Malat-1 | FTY-P | RN02 | P | PEG4-DBCO-C6 | SP01 | ASO06 | C*T*A*g*t*t*c*a*c* t*g*a*a*T*G*C | 11 | SO06 | G*C*A*UUCAG UGAAC*U*A*G | 12 |

In Table 10, a lowercase letter represents DNA, an underlined uppercase letter represents LNA (C represents LNA methylcytosine), an uppercase letter represents RNA, an uppercase italic letter represents 2'-O-methyl sugar modification, a lowercase italic letter represents a morpholinonucleic acid, and an asterisk represents a phosphorothioate bond.

In Example 2, the S1 ligand-binding HDO targeting mouse Malat1 ncRNA was prepared with the use of the antisense strand ASO06 (SEQ ID NO: 11), the complementary strand SO06 (SEQ ID NO: 12), the RN001 ligand, and the RN02 ligand.
ASO06 (SEQ ID NO: 11): 5'-C*T*A*g*t*t*c*a*c*t*g*a*a*T*G*C-3'
SO06 (SEQ ID NO: 12): 5'-G*C*A*UUCAGUGAAC*U*A*G-3'

A lowercase letter represents DNA, an underlined uppercase letter represents LNA (C represents LNA methylcytosine), an uppercase letter represents RNA, a double-underlined uppercase letter represents 2'-O-methyl sugar modification, and an asterisk represents a phosphorothioate bond.

ASO06 (SEQ ID NO: 11) is an antisense strand consisting of 16 nucleic acids (16mer) targeting mouse Malat1 ncRNA, ASO06 targets mouse Malat1 ncRNA, and ASO06 comprises a sequence that can hybridize to the target site of mouse Malat1 ncRNA.

SO06 (SEQ ID NO: 12) is a complementary strand consisting of 16 nucleic acids (16mer) having a sequence complementary to the antisense strand ASO06 (SEQ ID NO: 11), and SO06 comprises a sequence that can hybridize to ASO06.

At the outset, the 5' terminus of SO06 was modified with a dibenzocyclooctyne-succinyl-hexylamino group.

The sequence indicated below was prepared in the manner described below:
5'-XG*C*A*UUCAGUGAAC*U*A*G-3' (wherein X represents a dibenzocyclooctyne-succinyl-hexylamino group, * represents a phosphorothioate bond, an uppercase italic letter represents 2'-O-methyl sugar modification, and an uppercase letter represents RNA).

The RN01-N-PEG4-azide solution (0.5 mM, 12 ml) prepared in Example 1 was added to SO06 (1.0 mM, 3.0 ml), and the mixture was agitated and then allowed to stand for 5 minutes to 36 hours. The resultant was diluted in an aqueous solution of 8 mM triethylamine, 100 mM hexafluoroisopropanol, and 5% methanol and purified by HPLC (column: YMC-Triart C18, 150 × 10.0 mm ID S-5 µm; eluate A: an aqueous solution of 8 mM triethylamine, 100 mM hexafluoroisopropanol, and 5% methanol; eluate B: an aqueous solution of 80% methanol; elution condition: 4.7 ml/min, 0-60% B). The solvent was removed by distillation under reduced pressure, the resultant was dissolved again in 1 ml of water, 30 µl of an aqueous solution of sodium chloride (5.0 M) was added thereto, the mixture was agitated, and 4 ml of EtOH was added thereto, followed by centrifugation. The precipitate obtained was washed with 80% EtOH and dried under reduced pressure to obtain RN01-N-SP01-SO06.

In the same manner as described above, an antisense strand or a complementary strand comprising a different nucleic acid sequence may be used instead of the nucleic acid strand SO06, so that a nucleic acid strand comprising the S1P ligand bound thereto can be prepared.

In some embodiments, RN01-N-SP01-ASO06 was prepared in the same manner as described above, except for the use of the sequence ASO06 instead of SO06.

In another embodiment, RN02-N-SP01-ASO06 was prepared with the use of the sequence ASO06 instead of SO06 and RN02-N-PEG4-azide instead of RN01-N-PEG4-azide.

HDO comprising the S1P ligand bound thereto was prepared by the method described below. ASO06 (0.2 mM, 750 µl) was mixed with RN01-N-SP01-SO06 (0.20 mM, 750 µl), the solvent was removed therefrom, and HDO comprising a ligand bound thereto; i.e., RN01-N-SP01-HDO06 (0.1 mM, 1.5 ml), was obtained. Physiological saline (Otsuka Normal Saline (Otsuka Pharmaceutical Co., Ltd.)) was added to RN01-N-SP01-HDO06, the solution was heated at 95°C for 5 minutes, and the solution was slowly cooled to room temperature for annealing.

In other embodiments, RN01-P-SP01-HDO06, RN01-N-SP02-HDO06, RN01-N-SP03-HDO06, RN01-N-SP04-HDO06, RN02-N-SP01-HDO06, and RN02-P-SP01-HDO06 were prepared with the use of RN01-P-PEG4-azide, RN01-N-PEG1-azide, RN01-N-PEG9-azide, RN01-N-C10-azide, RN02-N-PEG4-azide, and RN02-P-PEG4-azide, respectively, instead of RN01-N-PEG4-azide.

In another embodiment, RN01-N-SP05-HDO was obtained with the use of the 5' bicyclooctyne-succinyl-hexylamino oligonucleic acid instead of the 5' dibenzocyclooctyne-succinyl-hexylamino oligonucleic acid.

Tm of each HDO was measured. The measured values are shown in Table 11.

**[Table 11]**

| Name of oligonucleotides | Tm |
|---|---|
| RN01-N-SP01-HDO06 | 64°C |
| RN01-P-SP01-HDO06 | 65°C |
| RN01-N-SP02-HDO06 | 63°C |
| RN01-N-SP03-HDO06 | 63°C |
| RN01-N-SP04-HDO06 | 63°C |
| RN01-N-SP05-HDO06 | 62°C |
| RN02-N-SP01-HDO06 | 62°C |
| RN02-P-SP01-HDO06 | 63°C |

In another embodiment, tris(2-carboxyethyl)phosphine (0.1 M, 0.1 ml) was added to 5' 3-(2-pyridyldithiopropionyl-hexylamino-SO006 (1.0 mM, 1.0 ml) comprising the complementary strand SO06 (SEQ ID NO: 12) modified at its 5' terminus with the 3-(2-pyridyldithiopropionyl-hexylamino group and the mixture was then agitated:
SEQ ID NO: 006: 5'-X*G***C***A**UUCAGUGAAC**U***A***G*-3' (wherein X represents a 3-(2-pyridyldithiopropionyl-hexylamino group; * represents a phosphorothioate bond; an uppercase italic letter represents 2'-O-methyl sugar modification; and an uppercase letter represents RNA). The resultant was diluted with an aqueous solution of 8 mM triethylamine, 100 mM hexafluoroisopropanol, and 5% methanol and then purified by HPLC (column: YMC-Triart C18, 150 × 10.0 mm ID S-5 µm; eluate A: an aqueous solution of 8 mM triethylamine, 100 mM hexafluoroisopropanol, and 5% methanol; eluate B: an aqueous solution of 80% methanol; elution condition: 4.7 ml/min, 0-60% B). RN01-N-PEG4-SPDP was added to the resulting solution, and the mixture was agitated and then allowed to stand for 5 minutes to 36 hours. The resultant was diluted in an aqueous solution of 8 mM triethylamine, 100 mM hexafluoroisopropanol, and 5% methanol and purified by HPLC (column: YMC-Triart C18, 150 × 10.0 mm ID S-5µm; eluate A: an aqueous solution of 8 mM triethylamine, 100 mM hexafluoroisopropanol, and 5% methanol; eluate B: an aqueous solution of 80% methanol; elution condition: 4.7 ml/min, 0-60% B). The solvent was removed by distillation, the resultant was dissolved again in 1 ml of water, an aqueous solution of sodium chloride (5.0 M, 30 µl) was added thereto, the mixture was agitated, and 4 ml of ethanol was added thereto, followed by centrifugation. The precipitate obtained was washed with 80% EtOH and dried under reduced pressure to obtain RN01-N-SP06-SO06.

ASO06 (0.2 mM, 750 µl) was mixed with RN01-P-SP08-SO06 (0.20 mM, 750 µl), the solvent was removed therefrom, and HDO comprising a ligand bound thereto; i.e., RN01-P-SP08-HDO06 (0.1 mM, 1.5 ml), was obtained. Physiological saline (Otsuka Normal Saline (Otsuka Pharmaceutical Co., Ltd.)) was added to RN01-P-SP06-HDO06, the solution was heated at 95°C for 5 minutes, and the solution was slowly cooled to room temperature for annealing.

RN01-N-SP07-HDO06 was obtained with the use of RN01-N-PEG4-maleimide instead of RN01-N-PEG4-SPDP.

Tm of each HDO was measured. The measured values are shown in Table 12.

**[Table 12]**

| Name of oligonucleotides | Tm |
|---|---|
| RN01-N-SP06-HDO06 | 62°C |
| RN01-N-SP07-HDO06 | 62°C |

A 6.3-ml reactor was filled with 0.5 g (175 µmol) of Primer Support^{™} 5G Unylinker 350 and mounted on an automated nucleic acid synthesizer (AKTA Oligopilot 100). The elongation cycle for synthesis was determined under the conditions below, and an oligomer comprising RN01-P-SP08-SO06 supported thereon was synthesized.

In the deblocking step, a solution of 3% (v/v) dichloroacetic acid in toluene was used as a deblocking solution. RNA coupling to a 2' OMe nucleotide was performed by delivering a solution of RNA and 2' OMe amidite dissolved to 0.15 M and a 0.25 mol/l of 5-benzylthio-1H-tetrazole/acetonitrile solution at 2:3. RN01-P coupling was performed by delivering a solution of RN01-P-amidite dissolved to 0.1 M and 0.25 mol/l of a 5-benzylthio-1H-tetrazole/acetonitrile solution at 1:4. In the step of sulfidation, a solution of phenylacetyl disulfide in acetonitrile and 3-picoline (1:1) was used as a sulfidation solution. In the step of oxidation, a solution of 50 mM iodine in water: pyridine (9:1) was used as an oxidation solution. In the step of capping, Solution A: a mixture of 2-methylimidazole and acetonitrile (2:8); and Solution B: a mixture of acetic anhydride, 2,6-lutidine, and acetonitrile (2:3:5) were delivered at 1:1.

Resin comprising the synthesized RN01-P-SP08-SO06 supported thereon was collected from the reactor and air-dried for 15 minutes or longer. To 300 mg of the dried resin, 3 ml of 20% methylamine/isopropanol (1:1) was added, and the resultant was agitated at 40°C for 2 hours. Resin was separated by filtration and washed with 10 ml of DMSO. To the resulting filtrate, 5 ml of hydrogen trifluoride/3-3triethylamine was added, and the resultant was agitated at 60°C for 2 hours. Thereafter, 30 ml of ethanol was added to precipitate RN01-P-SP08-SO06, and the supernatant was removed. The precipitate was dissolved in an aqueous solution of 8 mM triethylamine, 100 mM hexafluoroisopropanol, and 5% methanol and then purified by HPLC (column: YMC-Triart C18, 150 × 10.0 mm ID S-5µm; eluate A: an aqueous solution of 8 mM triethylamine, 100 mM hexafluoroisopropanol, and 5% methanol; eluate B: an aqueous solution of 80% methanol; elution condition: 4.7 ml/min, 0-60% B). The solvent was removed by distillation, the resultant was dissolved again in 1 ml of water, an aqueous solution of sodium chloride (5.0 M, 30 µl) was added thereto, the mixture was agitated, and 4 ml of ethanol was added thereto, followed by centrifugation. The precipitate obtained was washed with 80% EtOH and dried under reduced pressure to obtain RN01-P-SP08-HDO06.

ASO06 (0.2 mM, 750 µl) was mixed with RN01-P-SP08-SO06 (0.20 mM, 750 µl), the solvent was removed therefrom, and HDO comprising a ligand bound thereto; i.e., RN01-P-SP08-HDO06 (0.1 mM, 1.5 ml), was obtained. Physiological saline (Otsuka Normal Saline (Otsuka Pharmaceutical Co., Ltd.)) was added to RN01-P-SP08-HDO06, the solution was heated at 95°C for 5 minutes, and the solution was slowly cooled to room temperature for annealing.

Tm of RN01-P-SP08-HDO06 was measured. The measured value is shown in Table 13.

**[Table 13]**

| Name of oligonucleotides | Tm |
|---|---|
| RN01-P-SP08-HDO06 | 64°C |

### [Example 3]

The double-stranded antisense nucleic acid complexes prepared in Example 2; i.e., RN01-N-SP01-HDO06 and RN01-P-SP01-HDO06, were examined as to the target gene knock down effects in cell culture. In the experiment, the mouse fibroblast cell line NIH/3T3 expressing the S1P receptor, which is the target receptor of RN01-N-SP01-HDO06 and RN01-P-SP01-HDO06, was used. When RN01-N-SP01-HDO06 and RN01-P-SP01-HDO06 were integrated into a cell with the aid of a receptor on the cell membrane, lowering in the expression levels of Malat1 ncRNA, which is the target transcription product of RN01-N-SP01-HDO06 and RN01-P-SP01-HDO06, was observed.

### Cell culture

The mouse fibroblast cell line NIH/3T3 was purchased from the National Institutes of Biomedical Innovation, Health and Nutrition. The NIH/3T cell was maintained at 37°C in 5% CO₂ in the Dulbecco's Modified Eagle Medium (Thermo Fisher Scientific) supplemented with 10% fetal bovine serum (Thermo Fisher Scientific), 100 U/ml penicillin, and 100 µg/ml streptomycin.

### Test of gene knockdown effects in vitro

Cells of the mouse fibroblast cell line NIH/3T3 were sowed at 1 × 10⁵ cells/0.5 ml/well on a commercially available 24-well plate and cultured in a CO₂ incubator for 24 hours. On the following day, 10 nmol/l, 30 nmol/l, or 100 nmol/l of RN01-N-SP01-HDO06 or RN01-P-SP01-HDO06 (hereafter referred to as "HDO") was added to the medium and integrated into the cells. As a negative control, a PBS-supplemented group was provided. Cells were collected 24 hours after the addition of HDO. Total RNA was purified with the use of the SV96 Total RNA Isolation System (Promega), and cDNA was synthesized from total RNA using PrimeScript^{™} RT Master Mix (TaKaRa, RR036A). In order to compare and analyze the expression levels of endogenous gene Malat1 ncRNA, qPCR was performed using StepOnePlus (Thermo Fisher Scientific), reagents designed by TaqMan Gene Expression Assays (Thermo Fisher Scientific) were used as primers/probes for mouse Malat1 and mouse 18SrRNA, Ct of mouse Malat1 and that of mouse 18S rRNA were determined, and the mRNA expression levels were then determined by relative quantification based on the ΔΔCt method.

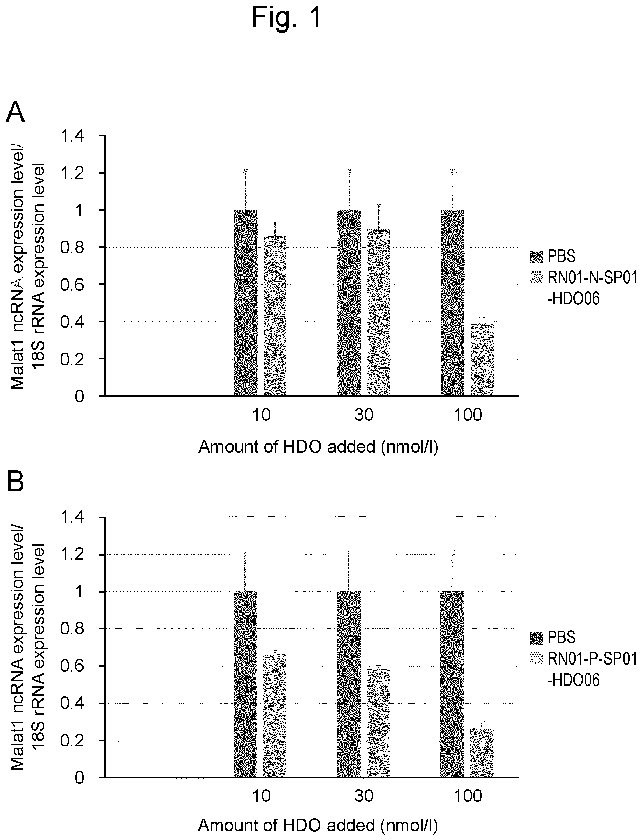

In the manner described above, each HDO was examined in terms of the knockdown effects of the endogenous gene Malat1. The results are shown in Figure 1A and in Figure 1B. In Figure 1, the horizontal axis indicates PBS at 10 nmol/l, 30 nmol/l, and 100 nmol/l and RN01-N-SP01-HDO01 (Figure 1A) or RN01-N-SP01-HDO02 (Figure 1B). The vertical axis indicates the relative expression level (Malat1 ncRNA expression level/18SrRNA expression level).

As shown in Figure 1A, Malat1 expression was knocked down by 14%, 10.2%, or 61.1% with the addition of RN01-N-SP01-HDO06 at 10 nmol/l, 30 nmol/l, or 100 nmol/l. As shown in Figure 1B, Malat1 ncRNA expression was knocked down by 33.4%, 41.8%, or 72.9% with the addition of RN01-P-SP01-HDO06 at 10 nmol/l, 30 nmol/l, or 100 nmol/l.

### [Example 4]

### Reagent

The nucleic acid complexes used in Example 4 are RN01-N-SP01-HDO06 and RN01-P-SP01-HDO06, each of which is a conjugate of 16-mer HDO targeting mouse Malat1 ncRNA prepared in Example 2 and the S1P ligand. The present inventors adjusted the oligo complexes to 100 µM with physiological saline (Otsuka Pharmaceutical Co., Ltd.), and double-stranded HDO was denatured in a block bath (CDB-105, AS ONE Corporation) at 90°C for 5 minutes and naturally cooled to room temperature for about 2 hours for annealing.

### Animal

c57BL/6J mice (25 mice, Charles River Laboratories Japan, Inc., female, 4-week-old when arrived) were used. Five mice were raised in a plastic cage at room temperature (24 ± 2°C) at humidity (55 ± 5%) under light conditions for 12 hours (8:00 to 20:00), and mice were conditioned for 1 week while given free access to drinking water and a solid feed (MF, Oriental Yeast Co., Ltd.).

### Method

Mice were divided into groups depending on body weights: the vehicle (saline, Otsuka Pharmaceutical Co., Ltd.) administration group; the RN01-P-SP01-HDO06 (1 µmol/kg) administration group; and the RN01-N-SP01-HDO06 (VN-H, 1 µmol/kg) administration group (each group consisting of 5 mice (n = 5)). On the day of reagent administration, mice were retained in a retainer in the awakened state, and a reagent was slowly administered through the caudal vein (10 ml/kg, Day 0). After administration, hemostasis at the site of administration was confirmed and the mice were returned to the cages. The mice were subjected to autopsy 3 days after administration (Day 3). On the day of autopsy, body weight of each mouse was measured, a blood sample was taken from the heart under anesthesia with isoflurane (introduced at 3% to 5%, maintained at 1% to 3%) (Terumo syringe SS-01P2525, containing the anticoagulant heparin), and mice were euthanized by exsanguination. The liver, the kidney, and the spleen were extracted by laparotomy. Subsequently, the lung, the heart, and the thymic gland were extracted by thoracotomy. The extracted organs were immediately soaked in ISOGEN and subjected to mRNA extraction. Parts of the liver, the kidney, and the lung were cryopreserved in dry ice, and subjected to ELOSA measurement of ASO. A blood sample was introduced into a 1.5-ml Eppendorf tube and centrifuged at 10,000 rpm for 5 minutes, the supernatant was cryopreserved at -30°C, and the resultant was used for measurement of the levels of liver deviation enzymes; i.e., alanine aminotransferase (ALT) and aspartate aminotransferase (AST), in blood.

Tissue was fractured using a biomasher, GentleMACS Dissociators (Miltenyi Biotec) or FastPrep-24 5G (MP Biomedicals). Total RNA was extracted from the solution containing fractured cells using the ReliaPrep^{™} RNA Tissue Miniprep System (Z6112, Promega Corporation). cDNA was prepared from total RNA by reverse transcription using the PrimeScript^{™} RT Master Mix (RR036A, TaKaRa). mRNA expression of each gene was measured using the real-time PCR system (StepOnePlus, Applied Biosystems). The TaqMan probe set for mouse Malat1 (Mm 01227912-s1, Thermo Fisher Scientific) and 18S rRNA (Mm 03928990-g1, Thermo Fisher Scientific) mRNA was used.

ALT/AST activity in blood was measured using the Test Wako ALT/AST assay kit (431-3090, FUJIFILM Wako Pure Chemical Corporation) and an enzyme calibrator (416-57191, FUJIFILM Wako Pure Chemical Corporation) (*in vivo* protocol/Test Wako ALT/AST assay).

All the measured values were indicated in terms of "mean ± standard deviation." For statistical analysis, the one-way analysis of variance (ANOVA) was performed using GraphPad Prism 7.04. For comparison between groups, the Dunnett's multiple comparison was employed. A risk of lower than 5% is designated significant.

### Result

### 1. General conditions

No apparent changes were observed in coats, behaviors, and stools of mice between before and after administration. The reagent was administered through the caudal vein at 10 ml/kg, and no abnormality was observed in behavior immediately after administration.

### 2. Body weight

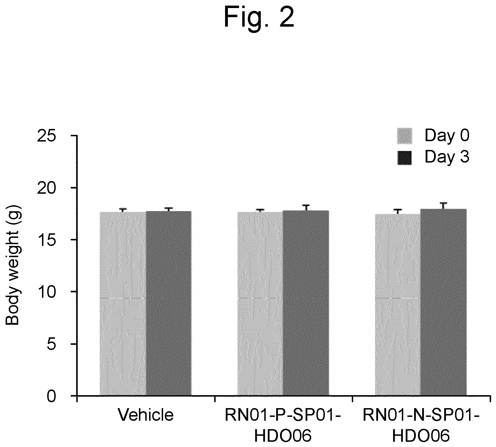

Before administration, the average body weight was 17 g in each animal group, and no significant change was observed between before and after administration. In comparison with the vehicle group, no significant change was observed in the average body weight of the oligo administration group (Figure 2).

### 3. ALT/AST activity in blood

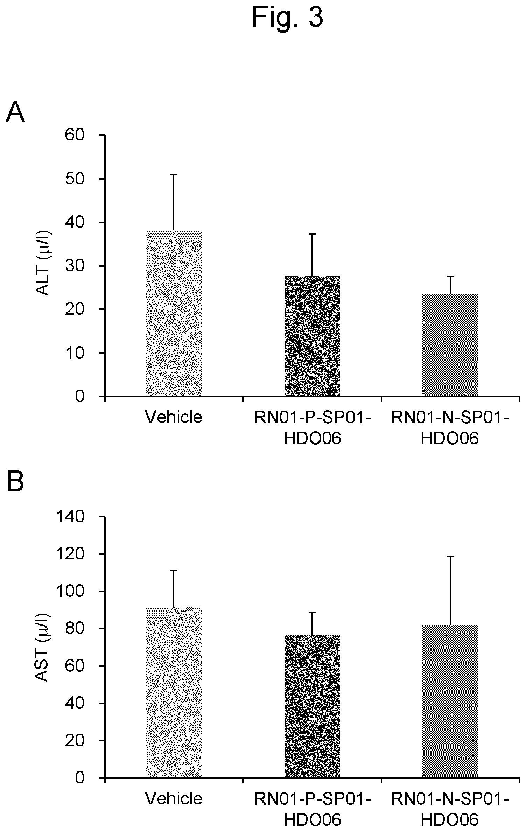

Figure 3A shows changes in ALT activity in blood 3 days after administration of Malat1 oligo to mice.

Figure 3B shows changes in AST activity in blood 3 days after administration of Malat1 oligo to mice.

No significant changes were observed in ALT activity and AST activity of the Malat1oligo administration group, compared with those of the vehicle administration group.

### 4. Changes in Malat1 ncRNA expression in tissue

### 4.1 Liver

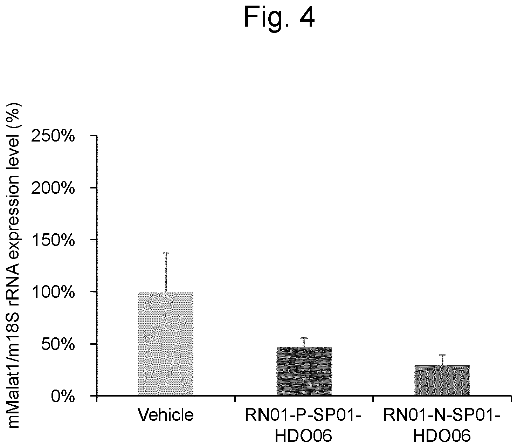

The Malat1 ncRNA expression level in the liver was lowered in the RN01-N-SP01-HDO06 administration group and in the RN01-P-SP01-HDO06 administration group, compared with that in the vehicle administration group, although no significant difference was observed. However, Malat1 ncRNA expression was knocked down to a significant extent, compared with the carrier strand administration groups (Figure 4).

### 4.2 Lung

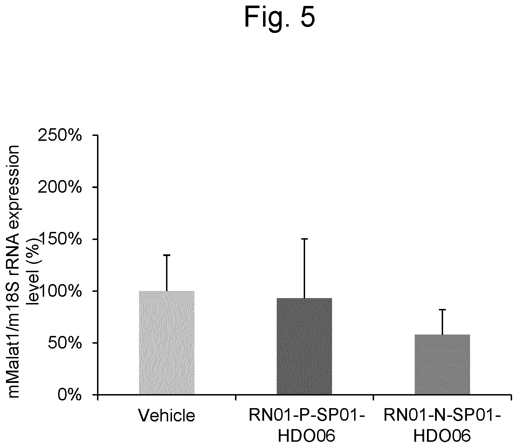

The Malat1 ncRNA expression level in the lung was lowered in the RN01-N-SP01-HDO06 administration group and in the RN01-P-SP01-HDO06 administration group, compared with that in the vehicle administration group (Figure 5).

### 4.3 Kidney

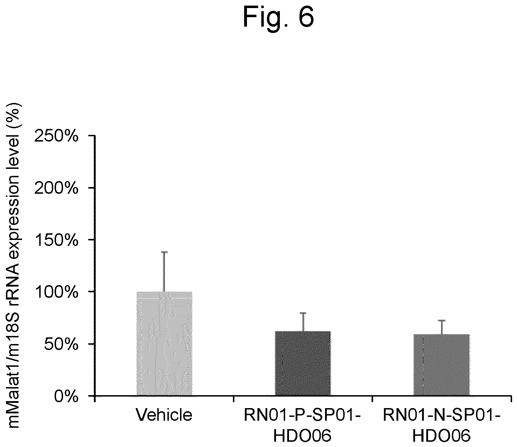

The Malat1 ncRNA expression level in the kidney was lowered in the RN01-N-SP01-HDO06 administration group and in the RN01-P-SP01-HDO06 administration group, compared with that in the vehicle administration group (Figure 6).

### [Example 5]

### Reagent

The nucleic acid complexes used in Example 5 are RN02-P-SP01-HDO06 and RN02-N-SP01-HDO06 targeting mouse Malat1 ncRNA prepared in Example 2. The present inventors adjusted the oligo complexes to 100 µM with physiological saline (Otsuka Pharmaceutical Co., Ltd.), and double-stranded HDO was denatured in a block bath (CDB-105, AS ONE Corporation) at 90°C for 5 minutes and naturally cooled to room temperature for about 2 hours for annealing.

### Animal

c57BL/6J mice (25 mice, Charles River Laboratories Japan, Inc., female, 4-week-old when arrived) were used. Five mice were raised in a plastic cage at room temperature (24 ± 2°C) at humidity (55 ± 5%) under light conditions for 12 hours (8:00 to 20:00), and mice were conditioned for 1 week while given free access to drinking water and a solid feed (MF, Oriental Yeast Co., Ltd.).

### Method

Mice were divided into groups depending on body weights: the vehicle (saline, Otsuka Pharmaceutical Co., Ltd.) administration group; the RN02-P-SP01-HDO06 (1 µmol/kg) administration group; and the RN02-N-SP01-HDO06 (1 µmol/kg) administration group (each group consisting of 5 mice (n = 5)). On the day of reagent administration, mice were retained in a retainer in the awakened state, and a reagent was slowly administered through the caudal vein (10 ml/kg, Day 0). The L001 complementary strand (1 µmol/kg) administration group was used as a negative control. After administration, hemostasis at the site of administration was confirmed and the mice were returned to the cages. The mice were subjected to autopsy 3 days after administration (Day 3). On the day of autopsy, body weight of each mouse was measured, a blood sample was taken from the heart under anesthesia with isoflurane (introduced at 3% to 5%, maintained at 1% to 3%) (Terumo syringe SS-01P2525, containing the anticoagulant heparin), and mice were euthanized by exsanguination. The liver and the kidney were extracted by laparotomy. The extracted organs were immediately soaked in ISOGEN and subjected to mRNA extraction. A blood sample was introduced into a 1.5-ml Eppendorf tube and centrifuged at 10,000 rpm for 5 minutes, the supernatant was cryopreserved at -30°C, and the resultant was used for measurement of the levels of liver deviation enzymes; i.e., alanine aminotransferase (ALT) and aspartate aminotransferase (AST), in blood.

Tissue was fractured using a biomasher, GentleMACS Dissociators (Miltenyi Biotec) or FastPrep-24 5G (MP Biomedicals). Total RNA was extracted from the solution containing fractured cells using the ReliaPrep^{™} RNA Tissue Miniprep System (Z6112, Promega Corporation). cDNA was prepared from total RNA by reverse transcription using the PrimeScript^{™} RT Master Mix (RR036A, TaKaRa). mRNA expression of each gene was measured using the real-time PCR system (StepOnePlus, Applied Biosystems). The TaqMan probe set for mouse Malat1 (Mm 01227912-s1, Thermo Fisher Scientific) and 18S rRNA (Mm 03928990-g1, Thermo Fisher Scientific) mRNA was used.

ALT/AST activity in blood was measured using the Test Wako ALT/AST assay kit (431-3090, FUJIFILM Wako Pure Chemical Corporation) and an enzyme calibrator (416-57191, FUJIFILM Wako Pure Chemical Corporation).

All the measured values were indicated in terms of "mean ± standard deviation." For statistical analysis, the one-way analysis of variance (ANOVA) was performed using GraphPad Prism 7.04. For comparison between groups, the Dunnett's multiple comparison was employed. A risk of lower than 5% is designated significant.

### Result

### 1. General conditions

No apparent changes were observed in coats, behaviors, and stools of mice between before and after administration. The reagent was administered through the caudal vein at 10 ml/kg, and no abnormality was observed in behavior immediately after administration.

### 2. Body weight

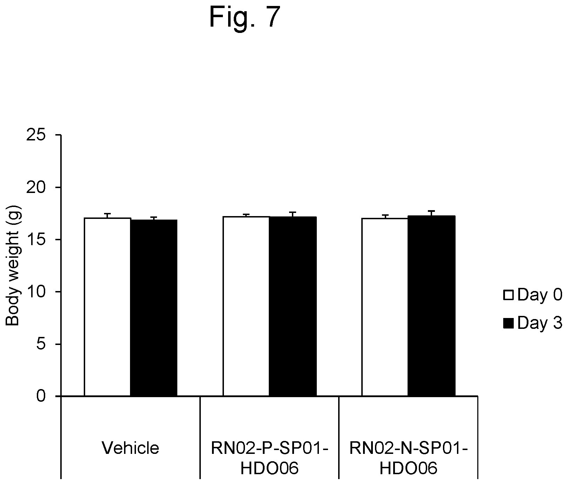

Before administration, the average body weight was 17 g in each animal group, and no significant change was observed between before and after administration. In comparison with the vehicle group, no significant change was observed in the average body weight of the oligo administration group (Figure 7).

### 3. ALT/AST activity in blood

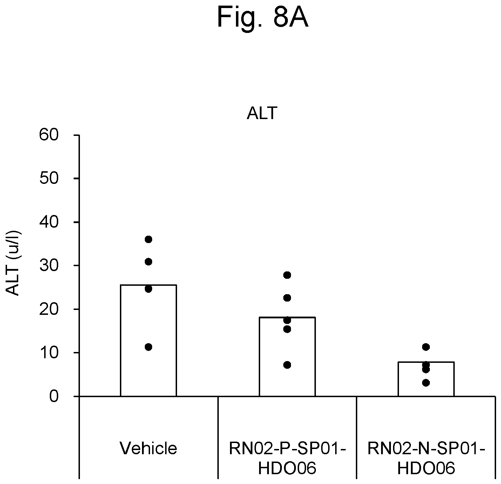

Figure 8A shows changes in ALT activity in blood 3 days after administration of Malat1 oligo to mice.

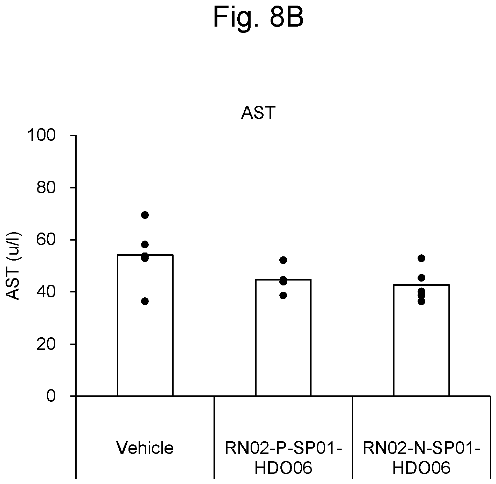

Figure 8B shows changes in AST activity in blood 3 days after administration of Malat1 oligo to mice.

No significant increase was observed in ALT activity and AST activity of the Malat1oligo administration group, compared with those of the vehicle administration group (Figure 8A, Figure B).

### 4. Changes in Malat1 ncRNA expression in tissue

### 4.1 Liver

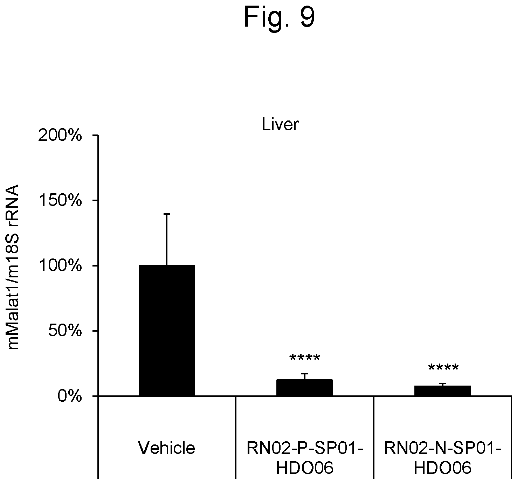

The Malat1 ncRNA expression level in the liver was lowered in the RN02-P-SP01-HDO06 administration group and in the RN02-N-SP01-HDO06 administration group to a significant extent, compared with that in the vehicle administration group (Figure 9).

Figure 9 shows comparison with the vehicle administration group and **** indicates p < 0.0001.

### 4.2 Kidney

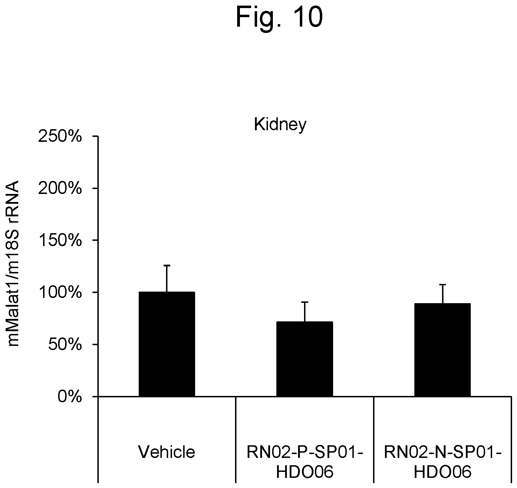

The Malat1 ncRNA expression level in the kidney was likely to be lower in the RN02-P-SP01-HDO06 administration group, compared with that in the vehicle administration group (Figure 10).

### 4.3 Lung

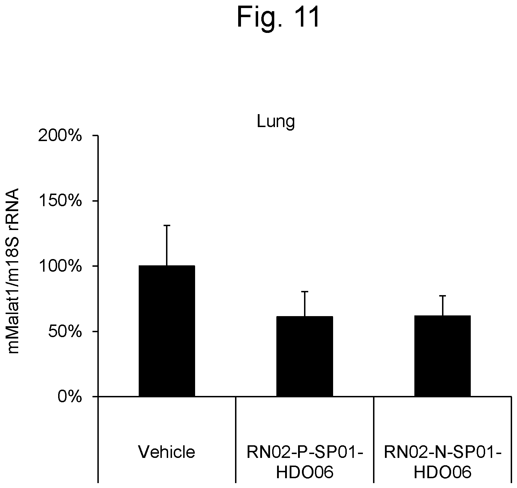

The Malat1 ncRNA expression level in lung tissue was likely to be lower in the RN02-P-SP01-HDO06 administration group and in the RN02-N-SP01-HDO06 administration group, compared with that in the vehicle administration group (Figure 11).

### [Example 6]

### Reagent

In some embodiments, nucleic acid complexes shown in Table 14 can be used. Table 14 shows oligo names of nucleic acid complexes (Name of Oligonucleotides) and structural formulae of compounds binding to nucleic acid molecules.

In Example 6, various nucleic acid complexes, each of which is a conjugate of 16-mer HDO06 or ASO06 targeting mouse Malat1 ncRNA prepared in the example above and the S1P ligand, were subjected to the following experiment. The present inventors adjusted the oligo complexes to 30 or 100 □mol/ml with physiological saline (Otsuka Pharmaceutical Co., Ltd.), and double-stranded HDO was denatured in a block bath (CDB-105, AS ONE Corporation) at 95°C for 10 minutes and naturally cooled to room temperature for about 2 hours for annealing.

**[Table 14]**

| Reagent | Compound No. | Structural formula of compound binding to nucleic acid molecule |
|---|---|---|
| DBCO | DBCO-HDO06 | 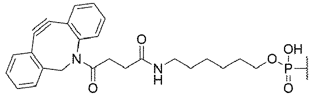 |
| VN-DBCO | RN01-N-SP01-HDO06 | 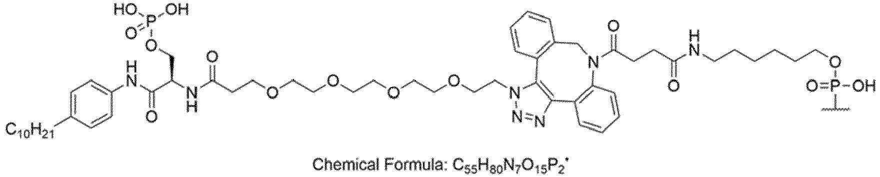 |
| VN-BCN | RN01-N-SP05-HDO06 | 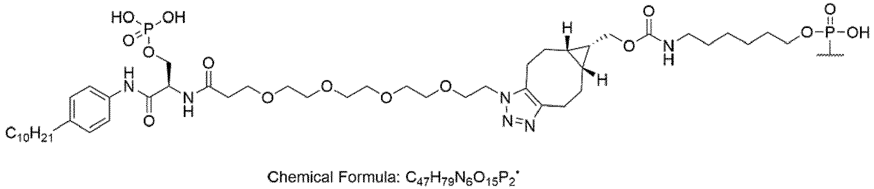 |
| VN-Mal-S | RN01-N-SP07-HDO06 | 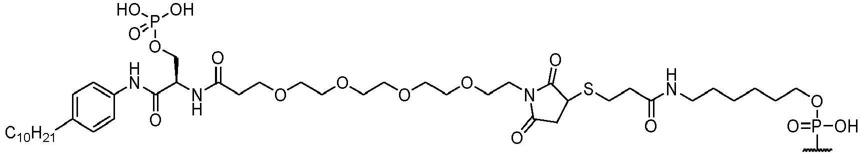 |
| VN-S-S | RN01-N-SP06-HDO06 | 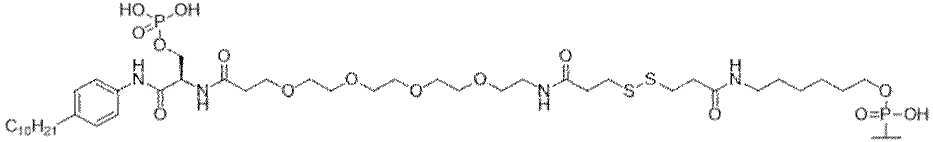 |
| VP-direct | RN01-P-SP08-HDO06 | 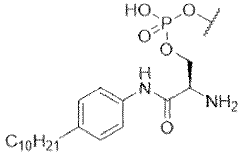 |
| VN-PEG4 | RN01-N-SP01-HDO06 | 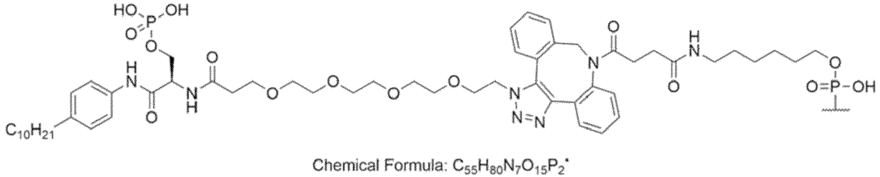 |
| VN-PEG1 | RN01-N-SP02-HDO06 | 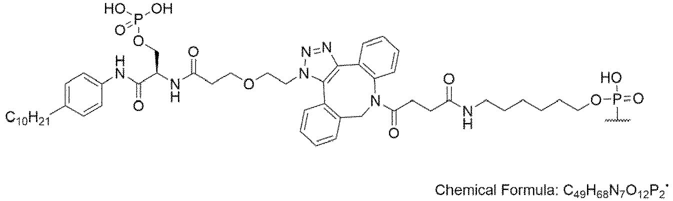 |
| VN-PEG9 | RN01-N-SP03-HDO06 | 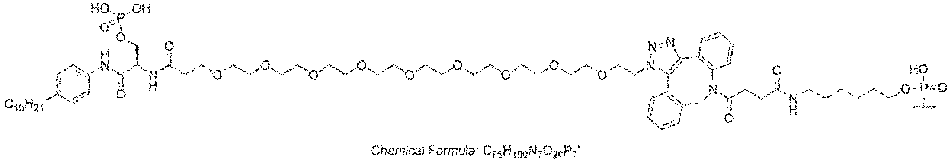 |
| VN-AlkylC10 | RN01-N-SP04-HDO06 | 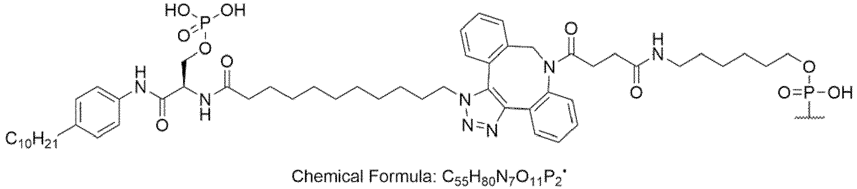 |
| DBCO-ASO | DBCO-ASO06 | 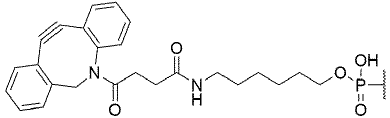 |
| FTY-ASO | RN01-N-SP01-ASO06 | 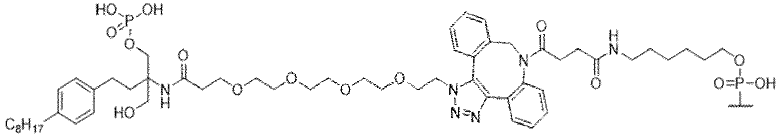 |
| VPC-ASO | RN02-N-SP01-ASO06 | 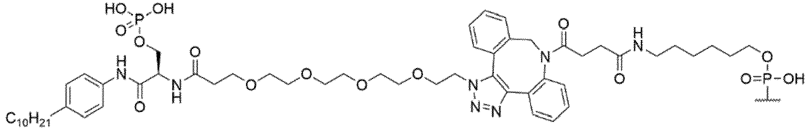 |

### Animal

c57BL/6J mice (Japan SLC, Inc., female, 4-week-old when arrived) were used. Five mice were raised in a plastic cage at room temperature (24 ± 2°C) at humidity (55 ± 5%) under light conditions for 12 hours (7:00 to 19:00), and mice were conditioned for 1 week while given free access to drinking water and a solid feed (MF, Oriental Yeast Co., Ltd.).

### Method

Mice were divided into groups depending on body weights: the vehicle (saline, Otsuka Pharmaceutical Co., Ltd.) administration group; and HDO administration groups (various types of HDOs shown in Table 14, hereafter referred to as "reagent") (1 µmol/kg) (each group consisting of 5 mice (n = 5)). On the day of reagent administration, mice were retained in a retainer in the awakened state, and a reagent was slowly administered through the caudal vein (10 ml/kg, Day 0). After administration, hemostasis at the site of administration was confirmed and the mice were returned to the cages. The mice were subjected to autopsy 3 days after administration (Day 3). On the day of autopsy, mice were euthanized by exsanguination under anesthesia with isoflurane (introduced at 3% to 5%, maintained at 1% to 3%). The abdomen was opened, and the liver, the kidney, the lung, the heart, and the thigh muscle were extracted. The extracted organs were immediately soaked in ISOGEN and subjected to mRNA extraction.

Tissue was fractured using FastPrep-24 5G (MP Biomedicals) to extract mRNA. Total RNA was extracted from the solution containing fractured cells using the ReliaPrep^{™} RNA Tissue Miniprep System (Z6112, Promega Corporation). cDNA was prepared from total RNA by reverse transcription using the PrimeScript^{™} RT Master Mix (RR036A, TaKaRa). mRNA expression of each gene was measured using the real-time PCR system (StepOnePlus, Applied Biosystems). The TaqMan probe set for mouse Malat1 (Mm 01227912-s1, Thermo Fisher Scientific) and 18S rRNA (Mm 03928990-g1, Thermo Fisher Scientific) mRNA was used.

All the measured values were indicated in terms of "mean ± standard deviation." For statistical analysis, the one-way analysis of variance (ANOVA) was performed using GraphPad Prism 7.04. For comparison between groups, the Dunnett's multiple comparison was employed. A risk of lower than 5% is designated significant.

### Result

### 1. Examination of influence of conjugation unit on expression of target transcription factor

1.1. Influence on Malat1 ncRNA expression in the skeletal muscle was compared among the vehicle administration, the DBCO-HDO06 administration group, and the RN01-N-SP01-HDO06 administration group (DBCO-HDO06 and RN01-N-SP01-HDO06 are the reagents shown in Table 14).

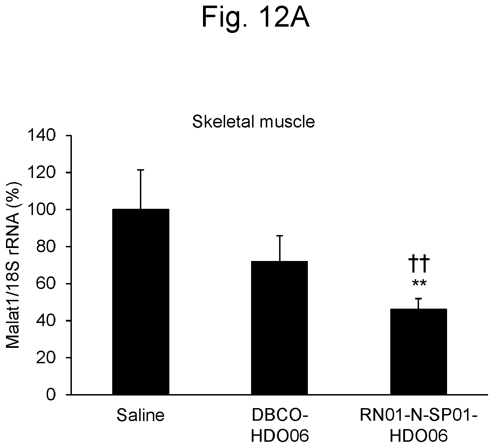

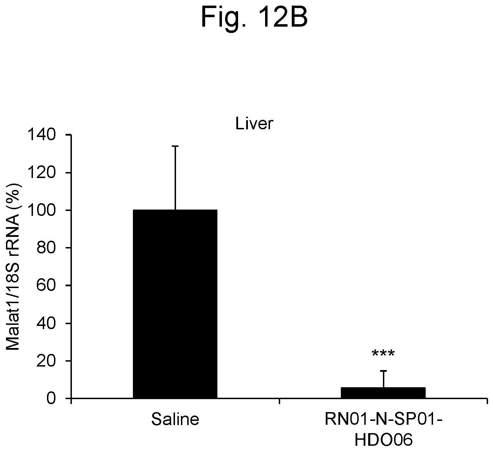

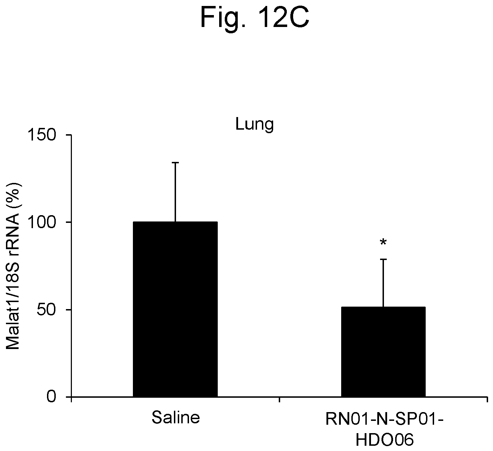

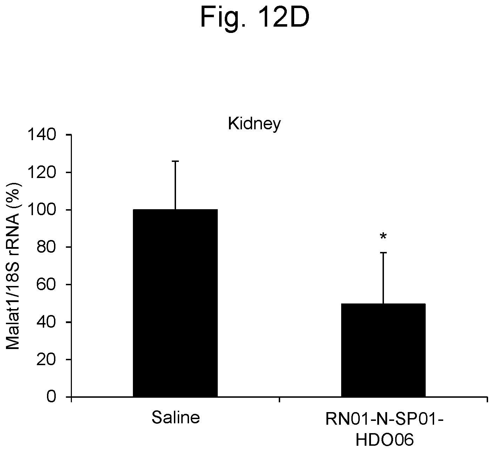

The Malat1 ncRNA expression in the skeletal muscle was not suppressed to a statistically significant extent in the HDO (DBCO-HDO06) administration group to which the S1P ligand (VPC22199) had not been applied. In the HDO (RN01-N-SP01-HDO06) administration group to which VPC22199 had been applied, in contrast, the Malat1 ncRNA expression was lowered to a significant extent, compared with that in the vehicle administration group. When compared in terms of the presence or absence of a ligand, the Malat1 ncRNA level was significantly lower in the HDO (RN01-N-SP01-HDO06) administration group to which VPC22199 had been applied than that in the HDO (DBCO-HDO06) administration group to which VPC22199 had not been applied (Figure 12A). In the HDO administration group to which VPC22199 had been applied, in addition, the Malat1 ncRNA expression in the liver, the lung, and the kidney was suppressed to a statistically significant extent, in addition to that in the skeletal muscle (Figure 12B, Figure 12C, and Figure 12D).

### 1.2. RN01-N-SP05-HDO06

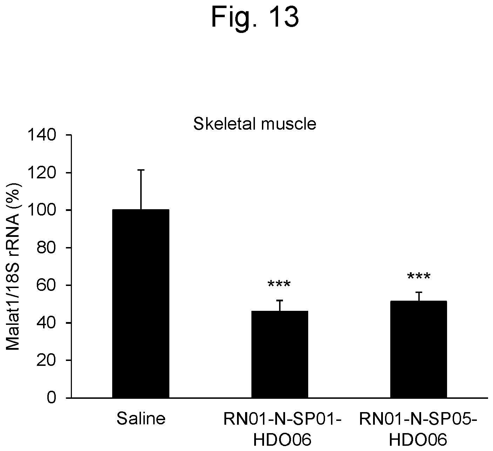

The Malat1 ncRNA expression level in the skeletal muscle was lowered to a significant extent in the HDO (RN01-N-SP05-HDO06) administration group to which VPC22199 had been applied, compared with that in the vehicle administration group (Figure 13).

### 1.3. RN01-N-SP07-HDO06

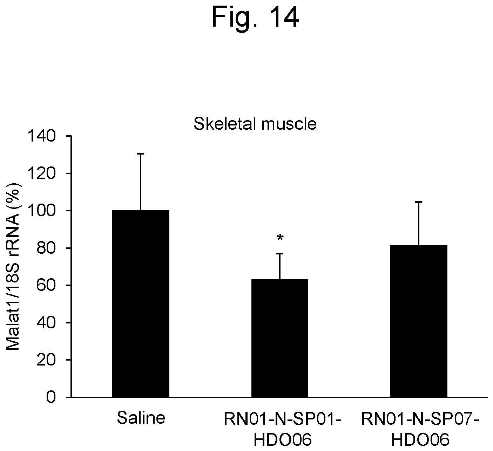

The Malat1 ncRNA expression level in the skeletal muscle was likely to be lower in the HDO (RN01-N-SP07-HDO06) administration group to which VPC22199 had been applied, compared with that in the vehicle administration group (Figure 14).

### 1.4. RN01-N-SP06-HDO06

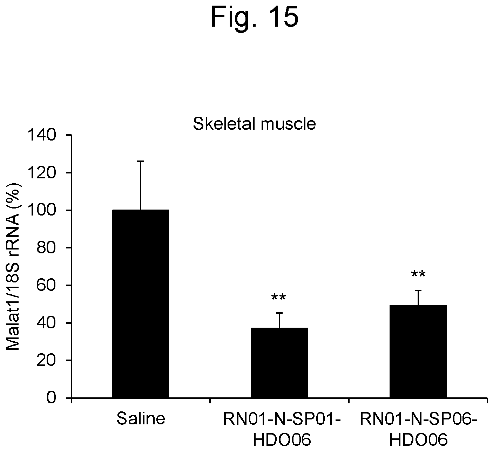

The Malat1 ncRNA expression level in the skeletal muscle was lower to a significant extent in the HDO (RN01-N-SP06-HDO06) administration group to which VPC22199 had been applied, compared with that in the vehicle administration group (Figure 15).

### 1.5. RN01-P-SP08-HDO06

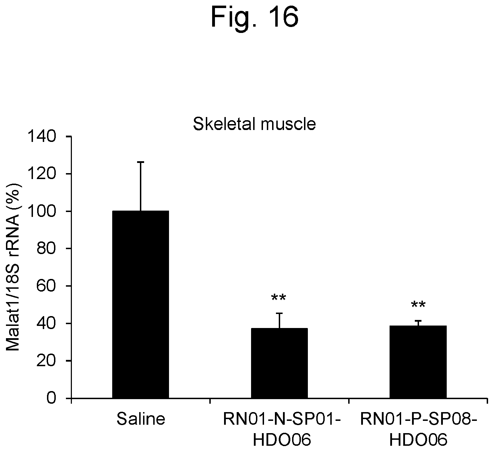

The Malat1 ncRNA expression level in the skeletal muscle was likely to be lower in the HDO (RN01-P-SP08-HDO06) administration group to which VPC22199 had been applied, compared with that in the vehicle administration group (Figure 16).

### 2. Spacer

### 2.1. RN01-N-SP01-HDO06, RN01-N-SP02-HDO06, RN01-N-SP03-HDO06

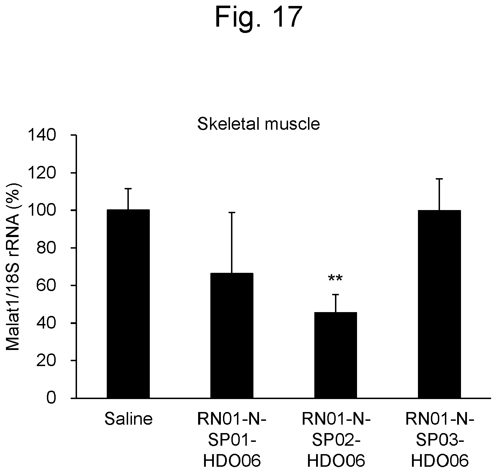

The Malat1 ncRNA expression level in the skeletal muscle was lowered to a statistically significant extent in the HDO (RN01-N-SP02-HDO06) administration group to which VPC22199 had been applied, compared with that in the vehicle administration group. While the Malat1 ncRNA expression level was likely to be lower by administration of RN01-N-SP01-HDO06, such expression level was not lowered by administration of RN01-N-SP03-HDO06 (Figure 17).

### 2.2. Alkyl C10

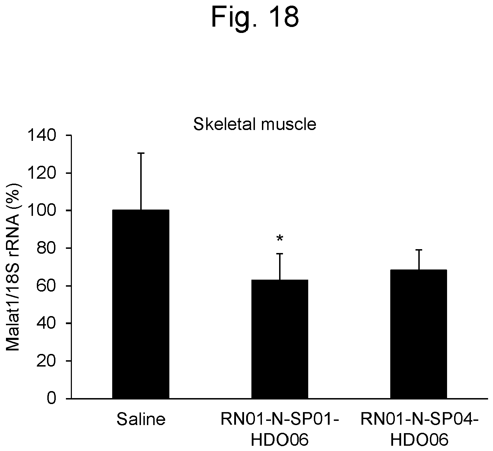

The Malat1 ncRNA expression level in the skeletal muscle was likely to be lower in the HDO (RN01-N-SP04-HDO06) administration group to which VPC22199 had been applied, compared with that in the vehicle administration group (Figure 18).

### 3. Antisense oligonucleotides

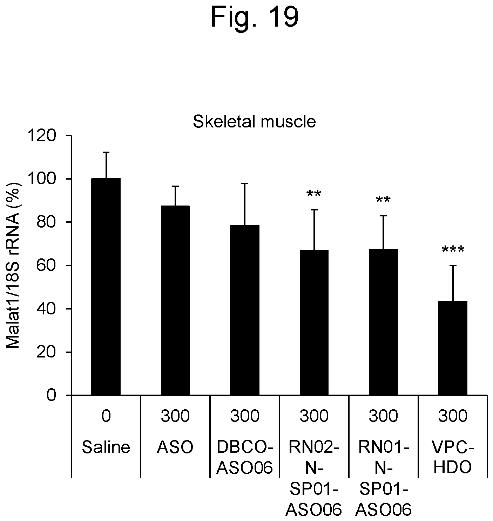

The Malat1 ncRNA expression in the skeletal muscle was not suppressed to a statistically significant extent by administration of ASO or DBCO-ASO06, compared with that in the vehicle administration group. However, the Malat1 ncRNA expression level was lowered to a statistically significant extent by administration of ASO or HDO to which the S1P ligand; i.e., VPC or FTY, was conjugated (Figure 19).

### Industrial Applicability

The ligand-binding nucleic acid complex of the present invention can be used for nucleic acid medicine that is delivered to an organ to regulate expression or editing of a target gene or a transcription product thereof.

All publications, patents, and patent applications cited herein are incorporated herein by reference in their entirety.

## Claims

1. A ligand-binding nucleic acid complex in which a nucleic acid molecule that regulates expression or editing of a target gene or a transcription or translation product thereof binds to a compound represented by a formula below or a pharmaceutically acceptable salt with or without a linker: 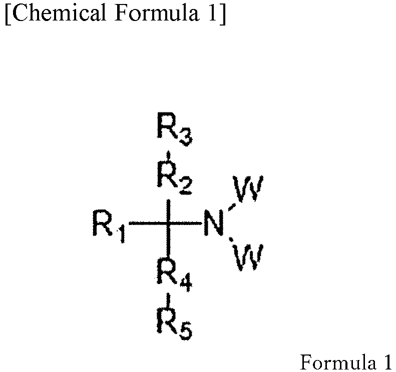 wherein
R₁ represents a C₆-C₅₀ linear or branched carbon chain, and the carbon chain may comprise at least one partial structure, bond, or hetero atom selected from among a double bond, a triple bond, carbocyclic Z, O, S, and N, and/or carbonyl and at least one Y as a substituent;
R₂ and R₄ each represent (CH₂)ₘ;
m is an integer of 0 to 4;
R₃ and R₅ are each selected from the group consisting of -H, -X-, -XW, -P(=X)(-XW)₂, and -XP(=X) (-XW)₂;
X is selected from the group consisting of O, S, and NH;
Y is selected from the group consisting of alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, alkyl halide, hydroxy, carboxy, sulfonic acid, sulfonic acid derivative, azide, and thiol;
Z represents a C₁-C₃₀ linear or branched carbon chain, the carbon chain is selected from the group consisting of cycloalkylalkyl, arylalkyl, heterocycloalkylalkyl, and heteroarylalkyl, and the carbon chain may comprise a bond or hetero atom selected from among a double bond, a triple bond, O, S, N, sulfinyl, sulfonyl, and/or carbonyl and at least one Y as a substituent; and
W independently represents H or a C₁-C₆₀ linear or branched carbon chain, and the carbon chain may comprise at least one partial structure, bond, or hetero atom selected from among a double bond, a triple bond, carbocyclic Z, O, S, and N, and/or carbonyl and at least one Y as a substituent.

2. The compound and the pharmaceutically acceptable salt according to claim 1, wherein, in Formula 1,
R₁ represents a C₆-C₅₀ linear or branched carbon chain, and the carbon chain may comprise at least one partial structure, bond, or hetero atom selected from among a double bond, a triple bond, carbocyclic Z, O, and N, and/or carbonyl and at least one Y as a substituent;
R₂ and R₄ each represent (CH₂)ₘ;
m is an integer of 0 to 4;
R₃ and R₅ are each selected from the group consisting of -H, -X-, -XW, and -XP(=X) (-XW)₂;
X is selected from the group consisting of O and S;
Y is selected from the group consisting of alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, alkyl halide, hydroxy, carboxy, sulfonic acid, azide, and thiol;
Z represents a C₁-C₃₀ linear or branched carbon chain, the carbon chain is selected from the group consisting of arylalkyl and heteroarylalkyl, and the carbon chain may comprise a bond or hetero atom selected from among a double bond, a triple bond, O, S, N, sulfinyl, sulfonyl, and/or carbonyl and at least one Y as a substituent; and
W independently represents H or a C₁-C₆₀ linear or branched carbon chain, and the carbon chain may comprise at least one partial structure, bond, or hetero atom selected from among a double bond, a triple bond, carbocyclic Z, O, S, and N, and/or carbonyl and at least one Y as a substituent.

3. The compound and the pharmaceutically acceptable salt according to claim 1, wherein, in Formula 1,
R₁ represents a C₆-C₅₀ linear or branched carbon chain, and the carbon chain may comprise at least one partial structure, bond, or hetero atom selected from among a double bond, a triple bond, carbocyclic Z, O, and S, and/or carbonyl and at least one Y as a substituent;
R₂ and R₄ each represent (CH₂)ₘ;
m is an integer of 0 to 4;
R₃ and R₅ are each selected from the group consisting of -H, -X-, -XW, and -XP(=X) (-XW)₂;
X is selected from the group consisting of O and S;
Y is selected from the group consisting of alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, alkyl halide, hydroxy, carboxy, and azide;
Z represents a C₁-C₃₀ arylalkyl, and the arylalkyl chain may comprise a bond or hetero atom selected from among a double bond, a triple bond, O, S, N, sulfinyl, and carbonyl and at least one Y as a substituent; and
W independently represents H or a C₁-C₅ linear or branched group selected from the group consisting of alkyl, acyl, and alkoxy.

4. The compound and the pharmaceutically acceptable salt according to claim 1, which are (R)-1-azide-17-((4-decylphenyl)carbamoyl)-15-oxo-3,6,9,12-tetraoxa-16-azaoctadeca-18-nyl phosphate or (R)-2-amino-3-((4-decylphenyl)amino)-3-oxopropyl(14-azide-3,6,9,12-tetraoxatetradecyl)phosphate.

5. The compound and the pharmaceutically acceptable salt according to claim 1, which are 1-azide-17-(hydroxymethyl)-17-(4-octylphenethyl)-15-oxo-3,6,9,12-tetraoxa-16-azaoctadeca-18-nyl phosphate or 2-amino-2-(hydroxymethyl)-4-(4-octylphenyl)butyl(14-azide-3,6,9,12-tetraoxatetradecyl)phosphate.

6. The compound and the pharmaceutically acceptable salt according to claim 1, wherein the compound represented by Formula 1 or a conjugate of the compound represented by Formula 1 and a linker is a compound selected from among compounds having structures shown below.
| Compound No. | Structural formula |
|---|---|
| RN01-N-PEG4-azide | 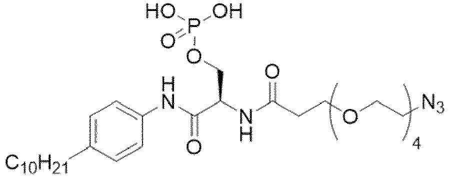 |
| RN01-P-PEG4-azide | 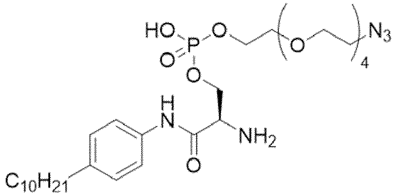 |
| RN02-N-PEG4-azide | 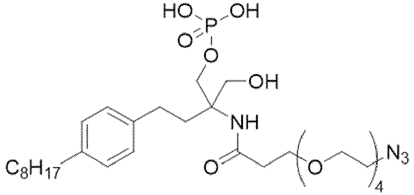 |
| RN02-P-PEG4-azide | 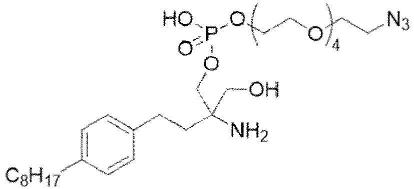 |
| RN01-N-PEG1-azide | 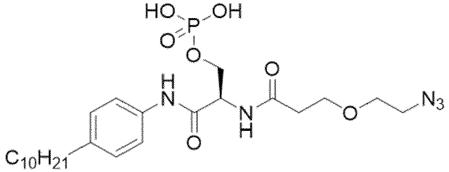 |
| RN01-N-PEG9-azide | 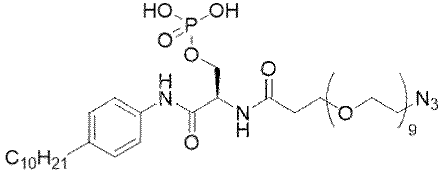 |
| RN01-N-C10-azide | 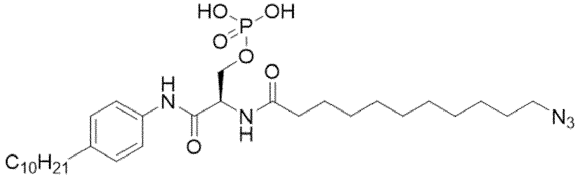 |
| RN01-N-PEG4-maleimide | 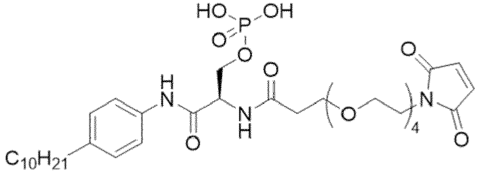 |
| RN01-P-amidite | 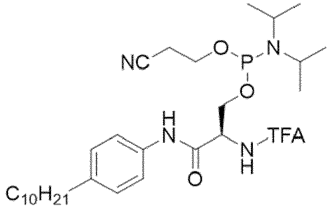 |
| RN01-N-PEG4SPDP | 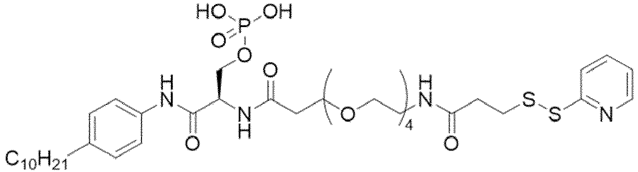 |

7. The ligand-binding nucleic acid complex according to any of claims 1 to 6, wherein the nucleic acid binds to the compound represented by Formula 1 or a pharmaceutically acceptable salt via a linker.

8. The ligand-binding nucleic acid complex according to claim 7, wherein the linker is a cleavable linker having a cleavable structure.

9. The ligand-binding nucleic acid complex according to claim 8, wherein the linker has the structure shown below.
| Linker type | Structural formula |
|---|---|
| Having a disulfide bond | 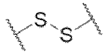 |
| Having an amide bond (n=0-9) | 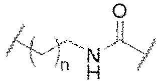 |
| Having a polyethylene glycol group (n=1-30) | 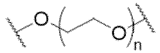 |
| Having a phosphate group | 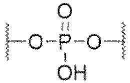 |
| Having a pyrrolidinyl group | 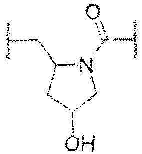 |
| Having a BCN group | 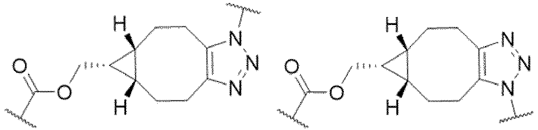 |
| Having a DBCO group | 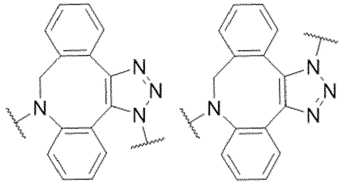 |
| Having a maleimide thioether group | 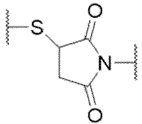 |

10. The ligand-binding nucleic acid complex according to claim 1, wherein the nucleic acid molecule is selected from among a nucleic acid molecule comprising an antisense strand consisting of an oligonucleotide having a nucleic acid sequence complementary to a target gene or a transcription product thereof, an aptamer comprising a nucleic acid sequence binding specifically to a target protein, and a decoy consisting of an oligonucleotide having a nucleic acid sequence complementary to a target transcription factor.

11. The ligand-binding nucleic acid complex according to claim 1, wherein the nucleic acid molecule is selected from among ADO, ASO, HDO, and RNAi.

12. The ligand-binding nucleic acid complex according to claim 11, wherein the antisense strand of the nucleic acid molecule consists of 12 to 30 continuous nucleotides.

13. The ligand-binding nucleic acid complex according to claim 9, wherein the nucleic acid molecule is a decoy comprising a nucleic acid sequence complementary to a target transcription factor and consisting of 8 to 30 nucleotides.

14. The ligand-binding nucleic acid complex according to claim 10, wherein the oligonucleotide is siRNA consisting of an antisense strand consisting of RNA and a nucleic acid strand complementary to the antisense strand.

15. A ligand-binding nucleic acid complex, wherein a nucleic acid molecule that regulates expression or editing of a target gene or a transcription or translation product thereof binds to a ligand reacting with an S1P receptor.

16. The ligand-binding nucleic acid complex according to claim 15, wherein the nucleic acid molecule that regulates expression or editing of a target gene or a transcription or translation product thereof binds to the ligand reacting with an S1P receptor via a linker.

17. The ligand-binding nucleic acid complex according to claim 16, wherein the linker is a cleavable linker having a cleavable structure.

18. The ligand-binding nucleic acid complex according to claim 15, wherein the nucleic acid molecule is selected from among a nucleic acid molecule comprising an antisense strand consisting of an oligonucleotide having a nucleic acid sequence complementary to a target gene or a transcription product thereof, an aptamer comprising a nucleic acid sequence binding specifically to a target protein, and a decoy consisting of an oligonucleotide having a nucleic acid sequence complementary to a target transcription factor.

19. The ligand-binding nucleic acid complex according to claim 18, wherein the nucleic acid molecule is selected from among ASO, HDO, and RNAi.

20. The ligand-binding nucleic acid complex according to claim 15, wherein the nucleic acid strand of the nucleic acid molecule comprises nucleotides, modified nucleotides, and/or nucleotide analogs.

21. The ligand-binding nucleic acid complex according to claim 20, wherein the total number of nucleotides, modified nucleotides, and nucleotide analogs in the antisense strand of the nucleic acid molecule is 12 to 30.

22. The ligand-binding nucleic acid complex according to claim 20, wherein the nucleic acid molecule is HDO and the total number of nucleotides, modified nucleotides, and nucleotide analogs in the antisense strand and that in the complementary strand of HDO are each 12 to 30.

23. The ligand-binding nucleic acid complex according to claim 15 or 16, wherein the nucleic acid molecule is HDO comprising an antisense strand consisting of an oligonucleotide having a nucleic acid sequence complementary to a target gene or transcription product thereof and a complementary strand having a nucleic acid sequence complementary to the antisense strand, and the ligand reacting with the S1P receptor binds to the complementary strand of HDO.

24. The ligand-binding nucleic acid complex according to claim 15, wherein the nucleic acid molecule is a decoy having a nucleic acid sequence complementary to a target transcription factor and consisting of 8 to 30 nucleotides.

25. The ligand-binding nucleic acid complex according to claim 20, wherein the antisense strand is a gapmer and consists of a gap region comprising nucleotides and/or modified nucleotides and a wing region (or wing regions) comprising one or a plurality of nucleotide analogs and/or modified nucleotides provided on the 5' terminal side and/or 3' terminal side thereof.

26. The ligand-binding nucleic acid complex according to claim 20, wherein the antisense strand is a non-gapmer and comprises nucleotides, modified nucleotides, and/or nucleotide analogs.

27. The ligand-binding nucleic acid complex according to claim 22, wherein the complementary strand comprises nucleotides, modified nucleotides, and/or nucleotide analogs.

28. The ligand-binding nucleic acid complex according to claim 27, wherein the complementary strand consists of a center region comprising nucleotides and/or modified nucleotides and a wing region (or wing regions) comprising one or a plurality of nucleotide analogs and/or modified nucleotides provided on the 5' terminal side and/or 3' terminal side thereof.

29. The ligand-binding nucleic acid complex according to claim 20, wherein the modified nucleotides are nucleotides comprising a 2'-O-CH₃ group or a 2'-O-CH₂CH₂OCH₃ (MOE) group.

30. The ligand-binding nucleic acid complex according to claim 20, wherein the nucleotide analogs include bridged nucleotides selected independently from among LNA, cEt-BNA, amide BNA (AmNA), and cMOE-BNA.

31. The ligand-binding nucleic acid complex according to claim 20, wherein the nucleotide analogs include bridged nucleotides selected independently from among LNA, cEt-BNA, amide BNA (AmNA), and cMOE-BNA.

32. The ligand-binding nucleic acid complex according to claim 20, wherein the nucleotide analogs are selected independently from among PNA, GNA, TNA, tcDNA, morpholino nucleic acid, and BNA.

33. The ligand-binding nucleic acid complex according to claim 20, wherein at least one nucleotide or modified nucleotide in the nucleic acid molecule is phosphorothioated or boranophosphated.
